(19) Europäisches Patentamt
European Patent Office
Office européen des brevets

(11) **EP 4 203 781 B1**

(12) # EUROPEAN PATENT SPECIFICATION

(45) Date of publication and mention of the grant of the patent:
**18.02.2026 Bulletin 2026/08**

(21) Application number: **21859468.7**

(22) Date of filing: **26.08.2021**

(51) International Patent Classification (IPC):
***A61B 5/0275*** *(2006.01)* ***A61B 6/46*** *(2024.01)*
***G16H 50/70*** *(2018.01)* ***G16H 50/50*** *(2018.01)*
***G16H 30/20*** *(2018.01)* ***A61B 8/08*** *(2006.01)*
***A61B 6/50*** *(2024.01)* ***A61B 6/00*** *(2024.01)*
***G16H 30/40*** *(2018.01)* ***A61B 6/03*** *(2006.01)*
***A61B 5/055*** *(2006.01)* ***A61B 5/026*** *(2006.01)*
***A61B 8/00*** *(2006.01)*

(52) Cooperative Patent Classification (CPC):
**A61B 6/486; A61B 5/026; A61B 5/0275; A61B 6/032; A61B 6/481; A61B 6/503; A61B 6/504; A61B 6/507; A61B 6/5217; A61B 6/5288; G16H 30/20; G16H 30/40; G16H 50/50; G16H 50/70;** A61B 5/0263; (Cont.)

(86) International application number:
**PCT/CA2021/051189**

(87) International publication number:
**WO 2022/040806 (03.03.2022 Gazette 2022/09)**

(54) **BLOOD FLOW IMAGING**
BLUTFLUSSBILDGEBUNG
IMAGERIE DU DÉBIT SANGUIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.08.2020 US 202063070531 P**

(43) Date of publication of application:
**05.07.2023 Bulletin 2023/27**

(73) Proprietor: **London Health Sciences Centre Research Inc.**
**London, ON N6C 2R5 (CA)**

(72) Inventors:
- **SO, Aaron**
  **Mississauga, Ontario L5M 6S1 (CA)**
- **LEE, Ting-Yim**
  **London, Ontario N6G 1X3 (CA)**

(74) Representative: **HGF**
**HGF Limited**
**4th Floor, 1 City Square**
**Leeds LS1 2ES (GB)**

(56) References cited:
**WO-A1-2010/045003** **WO-A1-2019/218076**
**US-A1- 2007 167 731** **US-A1- 2007 167 731**
**US-A1- 2011 150 309** **US-B2- 7 689 267**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)
A61B 5/055; A61B 6/466; A61B 8/0891; A61B 8/485

## Description

BACKGROUND OF THE INVENTION

Field of the Invention

The present invention relates to dynamic imaging of flow, and more particularly to assessment of a blood flow characteristic in a subject based on dynamic imaging of contrast agent flow through a blood vessel or heart structure.

Description of the Related Art

Dynamic contrast-enhanced (DCE) computed tomography (CT) has been used to assess blood flow and flow pressure in blood vessels, for example as described in co-owned International PCT Application No. PCT/CA2019/050668 filed 16 May 2019 (published as WO2019/218076 on 21 November 2019). Blood flow assessment derived from DCE CT represent average values over the course of many seconds of imaging scans, often greater than 10 seconds. Therefore, DCE CT does not have sufficient temporal resolution to achieve 4D flow imaging, that is, to track a blood flow characteristic in selected image voxels at very fine temporal resolution, for example calculating changes in flow velocity at time intervals of less than 1 second.

Currently available techniques for non-invasive 4D flow imaging assessment are Doppler echocardiography and MRI 4D flow. A potential drawback of Doppler echocardiography is that the blood flow assessment can be less accurate if the ultrasound beam is not aligned well with the flow jet. A potential drawback of the MRI 4D flow technique is the limited accessibility of MRI scanners and higher operating cost and longer examination times.

A recently described 4D flow CT technique (Lantz et al. (2018) Intracardiac Flow at 4D CT: Comparison with 4D Flow MRI; Radiology, Vol 289, pgs 51-58) uses computer simulation of fluid dynamics based on Navier-Stoke equations to simulate 4D flow maps to predict the magnitude and direction of flow in the aorta.

Using computer simulation of fluid dynamics to predict the flow direction and magnitude suffers from disadvantages. One potential disadvantage of using computer simulation is that currently the three dimensional Navier-Stoke equations cannot be fully solved, i.e. an exact analytic solution may not be available. Hence, accurate simulation of flow characteristics may be difficult in some situations. Another disadvantage of using computer simulation is that intensive computer simulation is time-intensive and requires extensive computer processing which does not facilitate bulk assessments at fine temporal resolution. Such computer simulations require supercomputing architecture to provide assessment that can reasonably approach temporal resolution of less than 1 second, and when performed without supercomputing facilities can require many minutes and perhaps even multiple hours to provide an assessment from the starting point of image acquisition.

Accordingly, there is a continuing need for alternative methods and systems for blood flow imaging based assessment of a blood vessel in a subject.

SUMMARY OF THE INVENTION

In an aspect there is provided, a computer implemented method for blood flow imaging comprising:

obtaining image data comprising a plurality of corresponding images capturing at least a portion of both an increase phase and a decline phase of a contrast agent in a cardiovasculature of interest;
selecting a target voxel in the image data;
generating a time-enhancement curve of the contrast agent based on the image data;
selecting first and second time points from the time-enhancement curve;
determining a rate of change of enhancement by subtraction of image data at the selected first and second time points;
determining a blood flow characteristic of the target voxel based on the rate of change of enhancement.

In other aspects, systems and non-transitory computer-readable media for executing the method are also provided.

In a further aspect there is provided a system for blood flow imaging comprising:

a memory for storing image data comprising a plurality of corresponding images capturing at least a portion of both an increase phase and a decline phase of a contrast agent in a cardiovasculature of interest;
a processor configured to generate a time-enhancement curve of the contrast agent in a target voxel based on the image data; determine a time rate of change of enhancement between first and second time points based on subtraction of image data at the first and second time points; and determine a blood flow characteristic through the target voxel based on the time rate of change of enhancement.

BRIEF DESCRIPTION OF THE DRAWINGS

Figure 1 shows a schematic of a blood flow imaging system.
Figure 2 shows a flow diagram of a blood flow imaging method.
Figure 3 shows a flow diagram of a pre-scan preparation in the imaging method shown in Figure 2.
Figure 4 shows a flow diagram of scan data acquisition in the imaging method shown in Figure 2.
Figure 5 shows a flow diagram of time-enhancement curve (TEC) generation in the imaging method shown in Figure 2.
Figure 6 shows a flow diagram of determining a blood flow characteristic based on the TEC in the imaging method shown in Figure 2.
Figure 7A and Figure 7B show schematic illustrations of two approaches for studying the movement of fluid.
Figure 8 shows a schematic representation of voxel-defined control volume analysis of flow imaging data; Figure 8A shows a schematic representation of a control volume and its control surfaces; Figure 8B shows a schematic representation of movement of fluid with respect to a control volume.
Figures 9A and 9B shows schematic illustration for two different approaches of dynamic image acquisition and reconstruction for obtaining $\Delta HU/\Delta t$ that is a basis for absolute or relative flow velocity assessment. Figure 9A shows image acquisition and reconstruction at the same cardiac phase (e.g. 75% R-R interval or diastole) over multiple time points. Figure 9B shows image acquisition covering a full cardiac cycle (systole and diastole); multiple image sets corresponding to different cardiac phases are reconstructed.
Figures 10A, 10B, 10C and 10D show a schematic illustration of a simulation experiment. Figure 10A shows passage of tracers through a control volume over time. Figure 10B shows a representation of tracer and solution shown in Fig. 10A. Figure 10C shows dimension of the control volume where tracers pass through (not drawn to scale). Figure 10D shows mass of tracers in the control volume as a function of time.
Figure 11A shows a set-up of a flow phantom experiment. Figure 11B shows a schematic illustration of a simulated cardiac cycle R-R interval reference in the flow phantom experiment.
Figure 12 shows a passage of iodinated contrast solution in a plastic tube over time in the flow phantom experiment.
Figure 13A shows a ruler attached to the plastic tube of the flow phantom experiment for the measurement of flow velocity. Figure 13B shows a contrast solution mixed with green color dye in transition through the plastic tube.
Figure 14A shows a time-enhancement curve at a 3 mL/s contrast injection rate. Figure 14B shows a time-enhancement curve at a 6 mL/s contrast injection rate.
Figure 15 shows a time-enhancement curve measured in the ascending aorta of a 55 kg farm pig.
Figure 16 shows a velocity map of an isolated pig heart generated with the 4D MRI flow technique (Peper et al, Eur Radiol Exper 2019).
Figure 17A shows an MIP image taken on a transverse plane cross-section (medial-lateral direction) of the pulmonary arterial tree of the 55 kg pig with the arrow indicating the left pulmonary artery (LPA). Figure 17B shows an MIP image taken on a frontal/coronal plane cross-section (superior-inferior direction) of the pulmonary arterial tree of the 55 kg pig with the arrow indicating the LPA. Figure 17C shows a time-enhancement curve measured from LPA (indicated by the arrow).
Figure 18A shows regions of interest (ROIs) used to assess the flow characteristics in the ascending aorta (abbreviated labels: RV - right ventricle; LV - left ventricle; AA - ascending aorta; DA - descending aorta). Figure 18B shows changes in enhancement in these ROIs during the contrast wash-in phase (from time points 5 to 7 in Fig. 15).
Figure 19 shows a schematic illustration of characteristics of a laminar flow superimposed on the ascending aorta image shown in Fig. 18A.
Figure 20 shows plots of time-enhancement curves measured in the hollow tube of the phantom experiment at the 5% and 75% R-R intervals over 15 scans (time points) with a contrast injection rate of 6 mL/s.
Figure 21 shows plots of differences in time-enhancement curves with respect to the 5% R-R interval measured over 15 scans (time points) of the hollow tube in the phantom experiment with a contrast injection rate of 6 mL/s.
Figure 22 shows a baseline phase of the time-enhancement curves shown in Figure 21.
Figure 23 shows a contrast 'wash-in' phase of the time-enhancement curves shown in Figure 21.
Figure 24A shows a plot of change in enhancement as a function of time for a 6 mL/s contrast injection rate in the phantom experiment. Figure 24B shows a plot of change in enhancement as a function of time for a 3 mL/s contrast injection rate in the phantom experiment.
Figure 25A shows a time-enhancement curve in the ascending aorta of a pig within one cardiac cycle (not fully covered) measured at the location of ROI 1 as shown in Figure 18A; the dashed line represents the projected baseline level of the curve. Figures 25B and 25C show a schematic illustration of the invasive measurement of the aortic flow velocity with a probe inserted into a patient ascending aorta.

Figure 26A shows a CT image of an internal thoracic artery (marked by an arrow) and Figure 26B shows a CT image of an abdominal fat region (marked by an arrow) where the time-enhancement curves shown in Figure 26C and Figure 26D, respectively, are measured from. Figure 26C shows two curves of the internal thoracic artery as measured at a top/upper slice (Fig. 26A) and a bottom/lower slice (CT image not shown).

Figure 27A shows a sagittal plane view (anterior-posterior direction) of a DCE CT image of a porcine heart reconstructed at the 75% R-R interval at one time point after contrast injection. Figure 27B shows a difference image generated by subtracting the DCE image reconstructed at the 30% R-R interval from the DCE image reconstructed at the 35% R-R interval.

Figure 28A shows location of ROI 3 and 30 in the ascending aorta. Figures 28B and 28C show changes in enhancement over time in these ROIs with respect to systolic cardiac phase (30% R-R interval).

Figure 29A shows location of ROI 32 and 37 in the aortic arch. Figures 29B and 29C show changes in enhancement over time in these ROIs with respect to systolic cardiac phase (30% R-R interval).

Figure 30A shows location of ROI 23 and 29 in the left atrium. Figures 30B and 30C show changes in enhancement over time in these ROIs with respect to systolic cardiac phase (30% R-R interval).

Figure 31A shows location of ROI 15 and 17 in the left ventricle. Figures 31B and 31C show changes in enhancement over time in these ROIs with respect to systolic cardiac phase (30% R-R interval).

Figure 32A shows parasternal long-axis views of a series of DCE CT images of a human heart reconstructed at a series of R-R intervals after contrast injection with an ROI marked at an LVOT region. Figure 32B shows CT number values measured at the ROI plotted against the series of R-R intervals. Figure 32C shows absolute flow velocity values calculated from the measured enhancement values (shown in Figure 32B) plotted against the series of R-R intervals. Figure 32D shows a prior art Doppler echocardiogram as an independent presentation of LVOT blood flow profile for comparison purposes.

Figure 33A shows parasternal long-axis views of a series of DCE CT images of a human heart reconstructed at a series of R-R intervals after contrast injection with an ROI marked at an ascending aorta region. Figure 33B shows CT number values measured at the ROI plotted against the series of R-R intervals. Figure 33C shows absolute flow velocity values calculated from the measured enhancement values (shown in Figure 33B) plotted against the series of R-R intervals. Figure 33D presents the plot of Figure 33C and a comparable portion of the plot from Figure 32C on the same graph. Figure 33E shows pressure curves of LVOT and ascending aorta calculated from corresponding flow velocity values. Figure 33F shows a prior art pressure curve plot obtained with invasive cardiac catheterization as an independent presentation of LVOT and ascending aorta blood pressure curves for comparison purposes.

Figure 34A shows a schematic model of blood vessel stiffening. Figure 34B shows transverse views of a series of DCE CT images of a human heart reconstructed at a series of R-R intervals after contrast injection with ROIs marked at an ascending aorta region. Figure 34C shows absolute flow velocity values calculated from the measured enhancement values for a first patient for each ROI (shown in Figure 34B) plotted against the series of R-R intervals. Figure 34D shows absolute flow velocity values calculated from the measured enhancement values for a second patient for each ROI (shown in Figure 34B) plotted against the series of R-R intervals.

Figure 35A shows a schematic of a left ventricle, ascending aorta and aortic valve area to indicate geometric orifice area (GOA) and effective orifice area (EOA) anatomical locations. Figure 35B shows a coronal plane view of a DCE CT image of a human heart reconstructed at one time point after contrast injection with ROIs marked for EOA size estimation. Figure 35C shows examples of measured and averaged CT number values in ROIs( marked in Figure 35B) plotted against elapsed time. Figure 35D depicts EOA size estimation based on relative flow velocity profiles for ROIs (marked in Figure 35B).

Figure 36A shows a schematic map of blood flow from the ascending aorta to the right coronary artery (RCA) to the posterior descending artery (PD) to myocardial capillaries. Figure 36B shows the contrast-enhanced heart image at an illustrative time point during the wash-in phase with ROIs relevant to determining flow velocity at a capillary level placed in the image. Figure 36C shows a time-enhancement curves for the RCA-ROI marked in Fig. 36B. Figure 36D shows a time-enhancement curves for the PD-ROI marked in Fig. 36B. Figure 36E shows a time-enhancement curves for the myocardial Tissue-ROI marked in Fig. 36B.

Figure 37A shows the ascending aorta in the short-axis orientation at a first time point during the wash-in phase of contrast agent with a variety of ROIs (target voxels) marked. Figure 37B show the ascending aorta in the short-axis orientation at a second time point during the wash-in phase of contrast agent with the same ROIs (target voxels) marked as in Figure 37A. Figure 37C shows a coronal view of the aortic valve area to illustrate the location of the selected short-axis slice shown in Figures 37A and 37B. Figure 37D shows a plot of a time rate of change of CT number between the two selected time points in the circular (non-isotropic) ROI marked in Figures 37A and 37B. Figure 37E shows plots of time rate of change of CT number between the two selected time points in the plurality of square isotropic ROIs marked in Figures 37A and 37B.

DETAILED DESCRIPTION OF PREFERRED EMBODIMENTS

With reference to the drawings, a system and method for blood flow imaging is described. The system and method compare favourably with current 4D blood flow imaging techniques.

Figure 1 shows a computer implemented imaging system 2, incorporating a computed tomography (CT) scanner 4. The CT scanner 4 may be any mutli-row or multi-slice CT scanner typically comprising a radiation source and a radiation detector disposed in a gantry and an adjustable, often motorized, support or table for maintaining a subject in a desired position (for example, a prone or supine position) in an open central chamber formed in the gantry during a scan procedure. The radiation source generates radiation that traverses one or more predetermined sampling sites targeting a blood vessel of interest in the subject in synchronization with a contrast agent (also referred to as a tracer) administered to the subject. The radiation detector, often configured as a panel of rotating detectors, receives radiation that traverses the subject at the predetermined sampling site(s) providing projection data (also referred to as scan data) over a time range that encompasses the increase phase and also optionally the decrease phase of contrast agent flowing through the blood vessel of interest.

The imaging system 2 includes a data acquisition component 6 incorporating a data acquisition scheme or data acquisition computer code that receives, organizes and stores projection data from the radiation detector of the CT scanner. The projection data is sent to an image reconstruction component 8 incorporating an image reconstruction computer code. The projection data can then be processed using the image reconstruction computer code resulting in image data including multiple images of the predetermined sampling site(s) spanning the increase phase and also optionally the decrease phase of contrast agent flowing through the blood vessel of interest. The image reconstruction computer code can easily be varied to accommodate any available CT imaging technique. The image data can then be processed by an image analysis component 10 incorporating image analysis computer code that generates a time-enhancement curve of the contrast signal from the image data. The time-enhancement curve data can then be processed by a blood flow estimation component 12 incorporating a blood flow estimation computer code to determine a blood flow characteristic of the blood vessel of interest from the time-enhancement curve data. The imaging system 2 is controlled by a computer 16 with data and operational commands communicated through bus 14. The imaging system 2 may include any additional component as desired to assess a blood vessel of interest including multiplexers, digital/analog conversion boards, microcontrollers, physical computer interface devices, input/output devices, display devices, data storage devices and the like. The imaging system 2 may include controllers dedicated to different components of the CT scanner 4, such as a radiation source controller to provide power and timing signals to control the radiation source, a gantry controller to provide power and timing signals to a gantry motor to control rotation of the gantry and thereby control rotation of the radiation source and detector, and a table controller to provide power and timing signals to a table motor to control table position and thereby control position of a subject in the gantry by moving the subject along a z-axis through an opening of the gantry communicative with the interior open chamber of the gantry. The imaging system 2 is shown with a CT scanner as an illustrative example only, and the system may be modified to include other imaging modalities, including for example, non-CT X-ray imaging or MRI.

Figure 2 shows a computer implemented method 20 for 4D blood flow imaging. The method 20 comprises a pre-scan preparation 30 and positioning of a subject for CT scanning of a desired sampling site. Once the subject is prepared and positioned within a CT scanner, the subject is injected 40 with a contrast agent solution, with CT scanning 50 synchronized with the injection of the contrast agent solution to acquire projection data (also referred to as scan data) over a time range that includes flow of the contrast agent through a blood vessel at the sampling site. The projection data is processed to reconstruct 60 image data from the projection data. The image data is analyzed to generate 70 a time-enhancement curve of a contrast signal parameter, such as contrast signal intensity, extracted from the image data. A blood flow value is calculated 80 based on the time-enhancement curve.

Figure 3 shows an example of a pre-scan preparation 30 of a subject for CT scanning. The pre-scan preparation 30 includes identifying a region of interest 32 in the subject. For example, the region of interest may be a portion of a blood vessel targeted for assessment of blood flow in the blood vessel. Once a region of interest is established, sampling site(s) for CT scan slices are identified 34 at or near the region of interest. Based on the predetermined sampling site(s), the subject is positioned 36 in the CT scanner in an alignment that allows for a radiation source of the CT scanner to direct radiation at the sampling site(s). Prior to scanning, the subject optionally holds breath 38 and maintains a breath-hold throughout scanning. As a further option, a hyperemic condition can be induced in the subject, for example by administering a vasodilator to the subject

Figure 4 shows an example of CT scanning 50 synchronized to injection of the contrast agent. The synchronized CT scanning 50 includes initiating a dynamic CT scan at a desired time based on an injection of the contrast agent. Optionally, the CT scanning can be synchronized to an electrocardiogram (ECG), such as provided by prospectively electrocardiogram (ECG) gated contrast-enhanced dynamic CT imaging. When ECG gating is deployed retrospective ECG-gating or prospective ECG-gating may be used. The dynamic CT scan includes acquiring of projection data prior to entry 54 of contrast agent at the sampling site(s) to set a baseline, as well as acquiring projection data during an increase phase 56 of the contrast agent at the sampling site(s) and acquiring projection data during a decline phase 58 of the contrast agent at the sampling site. An increase phase refers to an increase of mass of contrast agent at the sampling site as time advances subsequent to initial entry of the contrast agent into the sampling site, while a decline phase or decrease phase refers to a decrease of mass of contrast agent at the sampling site as time advances prior to substantially complete clearance of the contrast agent from the sampling site. Peak (maximum value) mass of contrast agent at the sampling site occurs during progression from the increase phase to the decline phase. Time elapsed from entry to clearance of contrast agent at the sampling site may be referred to as a transit time of the contrast agent. The duration of CT scanning is not limited by a requirement to capture a complete transit time of contrast agent at the sampling site provided that at least a portion of both increase and decrease phases are captured.

Figure 5 shows an example of image analysis to generate 70 a time-enhancement curve. Generation of a time-enhancement curve can include identifying 72 a voxel of interest within a plurality of corresponding images at the sampling site. Contrast agent signal data is extracted 74, for example contrast agent signal intensity, from an area defined by the voxel of interest from each of the plurality of corresponding images. A time-enhancement curve is generated 74 based on the contrast agent signal data during the increase phase, the decrease phase, or both the increase phase and the decrease phase at the sampling site.

Figure 6 shows an example of estimating blood flow 80 in a voxel/blood vessel of interest based on the time-enhancement curve. The estimation of blood flow can be achieved by determining a flow velocity value based on the time-enhancement curve. The determination of a flow velocity value can include selecting first and second time points 82 from the time-enhancement curve. A rate of change of enhancement ($\Delta HU/\Delta t$) 84 can be determined by subtraction of the CT image data at the selected first and second time points. Calculating a rate of change of tracer mass (dm/dt) 85 based on the rate of change of enhancement ($\Delta HU/\Delta t$) and the fractional volume of tracer solution that passes through the target voxel per unit time, for example using Equation 17 (see below). The tracer density can be determined 86 from the area under the time-enhancement curve, for example using Equations 13, 14 and 15 (see below). Flow velocity through the target voxel is determined 88 based on the rate of change of tracer mass (dm/dt) and tracer density ($\rho$), for example using Equation 11B (see below). The determined flow velocity value can be communicated or displayed to a technician/operator or other end-user through any conventional computer or display device.

The 4D blood flow imaging system and method have been mathematically validated. Mathematical analysis described in the following paragraphs shows examples of deriving blood flow characteristics from a prospectively electrocardiogram (ECG) gated contrast-enhanced dynamic CT imaging session.

Fluid motion can typically be assessed by two approaches. The first approach is by monitoring the movement of individual particles in the fluid over time (Figure 7A). Navier-Stokes Equations (derived from Newton's Second Law) can be used to describe the movement of any individual particle in the fluid in any direction. The second approach is by monitoring the passage of a small fraction of fluid within a fixed region (volume) over time (Figure 7B). The monitoring region or frame of reference (rectangle outlined with dark dashed line in Fig. 7B) does not move over time. The small fraction of fluid contains many individual particles that cannot be resolved, but this second approach is computationally less intensive compared to the first approach, and the results give a good approximation of the fluid movement with a reasonably good spatial resolution. Without wishing to be bound by theory, the second approach provides the basis of the analytic imaging method disclosed herein.

In CT, an image voxel, or a block of image voxels, is selected as the fixed region to monitor the movement of fluid (e.g. blood) over time. This frame of reference is called a control volume and the surface on each side of the control volume is called a control surface (as illustrated in Figure 8A). Fluid can move in and out of the control surface in any direction.

For illustration, an example shown in Figure 8B depicts movement of a small amount of fluid (grey region outlined with a dashed grey line) with respect to a control volume (CV). Here, the CV is shown in a two-dimensional view. At time 0, the fluid of interest (the grey region marked with mid-grey stripes) fills the CV completely. At a later time (time $0+\Delta t$), the same fluid of interest starts to move out of the CV in the flow direction shown in Figure 8B. The dark-grey stripes denote the portion (mass) of fluid that leaves the CV at time $0+\Delta t$ and the vacant space within the CV is filled by the incoming fluid (marked with light-grey stripes). The mid-grey stripes represent the portion of fluid that remains in the CV at time $0+\Delta t$. If the mass of incoming fluid that fills the vacant space of CV equals the mass of fluid that leaves the CV, then the flow condition is considered as steady. If the two masses (light-grey and dark-grey portions) are not equal to each other, then the flow is considered as unsteady. It should be noted that unsteady implies the flow is either turbulent, or in the transition between the steady and turbulent states. At any time, the total mass of the fluid of interest (the fluid region delineated by the dashed grey line in Fig. 8B) is unchanged. That is, the mass in the area marked with the mid-grey stripes at time 0 should equal the total mass in the area marked with the mid-grey and dark-grey stripes at time $0+\Delta t$. The movement of fluid with respect to a CV can be described using the Reynolds Transport Theorem.

The Reynolds Transport Theorem states that:

$$\frac{dB}{dt} = \frac{\partial}{\partial t}\int_{CV} b\rho d\forall + \int_{CS} bp\left(\vec{V}\cdot\hat{n}\right)dA \quad (1)$$

where B is the external property of a system, and the differential term on the left side of the equation denotes the time rate of change of *B; CV* denotes a control volume and $d^{\forall}$ denotes a small volume element within the control volume; b is the external property of the system per unit mass of the system; $\rho$ is the density of the system; *CS* is a control surface and *dA* is a small area element on the control surface; $\hat{n}$ is the unit normal vector pointing outward of the CV and is perpendicular to dA; $\vec{V}$ is the velocity vector. In this example, with reference to the noted variables, the system is the fluid of interest (Fig. 8B) and the external property is the mass of the fluid of interest. According to the conservation of mass, $dB/dt = 0$:

$$0 = \frac{\partial}{\partial t}\int_{CV} b\rho\, d\forall + \int_{CS} b\rho\left(\vec{V}\cdot\hat{n}\right)dA \quad (2)$$

As *b* is the external property (i.e. mass) per unit mass, $b = 1$:

$$0 = \frac{\partial}{\partial t}\int_{CV} \rho\, d\forall + \int_{CS} \rho\left(\vec{V}\cdot\hat{n}\right)dA \quad (3)$$

Each CV (image voxel) has six surfaces where the system (fluid) can move in and out. Hence, the second integral term on the right-hand side of equation (3) is expanded to account for each individual control surface ($CS_1$, $CS_2$, ..., $CS_6$):

$$0 = \frac{\partial}{\partial t}\int_{CV} \rho\, d\forall + \int_{CS_1} \rho\left(\vec{V_1}\cdot\hat{n_1}\right)dA_1 + \int_{CS_2} \rho\left(\vec{V_2}\cdot\hat{n_2}\right)dA_2 + \int_{CS_3} \rho\left(\vec{V_3}\cdot\hat{n_3}\right)dA_3 + \int_{CS_4} \rho\left(\vec{V_4}\cdot\hat{n_4}\right)dA_4 + \int_{CS_5} \rho\left(\vec{V_5}\cdot\hat{n_5}\right)dA_5 + \int_{CS_6} \rho\left(\vec{V_6}\cdot\hat{n_6}\right)dA_6 \quad (4)$$

The dot product of the velocity and unit normal vectors in equation (4) can be rewritten as:

$$0 = \frac{\partial}{\partial t}\int_{CV} \rho\, d\forall + \int_{CS_1} \rho|V_1||n_1|\cos\theta_1\, dA_1 + \int_{CS_2} \rho|V_2||n_2|\cos\theta_2\, dA_2 + \int_{CS_3} \rho|V_3||n_3|\cos\theta_3\, dA_3 + \int_{CS_4} \rho\,|V_4||n_4|\cos\theta_4\, dA_4 + \int_{CS_5} \rho|V_5||n_5|\cos\theta_5\, dA_5 + \int_{CS_6} \rho|V_6||n_6|\cos\theta_6\, dA_6 \quad (5)$$

where | | denotes the absolute magnitude of the vector, and $\theta$ is the angle between the unit normal vector and the velocity vector. Given that each image voxel is very small in size, we assume the angle between these vectors is negligible. That is, each pair of the unit normal and velocity vectors is approximately parallel or anti-parallel to each other, i.e. the angle between these vectors is either 0 degree (for flow moving out of the CV) or 180 degrees (for flow moving into the CV). As cosine (0) = 1 and cosine ($\pi$) = -1, equation (5) can be rewritten as:

$$0 = \frac{\partial}{\partial t}\int_{CV} \rho \, d\forall \pm \int_{CS_1} \rho |V_1||n_1| \, dA_1 \pm \int_{CS_2} \rho |V_2||n_2| dA_2 \pm \int_{CS_3} \rho |V_3||n_3| dA_3 \pm \int_{CS_4} \rho \, |V_4||n_4| dA_4 \pm \int_{CS_5} \rho |V_5||n_5| \, dA_5 \pm \int_{CS_6} \rho |V_6||n_6| dA_6 \quad (6)$$

The "±" sign means that the integral term after it can either be positive or negative, depending on the direction of flow with respect to the CV. We can define the first velocity vector $V_1$ as the flow moving into the CV, i.e. $V_1$ is in the opposition direction to $n_1$ and hence results in a negative sign in their dot product. Furthermore, the magnitude of the unit normal vector is equal to unity, i.e. $|n| = 1$:

$$0 = \frac{\partial}{\partial t}\int_{CV} \rho \, d\forall - \int_{CS_1} \rho |V_1| \, dA_1 \pm \int_{CS_2} \rho |V_2| dA_2 \pm \int_{CS_3} \rho |V_3| dA_3 \pm \int_{CS_4} \rho \, |V_4| dA_4 \pm \int_{CS_5} \rho |V_5| \, dA_5 \pm \int_{CS_6} \rho |V_6| dA_6 \quad (7)$$

As each voxel in a CT image is very small in size, we assume the density of fluid at each control surface is uniform, i.e. the density is not a function of the surface area. Hence, the density term can be moved out of the integrals:

$$0 = \frac{\partial}{\partial t}\int_{CV} \rho \, d\forall - \rho\left\{\int_{CS_1} |V_1| \, dA_1 \pm \int_{CS_2} |V_2| dA_2 \pm \int_{CS_3} |V_3| dA_3 \pm \int_{CS_4} |V_4| dA_4 \pm \int_{CS_5} |V_5| \, dA_5 \pm \int_{CS_6} |V_6| dA_6\right\} \quad (8A)$$

The integration of dA (small area element) over the entire surface area equals A:

$$0 = \frac{\partial}{\partial t}\int_{CV} \rho \, d\forall - \rho\{|V_1| A_1 \pm |V_2| A_2 \pm |V_3| A_3 \pm |V_4| A_4 \pm |V_5| A_5 \pm |V_6| A_6\} \quad (8B)$$

By choosing an isotropic voxel (or a block of isotropic voxels) as the control volume, the area of each surface of the voxel (or block of voxels) is identical to each other, i.e. $A_1 = A_2 = ... = A_6$, and we can move the area term out of the bracket { }:

$$0 = \frac{\partial}{\partial t}\int_{CV} \rho \, d\forall - \rho A\{|V_1| \pm |V_2| \pm |V_3| \pm |V_4| \pm |V_5| \pm |V_6|\} \quad (9)$$

Let us denote $V_n$ as the net flow velocity of the system (fluid of interest) passing through an image voxel:

$$|V_n| = \{|V_1| \pm |V_2| \pm |V_3| \pm |V_4| \pm |V_5| \pm |V_6|\} \quad (10)$$

Substituting (10) into (9) yields:

$$0 = \frac{\partial}{\partial t}\int_{CV} \rho \, d\forall - \rho A |V_n| \quad \textbf{(11A)}$$

$$|V_n| = \frac{\frac{\partial}{\partial t}\int \rho d\forall}{\rho A} = \frac{\frac{\partial}{\partial t}\int dm}{\rho A} = \frac{\frac{\partial m}{\partial t}}{\rho A} \quad \textbf{(11B)}$$

In Equations (11A) an (11B) the term A is a surface are of CS according to Equation 1 and can be set to 1, but in practical operational terms A depends on the area of the selected ROI, and therefore is it an independent variable that can be selected/controlled by the operator, with the caveat that A has to fit in the physiological chamber/vessel being investigated and to satisfy Equations 8 and 9 the voxel/voxel grouping has to be isotropic. Equation (11B) states that the magnitude of the net flow velocity of the fluid in a given image voxel (control volume) can be estimated if the time rate of change of the mass of fluid in the control volume and the density of the fluid are known. Both pieces of information can be obtained from dynamic contrast-enhanced CT imaging and the methods are explained in the following section.

Estimation of the density of blood flow tracer (p). In 4D CT flow imaging, the fluid to be monitored over time is usually blood moving in large blood vessels or heart chambers. Prior to imaging, iodine-based contrast solution is injected into the blood stream at a peripheral vein (e.g. an antecubical vein) to increase the blood opacification (visibility) in CT images. As the region of interest (e.g. the aorta) is usually at a distance from the injection site, the contrast solution in the region of interest is well-mixed with blood. Thus, the ρ in Equation (11B) refers to the density of iodine in a mixture of blood and contrast solution. To estimate the density of iodine in a control volume (image voxel), we need to know: **i)** the total mass of iodine injected into the patient body, and, **ii)** the total volume of blood mixed with the iodine-based contrast solution.

**i)** The total mass of iodine injected into the patient body is given by the following equation:

$$M_i = C_o \times D \times V_t \quad \textbf{(12)}$$

where $M_i$ is the mass of iodine in unit of milligram (mg), $C_o$ is the original concentration of iodine-based contrast solution in unit of mg per millilitre (mg/mL), D is the contrast dilution factor ranges from 0 to 1, and $V_t$ is the total injected contrast volume in unit of millilitre (mL). If the contrast solution is diluted to 20% of its original concentration (e.g. 20% contrast + 80% saline) before applying to the patient, then the contrast dilution factor (D) is 0.2. If there is no dilution applied, the contrast dilution factor is 1. As an example, if a contrast solution at 370 mg/mL is injected into the patient for a total of 60 mL without dilution with saline, the total mass of iodine injected into the patient equals 22,200 milligram or 22.2 gram (370 mg/mL × 1 × 60 mL).

**ii)** The volume of blood mixed with the iodine-based contrast solution can be estimated using the area under the time-enhancement curve in a two-step process:

$$Q = \frac{M_i}{\int C(t)dt} \quad \textbf{(13)}$$

where Q is the volumetric flow rate in unit of millilitre per second (or litre per minute), $M_i$ is the total mass of iodine injected determined from equation (12), the integral of C(t) is the area under the time-enhancement curve sampled at the control volume and has a unit of (HU × second), which can be converted to the unit of (mg/mL × second) with the conversion factors that were previously determined from our phantom experiments (So A et al, Medical Physics 2016;43(8):4821). The area under curve (AUC) is calculated using the data during the first-pass circulation only (recirculation phase is excluded). If the first-pass phase of the time-enhancement curve is not fully covered, data extrapolation can be applied to estimate the AUC as an arterial time-enhancement curve in the first-pass phase is relatively symmetrical. As the volumetric flow rate describes the volume of blood passes through per unit time, the volume of blood mixed with iodine, $V_i$, can be determined by the following equation:

$$V_i = Q \times T_{en} \tag{14}$$

where $T_{en}$ is the duration of time that the signal intensity in the control volume (image voxel) is higher than the baseline level, which can be determined graphically from the measured time-enhancement curve (examples are provided in the phantom experiment section). Finally, the density of iodine in the control volume (image voxel) can be estimated using the following equation:

$$\rho = \frac{M_i}{V_i} \tag{15}$$

Equations (13) to (15) suggest that the density of iodine in a control volume is dependent on the flow rate (or velocity). This is justified by the fact that the tracers traveling in a blood vessel at a higher flow rate (or velocity) are more spread out and are mixed with a larger volume of blood, compared to the tracers traveling in a blood vessel at a slower flow rate.

Estimation of the time rate of change of the mass of blood flow tracer (dm/dt). The time rate of change of the tracer mass in a control volume (image voxel) can be determined from the following equation:

$$\frac{\partial m}{\partial t} = \frac{dc}{dt} \times V_f \tag{16}$$

where dc/dt is the change in tracer concentration in the image voxel per unit time, $V_f$ is the fractional volume of tracer solution that passes through the image voxel during this period of time. Equation (16) can be further expressed in this form:

$$\frac{\partial m}{\partial t} = \left(\frac{\Delta HU}{\Delta t} \times d\right) \times \left(V_t \times \frac{\Delta t}{T_{en}}\right) \tag{17}$$

where $\Delta$HU is the difference in CT number in the image voxel between the two selected time points; $\Delta$t is the difference in time between the two selected time points; d is the factor for converting the Hounsfield Unit (CT number) to tracer (iodine) concentration (see So et al, Medical Physics 2016); $V_t$ , as defined in Equation (12), is the total volume of tracer solution injected into the patient; $T_{en}$ , as defined in Equation (14), is the duration of time that the signal intensity in the image voxel is higher than baseline. The terms within the first bracket on the right side of Equation (17) gives dc/dt. The terms within the second bracket on the right side of Equation (17) is equivalent to the fraction of the total volume of tracer solution involved during the selected time duration.

Determine changes in enhancement as a function of time ($\Delta$HU vs. $\Delta$t) from CT images. Described herein is an image reconstruction and subtraction method to examine the changes in voxel enhancement as a function of time for the 4D CT flow imaging.

For illustration, we first consider the following scenario (example 1): Suppose that we have two CT thoracic images of a patient, $I_1$ and $I_2$, acquired at two different time points after contrast injection, $T_1$ and $T_2$, respectively. $\Delta$HU in any region of the thorax can be acquired by subtracting the two CT images, $I_2$ - $I_1$. Similarly, $\Delta$t can be acquired by subtracting the two times, $T_2$ - $T_1$. This approach is applicable for any time interval.

Now we consider another scenario (example 2): the X-ray tube of a CT scanner is turned on continuously over a short period of time to collect projections around the patient. A set of CT images of the patient are generated with the measured projections (scan data) in the following way for illustration: the 1$^{st}$ image is reconstructed with projections acquired from 0° to 360° of the projection angles; the 2$^{nd}$ image is reconstructed with projections acquired from 1° to 361°;

the 3rd image is reconstructed with projections acquired from 2° and 362°; and so forth. When the 1st image is subtracted from the 2nd image (2nd - 1st image), the resulting image reflect the temporal difference between the two images that is approximately equal to the difference in their data acquisition time. Similarly, the difference image of the 3rd and 2nd images (3rd - 2nd image) reflect the temporal difference roughly equal to the difference in their acquisition time.

The method illustrated in example 1 can be applied to any image set acquired with a prospectively electrocardiogram (ECG) gated contrast-enhanced dynamic imaging session to determine $\Delta$HU and $\Delta$t between any two selected time points, such as the time points within the contrast wash-in phase of an arterial or venous time-enhancement curve, for the estimation of blood flow velocity. This is further illustrated in Figure 9A, which shows that in a prospectively ECG gated dynamic CT imaging session, the images acquired at different time points are reconstructed at the same cardiac phase (e.g. the end-diastolic phase of every 1 or 2 cardiac cycles over 15 to 20 seconds); subtracting images between two selected time points yields the $\Delta$HU and $\Delta$t values that are needed for the flow velocity measurement. If the acquisition window at any of these time points is widened to cover the full cardiac cycle, multiple sets of images correspond to different cardiac phases can be reconstructed (Figure 9B), from which the changes in flow velocity within a cardiac cycle can be assessed using the method illustrated in example 2.

The method illustrated in example 1 or example 2 can incorporate one or both of prospective ECG gating or retrospective ECG gating. From a pragmatic clinical perspective, prospective ECG gating is beneficial with CT imaging to minimize patient exposure to radiation. However, with imaging modalities that have a lower exposure risk, such as MRI, the entire wash-in and wash-out phase of contrast may be continuously imaged/recorded and particular time points to either isolate a cardiac phase in consecutive cardiac cycles or time points to extract a single cardiac cycle may be retrospectively gated without any prospective gating.

The 4D blood flow imaging system and method have been validated by experimental testing. Experimental testing results demonstrate the ability of the 4D blood flow imaging system and method to determine one or more of several blood flow characteristics. The following experimental examples are for illustration purposes only and are not intended to be a limiting description.

<u>Experimental Exemplification: Experimental Example 1 (Simulation experiment).</u>

A simulation experiment is used to test the proposed algorithm for measuring the flow velocity relative to a control volume. In this simulation, the control volume represents an isotropic image voxel with a length of 1 cm, a width of 1 cm, and a height of 1 cm (Fig. 10C). The cross-sectional area of the control volume is 1 $cm^2$ (width $\times$ height). Each grey double-head arrow in the x-y plane represents 0.2 cm (Fig. 10A). The dimension of the control volume is not drawn to scale to facilitate the visualization of the passage of tracers through the control volume in a 2D view.

A bolus of tracers passes through the control volume in the direction from left to right (see the top grey arrows) during time = 0 s to time = 14 s. The tracers consist of many tiny particles mixed in a solution. Each dot in the diagram represents one gram of tiny particles and each square box represents 0.2 $cm^3$ of solution.

According to the simulation set up, the time rate of change of the mass of tracers in the control volume can be represented by the graph shown in Figure 10D. The time difference between the baseline and peak was approximately 7 seconds. The sampling interval was set to be 0.25 seconds.

This simulation experiment was designed to provide a simple yet realistic scenario to validate the proposed algorithm for the flow velocity measurement in an isotropic image voxel. The total mass of tracers used in this simulation was 41 grams. In real-world clinical applications, it is not uncommon that 100 mL of contrast solution at a concentration of 370 mgI/mL is injected into the patient (37 grams of iodine). The time rate of change of the tracer mass follows a bolus shape similar to that observed in a clinical dynamic contrast-enhanced CT imaging study.

As an example, we used the measurements taken at 0.25 s and 7.0 s to estimate the flow velocity of tracers relative to the control volume. The measurements are summarized in Table 1.

Table 1. Comparison between the estimated and theoretical flow velocities in the simulation experiment.

| Mass of tracers at 0.25 s | Mass of tracers at 7.0 s | Change in tracer mass (dm) | Change in time (dt) | Density of tracers (ρ) | Surface area of CV (A) | Estimated magnitude of flow velocity of tracers (\|V\|, cm/s) | Theoretical flow velocity of tracers (cm/s) |
|---|---|---|---|---|---|---|---|
| 0.25 | 33.0 | 32.75 | 6.75 | 22.78 | 1 | 0.21 | 0.2 |

Figure 10 shows that the total volume of tracer solution is 1.8 $cm^3$. As the total mass of tracers is 41 g, the corresponding density of tracers is 41/1.8 = 22.78 g/$cm^3$. Furthermore, it has been shown in the mathematical derivation section that only the area of one surface of an isotropic control volume is needed for the calculation. In this case, the inlet surface area of the control volume is used for the calculation.

The estimated flow velocity is 0.21 cm/s which is only 5% different from the theoretical flow velocity (0.2 cm/s). The subtle difference could be attributed to the relatively large sampling interval (0.25 s) used for the flow estimation.

Experimental Exemplification: Experimental Example 2 (Flow phantom experiment).

A plastic suction tube about 1 m in length and 1 cm in outer diameter (0.8 cm inner diameter) was used to simulate a large blood vessel. One end of the tube was connected to the contrast injection pump and the other end was connected to an empty beaker (Figure 11). The middle section of the hollow tube was taped down to two supporting beakers to minimize its movement during the passage of contrast solution.

The iodinated contrast solution was first diluted to 20% of its original concentration (from 300 mg/mL to 60 mg/mL) with water. Next, dilated contrast solution was injected into the suction tube via an injection pump at 6 mL/s, and the middle section of the tube was scanned 15 times with a CT scanner at 60 bpm simulated heart rate. The axial scan settings were: 100 kV tube voltage, 100 mA tube current, 280 ms gantry period.

The acquisition window of each axial scan was widened to cover slightly more than one full simulated cardiac cycle (R-R interval from 0 to 105%), in a similar fashion as depicted in Figure 9B. Within each acquisition window, projections are continuously collected around the plastic tube. After the 15 axial scans, the tube was flushed with water several times to ensure that no contrast solution remained in the tube. The experiment was then repeated with identical settings except that the contrast injection rate was changed to 3 mL/s.

For each injection rate, multiple sets of tube images were retrospectively reconstructed at different cardiac phases with an increment of 5% of the R-R interval, i.e. 5%, 10%, 15%, ... , 105% R-R interval. Figure 12 shows the image set acquired at the 75% R-R interval over 15 time points. The time interval between any two consecutive time points was 3 seconds.

The phantom experiment was repeated again for the 6 mL/s and 3 mL/s injection rates with the iodinated contrast solution mixed with a green-color food dye. The suction plastic tube was attached to a ruler and a stopwatch was used to determine the flow velocity corresponds to each injection rate. Specifically, the flow velocity was estimated as the time that the solution took to travel 25 cm (from the 5 cm mark to the 30 cm mark on the ruler, Figure 13).

The measured time-enhancement curves corresponding to the two injection rates (3 mL/s and 6 mL/s) are shown in Figures 14A and 14B, respectively. These time-enhancement curves were measured from the dynamic contrast-enhanced tube images reconstructed at 5% of the R-R interval over 15 different time points. For illustration, two consecutive data points within the contrast wash-in phase were used for estimating the flow velocity of the contrast solution associated with each injection rate. For 3 mL/s injection rate, the selected data points for the flow velocity calculation were #6 and #7. For 6 mL/s, the selected data points were #5 and #6. In each experiment, contrast solution was diluted with water and approximately 1200 mg of iodine were injected into the hollow tube via the injection pump. Table 2 summarizes the measurements acquired from the time-enhancement curves shown in Figures 14A and 14B.

Table 2. Measurements acquired from the time-enhancement curves in Fig. 14A and 14B for the calculation of flow velocity.

| | **Enhancement at 1st selected time point (HU)** | **Enhancement at 2nd selected time point (HU)** | **Change in enhancement (Δ HU)** | **Time difference (Δt, s)** | **Area under curve (HU × s)** | **Area under curve (mg/mL) × s** |
|---|---|---|---|---|---|---|
| 3 mL/s | -15.7 | 476.38 | 492.08 | 3 | 10756.5 | 430.26 |
| 6 mL/s | 1.6 | 496.08 | 494.48 | 3 | 4618.77 | 184.75 |

Figures 14A and 14B show that the duration of enhancement corresponding to the 3 mL/s and 6 mL/s injection rates was 24 and 12 seconds, respectively. Using Equation (13), the corresponding volumetric flow rates were estimated to be 2.8 mL/s and 6.4 mL/s, respectively. The small discrepancies (6.7 %) in the estimated volumetric flow rates with respect to the actual volumetric flow rates (which are the pump injection rates, 3 mL/s and 6 mL/s) could be related to the fact that the area under curve in each case was calculated without smoothing (de-nosing) the measured time-enhancement curves. For any real-world clinical study, the volumetric flow rate in any blood vessel of interest is not given and therefore it is advantageous to estimate the flow rate from the measured time-enhancement curve first. Using Equations (14) and (15), the tracer densities corresponding to the 3 mL/s and 6 mL/s injection rates were estimated to be 17.9 and 15.4 mg/cm$^3$, respectively. Using Equations (16) and (17), the changes in tracer mass per unit time corresponding to 3 mL/s and 6 mL/s were estimated to be 82.0 and 164.8 mg/s, respectively. The area of the control surface is 0.5 cm$^2$. With these parameters

and Equation (11), the flow velocities of the contrast solution in the tube corresponding to the 3 mL/s and 6 mL/s injection rates were estimated to be 9.1 and 21.3 cm/s, respectively. The actual flow velocity associated with each pump injection rate was measured by recording the time required for the coloured contrast solution to travel 25 cm in the tube (from the 5 cm mark to the 30 cm mark). Comparison between the flow velocities estimated by the 4D CT flow technology and the actual flow velocities measured by stopwatch in the phantom set-up shown in Figure 13 is provided in Table 3. The flow velocities estimated by the 4D CT flow technology were comparable to the actual flow velocities measured with the stopwatch.

Table 3. Flow velocity measurements corresponding to the two pump injection rates

| Injection rate | Estimated magnitude of flow velocity of tracers (\|V\|, cm/s) | Actual flow velocity of tracers (cm/s) | Difference between estimated flow velocity and actual flow velocity |
|---|---|---|---|
| 3 mL/s | 9.1 cm/s | 10.4 cm/s | -1.3 cm/s |
| 6 mL/s | 21.3 cm/s | 21.2 cm/s | +0.1 cm/s |

Experimental Exemplification: Experimental Example 3 (Large animal experiment).

Experimental Example 3A: flow velocity measurement in ascending aorta. In addition to the simulation (Example 1) and flow phantom (Example 2) experiments, the 4D CT flow technology was also tested in a large animal study. The study subject was a 55 kg farm pig. The pig was anesthetized and scanned in a supine position with a clinical CT scanner (Revolution CT, GE) following a bolus injection of iodinated contrast solution. Dynamic contrast-enhanced (DCE) images of the heart were acquired with these scan settings: 100 kV tube voltage, 100 mA tube current, 280 ms gantry speed, 16 cm axial coverage. The ECG signals of the pig were recorded in real-time throughout the dynamic CT imaging session. Multiple sets of DCE images were retrospectively reconstructed from 30% to 85% R-R intervals with a 5% increment (i.e. 30%, 35%, 40%, ..., 85%). The DCE image set reconstructed at the 30% R-R intervals was subtracted from the image sets reconstructed at the higher R-R intervals to generate multiple sets of difference images in a similar fashion to the flow phantom experiment.

For illustration, we first used the image set reconstructed at the 75% R-R interval (end-diastoles) to estimate the flow velocity in the ascending aorta. The time-enhancement curve measured from the ascending aorta over 22 time points is shown in Figure 15. Two time points within the contrast wash-in phase, #5 and #7, were selected for measuring the aortic flow velocity (for reference, time points 1 to 4 were within the baseline phase).

A total of 38 mL of contrast solution at a concentration of 370 mg/mL was injected into the pig. The total mass of iodine injected into the pig was 370 × 38 = 14060 mg. Table 4 summarizes the measurements acquired from the aortic time-enhancement curve shown in Fig. 15.

Table 4. Measurements acquired from the aortic time-enhancement curve of a 55 kg pig shown in Figure 15.

| Enhancement at time point 5 (HU) | Enhancement at time point 7 (HU) | Change in enhancement (Δ HU) | Time difference (Δt, s) | Area under curve (HU × s) | Area under curve (mg/mL × s) |
|---|---|---|---|---|---|
| 50 | 375 | 325 | 6 | 5882.80 | 235.31 |

The area under curve in Table 4 was calculated using the first-pass data only (the recirculation phase was excluded). Using Equation (13), the volumetric flow rate was estimated to be 3585.0 mL/min (3.6 L/min), which is within the normal range of cardiac output. According to the time-enhancement curve, the duration of enhancement in the first-pass circulation was approximately 23 seconds. Using Equations (14) and (15), we estimated that iodine was mixed with 1374.3 mL of blood and the density of iodine in blood was 10.23 mg/$cm^3$. Using Equations (16) and (17), the change in iodine mass per unit time was estimated to be 21.48 mg/s. Entering these parameters into Equation (11), the flow velocity in the ascending aorta was estimated to be 35.0 cm/s.

The aortic flow velocity of this pig estimated with the 4D CT flow technology is in good agreement with the value reported in other literatures using the 4D flow MRI technology. For instance, an article by Peper et al. (Peper et al. (2019) An isolated beating pig heart platform for a comprehensive evaluation of intracardiac blood flow with 4D flow MRI: a feasibility study. European Radiology Experimental, Vol 3 (1), pg 1-10) reported that the flow velocity in the ascending aorta in an isolated pig heart with a cardiac output of 3.2 L/min was approximately 35 to 40 cm/s (see arrow in Figure 16).

In Peper's article, the flow velocities of seven isolated pig hearts were reported. Pig heart #1 (Fig. 16) was selected for the comparison with our result because this heart had a cardiac output of 3.2 L/min which was similar to the cardiac output of our pig (3.6 L/min).

This result suggests that the flow velocity derived from the 4D CT flow technology is comparable to the 4D MRI flow method.

Experimental Example 3B: flow velocity measurement in pulmonary artery. The 4D CT flow technology was also applied to measure the flow velocity in the left pulmonary artery. Figure 17 shows the MIP (maximum intensity projection) images of the pulmonary arterial tree of the same 55 kg pig, and the time-enhancement curve measured from the left pulmonary artery. The CT measurements from the pulmonary time-enhancement curve are summarized in Table 5.

Table 5. Measurements acquired from the pulmonary time-enhancement curve shown in Figure 17C.

| Enhancement at time point 2 (HU) | Enhancement at time point 5 (HU) | Change in enhancement (Δ HU) | Time difference (Δt, s) | Area under curve (HU × s) | Area under curve (mg/mL × s) |
|---|---|---|---|---|---|
| 60 | 600 | 540 | 7 | 5882.80 | 235.31 |

The main pulmonary artery branches into the left and right pulmonary arteries. Previous literatures have suggested that the right pulmonary artery receives slightly more blood from the main pulmonary artery than the left pulmonary artery does due to the angulation of the branching. With the assumption that 40% of the contrast solution entering into the left pulmonary artery and the parameters measured from the left pulmonary arterial time-enhancement curve (Table 5), the flow velocity of the left pulmonary artery was estimated to be 18.5 cm/s, which is within the normal range of pulmonary artery flow velocity measured with 4D MRI flow (between 9±2 to 32±10 cm/s, Odagirl K et al SpringerPlus 2016; 5:1071), and is in good agreement with the fact that the pulmonary arterial flow velocity is usually lower than the aortic flow velocity (35.0 cm/s).

Experimental Exemplification: Experimental Example 4 (Assessment of flow characteristics and boundary conditions).

Flow velocity measurement acquired with the 4D CT flow technology can be used to assess the flow characteristics in a blood vessel (e.g., whether the blood flow follows a laminar flow pattern). Figure 18B shows the changes in enhancement over time along different flow paths (shown in Figure 18A) in the ascending aorta. The two central flow paths (ROI 1-4 and ROI 5-8) exhibited a much larger increase in enhancement compared to the peripheral flow path (ROI 9-10) over the same duration of time (~6 s). As illustrated in Equations (11B) and (17), the magnitude of flow velocity is proportional to the change in enhancement (ΔHU) per unit time (Δt). Thus, the graph in Figure 18B has two implications: first, the flow velocities along the two central flow paths were higher than the flow velocities along the peripheral flow paths; second, the flow velocities in each voxel along the central flow paths are relatively consistent with each other.

These findings suggest that the blood flow in the ascending aorta was laminar, since it exhibited a classic laminar flow pattern where the flow velocity is consistent along each central path and between different central flow paths, and gradually decreases towards the boundaries of the blood vessel (boundary condition, see Figure 19).

The Reynolds number (not related to the Reynolds transport theorem) is calculated using the following equation to independently confirm whether the blood flow in the ascending aorta was laminar:

$$Re = \frac{V \cdot D \cdot \rho}{\eta} \qquad (18)$$

where Re is the Reynolds number, V is the flow velocity in unit of meter per second (m/s), D is the diameter of the blood vessel in unit of meter (m), ρ is the blood density in unit of kilogram per cubic meter ($kg/m^3$), and η is the blood viscosity in unit of kg/ms. From the calculation steps shown above, the flow velocity in the ascending aorta was estimated to be 35.0 cm/s or 0.35 m/s. The diameter of the ascending aorta was 2.0 cm or 0.02 m. Previous literatures reported that the blood density and viscosity are approximately 1060 $kg/m^3$ and 0.004 kg/ms, respectively. With these parameters, the Reynolds number was estimated to be 1855. It is well known that the blood flow is laminar if the associated Reynolds number is less than 2300, and as such, the measurement with the 4D CT flow technology is consistent with what the Reynolds number predicts.

Experimental Exemplification: Experimental Example 5 (Assessment of flow acceleration).

The control volume analysis with the Reynolds transport theorem (RTT) can be used to assess the blood flow velocity at a temporal resolution superior to an area-under-curve (AUC) approach. This is because the RTT method uses only a fraction (e.g. front slope) of the time-enhancement curve for the flow velocity measurement (Fig. 9A), whereas the AUC method needs the entire first-pass phase in the time-enhancement curve for the flow velocity calculation. Data acquired from the flow phantom experiment and the large animal (55kg pig) experiment can be used to demonstrate how the blood flow velocity and flow pattern can be assessed at an even higher temporal resolution using the approach shown in Fig. 9B, from which changes in flow velocity (flow acceleration) can be visualized.

Figure 20 shows the time-enhancement curves measured from the tube images generated at two different image reconstruction phases (5% R-R vs. 75% R-R intervals) over 15 time points. These images correspond to the 6 mL/s contrast injection rate. The largest difference between the two time-enhancement curves occurred at time points 5 and 8, corresponding to the contrast "wash-in" and "wash-out" phases respectively.

The 5% R-R image set was subtracted from the image sets reconstructed at the higher R-R intervals, i.e. 10%-5%, 15%-5%, 20%-5%, ..., 105%-5%. From each set of difference images, the enhancement in the hollow tube was measured and plotted as a function of time (image number). The results are shown in Figure 21.

Partial plots focusing on the initial (baseline) phase of the curves from Fig.21 are shown in Figure 22. The difference in enhancement curves among different image reconstruction phases was minimal from time points 1 to 4; this is because the contrast solution had not arrived in the hollow tube during this period of time. Next, partial plots focusing on the contrast wash-in phase of the curves from Fig. 21 which covers from time points 4 to 6 are shown in Figure 23. The difference in enhancement among different image reconstruction phases became prominent during this period of time points 4 to 6, with the largest difference in enhancement occurred at time point 5.

A closer look at time point 5 reveals that the degree of enhancement change was not constant over time. Additionally, the rate of change in enhancement increased as the time difference between the two selected image reconstruction phases increased, i.e. 95%-5% R-R interval >> 10%-5% R-R interval. These differences in enhancement at time point 5 can be used to generate the graph in Figure 24A.

Figure 24A illustrates the time rate of change of enhancement in the hollow tube over one second at the 6 mL/s contrast injection rate. The y-axis of the graph represents the difference in enhancement ($\Delta$HU) with respect to the reference 5% R-R interval; the x-axis of the graph represents the time difference ($\Delta$t) with respect to the reference 5% R-R interval.

In the flow phantom experiment, the simulated heart rate was set to be 60 beats per minute, so the duration of a full cardiac cycle (R-R interval) was one second (1000 milliseconds). Given that the time difference associated to a 1% difference in the R-R interval was 10 milliseconds, the time difference between any two cardiac phases selected for image reconstruction can be calculated accordingly. For example, the time difference between the 10% and 5% R-R intervals was 50 milliseconds (ms). Using this approach, we obtained a $\Delta$HU value for any specific $\Delta$t as shown in Figure 24A. The $\Delta$HU versus $\Delta$t graph can be generated in a similar fashion for the 3 mL/s injection rate (Figure 24B).

Equation (16) shows that $\Delta$HU/$\Delta$t can be converted into dm/dt, which is proportional to the flow velocity according to Equation (11B). Hence, the plots shown in Figure 23 can be used to assess the time rate of change of flow velocity (flow acceleration).

As shown in Figure 24B, the flow of contrast solution in the tube at the 3 mL/s injection rate was approximately steady as the time rate of change in flow velocity was relatively constant between consecutive time intervals (~0.05 s per interval). By contrast, the flow of contrast solution in the tube at the 6 mL/s injection rate was clearly accelerating but was not yet a turbulent flow, which is characterized by random (chaotic) changes in the flow velocity. The flow acceleration at 6 mL/s did not appear to be random except at one time point (the little bump at 0.7 sec in the graph shown in Figure 24A). As the likelihood of turbulent flow increases with flow velocity, the flow at 6 mL/s was likely between steady and turbulent (transitional state).

Experimental Exemplification: Experimental Example 6 (Decomposition of absolute flow velocity into its components).

The time-enhancement curve of a pig ascending aorta shown in Figure 15 can be used to further illustrate that absolute flow velocity can be measured in very-small time intervals. As discussed in the large animal experiment section, the flow velocity in the ascending aorta of the pig was calculated using the data between time points 5 and 7 (within the contrast wash-in phase over a time interval of 6 seconds). The flow velocity measurement in the same region of the ascending aorta was repeated using time points 5 and 6, and time points 6 and 7. The results are summarized in Table 6.

Table 6. Comparison of the flow velocities in the ascending aorta measured with data spanning across different time intervals of the arterial time-enhancement curve shown in Figure 15.

| **Selected time points** | 5 and 6 | 6 and 7 | 5 and 7 |
|---|---|---|---|
| **Time interval (s)** | 3 | 3 | 6 |
| **Flow velocity (\|V\|, cm/s)** | 13.5 | 21.5 | 35.0 |

It is clear that the sum of the flow velocities associated with the time intervals "5-6" and "6-7" equals to the flow velocity associated with the time interval "5-7". This result demonstrates that the flow velocity can be decomposed into smaller components according to the time interval selected for the calculation.

This concept can be understood by the following analogy: Suppose that there is a car that can accelerate from 0 to 100 km/h in 5 seconds. This means when the foot pedal is pressed at time zero, the car will reach a speed of 100 km/h in five seconds later. Assuming that there is no change in the car direction and the change in car speed is constant over this 5-second interval, the car speed at each second mark should be: 20 km/h (at 1 s), 40 km/h (at 2 s), 60 km/h (at 3 s), 80 km/s (at 4 s), 100 km/h (at 5 s). The corresponding change in car speed at each second mark should be: +20 km/h (at 1 s), +20 km/h (at 2 s), +20 km/h (at 3 s), +20 km/h (at 4 s), +20 km/h (at 5 s). The latter can be interpreted as follows: at t = 1 s, there is an increase in 20 km/h with respect to t = 0 s; at t = 2 s, there is an increase in 20 km/h with respect to t = 1 s; at t = 3 s, there is an increase in 20 km/h with respect to t = 2 s or 40 km/h with respect to t = 1 s, and so forth.

Putting this analogy into perspective, the aortic flow velocity associated with the time interval "5-6" indicates an increase of 13.5 cm/s at time point 6 with respect to time point 5. Similarly, the aortic flow velocity associated with the time interval "6-7" indicates an increase of 21.5 cm/s at time point 7 with respect to time point 6, or an increase in 35.0 cm/s with respect to time point 5.

Experimental Exemplification: Experimental Example 7 (Reconstruction of cardiac-induced pulsation in the flow velocity profile of a blood vessel).

The ability of assessing the flow velocity in very-small time intervals facilitates the generation of the flow velocity profile of a blood vessel over a full cardiac cycle (from systole to diastole). Figure 25 shows that the time-enhancement curve measured in a porcine ascending aorta within one heart-beat is highly reminiscent to the flow velocity profile in a human ascending aorta acquired invasively with a catheter-based technique. Based on the discussion above, we can use the control volume analysis with the Reynolds transport theorem to construct a flow velocity profile from this time-enhancement curve.

Such pulsation is not only observed in the flow velocity profile of a large blood vessel but also in the curves acquired from much smaller blood vessels. Figure 26C shows that the time-enhancement curves of the internal thoracic artery at two slice locations (top slice section shown in Figure 26A) also manifested a prominent pulsation. The fluctuation in the curves was a consequence of the cardiac effect and the contribution from random noise was minimal. This is justified by the fact that the curve acquired from an abdominal fat region (CT slice image shown in Figure 26B) shown in Figure 26D did not exhibit such fluctuation. Figure 26C and Figure 26D curves are plotted with a Y-axis having the same range of CT number (300 HU) to facilitate visual comparison.

Experimental Exemplification: Experimental Example 8 (Reconstruction of cardiac-induced pulsation in the flow velocity profile of a heart chamber or the aorta proximal to the heart).

The control volume analysis with Reynolds transport theorem coupled with the image reconstruction and subtraction method detailed in previous sections can be used to assess the flow velocity and flow pattern in the heart chambers. The following examples are derived from a set of dynamic contrast-enhanced (DCE) images of a porcine heart acquired over a single cardiac cycle after a single bolus injection of contrast solution. The CT imaging settings were: 100 kV tube voltage, 100 mA tube current, 280 ms gantry speed, 16 cm axial coverage. The ECG signals of the pig were recorded in real-time during the dynamic imaging session. DCE heart images were retrospectively reconstructed at 30% to 85% R-R intervals with a 5% increment. The DCE image set reconstructed at 30% R-R interval was subtracted from the image sets reconstructed at other R-R intervals to generate different sets of difference images in a similar fashion to the flow phantom experiment (an example of a difference image is shown in Figure 27B).

Figures 28 to 31 show the changes in enhancement over time in different regions of the heart or the aorta proximal to the heart, measured from the corresponding difference images (an example of a difference image shown in Figure 27B). The plots of $\Delta$HU versus $\Delta$t in the same region of the heart or aorta exhibit similar patterns of oscillation and are different from the plots measured in other regions of the heart or aorta.

Figure 28 shows the changes in enhancement over time in two regions (shown in Fig. 28A) of the ascending aorta. The two ΔHU vs. Δt graphs (shown in Figs. 28B and 28C) show a similar pattern of enhancement change with respect to the systole (30% R-R interval). Towards the end of the systolic phase (phase 1 labelled in Fig. 28B), contrast solution ejected from the left ventricle arrived in the ascending aorta, so there was an initial increase in enhancement. The contrast solution then moved from the ascending to the descending aorta, leading to a reduced enhancement in the ascending aorta (phase 2 labelled in Fig. 28B). Later, the retrograde flow (backflow) in the aorta pushed a fraction of contrast solution back into the ascending aorta, leading to an increased enhancement in the ascending aorta towards the diastolic phase (80% R-R interval, phase 3 labelled in Fig. 28B).

Figure 29 shows the changes in enhancement over time in two regions (shown in Fig. 29A) of the aortic arch. Both ΔHU vs. Δt curves (shown in Figs. 28B and 28C) show a "jiggling" pattern that is very different from the oscillating pattern exhibited in the curves associated with the ascending aorta (Fig. 28). The magnitude of "jiggling" in each curve was different from others. Such "jiggling" pattern reflects random mixing of the contrast solution in the aortic arch, which is the region with a narrow and sharp curvature and the entrance to the left common carotid artery and the brachiocephalic artery.

Figures 30 and 31 show the changes in enhancement over time with respect to the systole in two regions of the left atrium (shown in Fig. 30A) and in two regions of the left ventricle (shown in Fig. 31A). Both ΔHU vs. Δt curves associated with the left atrium (shown in Figs. 30B and 30C) show a gradual decrease in enhancement over time with respect to the systolic phase. During the time between the systolic and diastolic phases, blood moved from the left atrium to the left ventricle, therefore the amount of contrast solution in the left atrium decreased, which sequentially reduced the enhancement in this chamber. By contrast, the ΔHU vs. Δt curves measured in the left ventricle (shown in Figs. 31B and 31C) showed a large increase in enhancement initially due to the squeezing of the myocardium during the systolic state (phase 1 labelled in Fig. 31C). The enhancement then decreased when a portion of contrast solution was pumped out of the left ventricle (phase 2 labelled in Fig. 31C). This chamber was then refilled by the contrast solution coming from the left atrium, compensating for the loss of contrast solution to the ascending aorta (phase 3 labelled in Fig. 31C).

Figure 32 shows the use of Reynolds Transport Theorem (RTT) to evaluate the temporal variation of flow velocity in a blood vessel during a cardiac cycle. Figure 32 illustrates how RTT can be applied to reconstructed image data to generate a flow velocity profile in the left ventricular outflow tract (LVOT) over a cardiac cycle in a human patient. After an intravenous bolus injection of contrast solution (50 mL at 350 mgI/mL concentration), a short contrast-enhanced CT cine scan covering the whole heart over a full cardiac cycle with retrospective electrocardiogram (ECG) gating was initiated (see Figure 9B for illustration). The patient's heart rate was 50 beats per minutes during the CT acquisition, so the duration of a full cardiac cycle was 1.2 seconds. The heart images were retrospectively reconstructed from the scan data at 5% to 95% of the R-R intervals with a 10% increment in cardiac phase (i.e. a total of 10 image sets were reconstructed). In each set of heart images, a region of interest (ROI) was placed in the LVOT in the parasternal long-axis view (Figure 32A; ROI additionally marked with a black arrow) and CT numbers were measured in the ROI in each of the images. The CT numbers (in Hounsfield Unit, Figure 32B) were used to derive the absolute flow velocity in the LVOT (in meter per second) with the RTT method as illustrated in the previous sections and plotted against temporal variation to produce a flow velocity profile over a cardiac cycle. The resulting flow velocity profile derived from dynamic CT (Figure 32C) is highly reminiscent to a blood flow profile independently obtained with Doppler echocardiology (Figure 32D; A wave marked with dashed white line and E wave marked with solid white line) in terms of the magnitude of the E and A waves and the overall shape of the flow velocity profile. Figure 32D is previously published (e-echocardiography.com) and is included here for comparison only.

Experimental Exemplification: Experimental Example 9 (Absolute versus relative flow velocity measurement).

The experimental examples have demonstrated that absolute flow velocity derived using the control volume analysis with the Reynolds transport theorem can be a reliable and effective tool for assessing blood flow characteristics. The experimental examples also demonstrate that flow velocity is proportional to the time rate of change of tracer (iodine) mass, which can be converted from the time rate of change of enhancement (ΔHU / Δt) measured from dynamic contrast-enhanced CT images. Therefore, it is possible to assess the relative flow velocity solely based on the time rate of change of enhancement. For example, Figure 18B demonstrates that the aortic flow velocity in regions near the boundary of the vessel wall is lower than that in the central regions, according to their relative differences in the time rate of change of enhancement.

However, while the relative flow assessment is advantageously applicable for comparing flow velocities among different flow paths within the same blood vessel, it may not be reliable for the comparison between different blood vessels and between studies that are independent from each other. An illustrative example, as to caveats of relative flow velocity assessment may be gleaned from the flow phantom experiment. Figure 14A and 14B show the time-enhancement curves associated with two separate bolus injections at different injection rates. The time rate of change of enhancement during the contrast wash-in phase associated with the two injection rates was almost identical between the two pump injection rates (see Table 2). Specifically, the percentage difference in enhancement during the same time interval was only 0.49 % (492.08 vs. 494.28 HU). However, the percentage difference in absolute flow velocity between the two pump injection rates was 80.3% (9.1 cm/s vs. 21.3 cm/s - see Table 3).

Another example illustrating a caveat of relative flow velocity assessment is the comparison between the aortic and pulmonary arterial flow velocities of the pig. Experimental Examples 3A and 3B determine an absolute flow velocity in the ascending aorta and the left pulmonary artery as 35.0 cm/s and 18.5 cm/s, respectively (aortic flow was about 2 times faster than left pulmonary artery flow). Table 4 and Table 5 show that $\Delta HU/\Delta t$ corresponding to the aortic and left pulmonary arteries was 51.4 (325/6) and 77.1 (540/7) respectively. Therefore, the relative flow comparison between two different blood vessels that is solely based on their difference in $\Delta HU/\Delta t$ may not be reliable.

Experimental Exemplification: Experimental Example 10 (systolic pressure gradient).

The ability to generate a flow velocity profile of a blood vessel over a full cardiac cycle has useful diagnostic applications. One example is to assess the systolic pressure gradient between the LVOT and ascending aorta to characterize different heart conditions such as aortic stenosis. The study patient characterized in Figure 32 is again used for illustration here. To assess systolic pressure gradient across the aortic valve, the flow velocity profile in the ascending aorta was generated in the same way as for the LVOT - A region of interest (ROI) was placed above the root of the ascending aorta in the parasternal long-axis view (Figure 33A; the ROI is additionally marked with a black arrow) to obtain the CT number at each cardiac phase during the systole (from 5% to 45% R-R interval, Figure 33B). Aortic flow velocity at each cardiac phase was then derived from the corresponding CT numbers with the RTT method described in previous sections (Figure 33C). Next, the flow velocity profiles of the LVOT and ascending aorta were linearly extrapolated from 5% to 0% R-R interval to estimate the corresponding initial flow velocities (Figure 33D). The extrapolation is needed for this study since the cardiac images were not reconstructed prior to 5% R-R interval. As can be seen from Figure 33D, the aortic flow velocity was much higher than the LVOT flow velocity during the systole. Next, the systolic pressure gradient between the LVOT and ascending aorta throughout the systole was estimated with the Bernoulli's equation, which describes the relationship between flow velocity and pressure at any two selected points A and B along a blood vessel, and has the following mathematical form:

$$P_A + \frac{1}{2}\rho V_A^2 + \rho g h_A = P_B + \frac{1}{2}\rho V_B^2 + \rho g h_B + P_L \qquad (19)$$

where $P_A$ and $P_B$ are the blood pressure at A and B respectively; $V_A$ and $V_B$ are the blood flow velocity at A and B respectively; $\rho$ is the density of blood and is approximately equal to 1.06 g/m$^3$; g is the gravitational acceleration and is approximately equal to 980 cm/s$^2$; $h_A$ and $h_B$ are the vertical height of A and B relative to a reference point. The location A can be selected as the reference point so that $h_A$ can be zero; $P_L$ is the pressure loss due to the viscosity of blood and friction against the vascular surface. $P_L$ can be estimated with the method described in co-owned PCT application no. PCT/CA2019/050668 filed 16 May 2019. Equation (19) can be rearranged into the following form:

$$\Delta P = P_B - P_A = \frac{1}{2}\rho(V_A^2 - V_B^2) - \rho g h_B - P_L \qquad (20)$$

It can be seen from Equation (20) that the systolic pressure gradient across the aortic valve can be estimated from the flow velocities of LVOT and ascending aorta at each cardiac phase within the systole. Prior to the CT scan, blood pressure of the study patient was measured noninvasively with a blood pressure cuff. The systolic blood pressure was found to be 175 mmHg, and this reading was assumed to be identical to the aortic pressure at 35% RR interval. The blood pressure of the LVOT at the same cardiac phase was estimated to be 198.36 mmHg using the Bernoulli's equation. This cardiac phase was used as the reference point to estimate the systolic pressure gradient between the LVOT and ascending aorta with the Bernoulli's equation in a similar fashion. Figure 33E shows the pressure curves of LVOT and ascending aorta. The maximum pressure gradients were also labeled in Figure 33E. These curves were highly similar to the ones obtained with invasive cardiac catheterization (Figure 33F). The mean systolic pressure gradient between the LVOT and aorta in our study patient was between 20 to 30 mmHg, which agreed with the level associated to moderate aortic stenosis as independently confirmed by Doppler echocardiography (mean pressure gradient of 37 mmHg). In contrast, the higher systolic pressure gradient (> 40 mmHg) shown in Figure 33F suggested the patient had a severe aortic stenosis. The source of Figure 33F is properly quoted and this figure is included here for comparison only. Figure 33F is previously published (Geske et al., JACC Cardiovascular Interventions, 2012) and is included here for comparison only.

Experimental Exemplification: Experimental Example 11 (Blood vessel stiffness).

The heart-induced pulsation graphs described above (e.g. Fig 25A and Fig. 26C) reveal the temporal changes in enhancement within a cardiac cycle (heart beat). The enhancement can be converted to the flow velocity (or flow rate) using the 4D blood flow imaging method, and the flow velocity (or flow rate) can be then converted to the flow pressure using the Bernoulli's equation (for example, as described in co-owned International PCT Application No. PCT/CA2019/050668 filed 16 May 2019 which describes flow pressure and fractional flow reserve (FFR) as blood flow characteristics that may be quantified). After the conversion, a plot can be obtained showing the temporal change of the flow pressure in a blood vessel within a cardiac cycle, which can assess blood vessel stiffness comparable to the wave form patterns for arterial stiffness shown in Figure 4 of van Varik et al. (van Varik et al. (2012) Mechanisms of arterial remodeling: lessons from genetic diseases. Frontiers in Genetics, Vol. 3, pg. 1-10). As can be seen in that Figure 4, the flow pressure profile of a normal artery is different from that of a stiffened artery. Therefore, this technique would be useful for assessing the stiffness of blood vessels, for example blood vessel stiffness in older patients with hypertension.

Arterial stiffening refers to the condition in which an artery (e.g. aorta) gradually loses its ability to expand and contract with alterations in blood pressure (Figure 34A).

Increase in arterial stiffening is thought to be related to aging and arteriosclerosis, and it is closely associated with hypertension and other adverse cardiovascular events. The underlying pathophysiology of arterial stiffening is complex, with recent studies suggesting that systemic inflammation may play an important role in the vascular remodeling process. As such, stiffening may simultaneously occur in more than one type of large artery. Arterial stiffening can be assessed noninvasively by measuring the pulse wave velocity (PWV) with ultrasound. PWV is conventionally measured as the velocity at which the blood pressure pulse travels from the carotid to femoral arteries. If these arteries are not imaged in a dynamic CT scan, the RRT method may offer an alternative approach to assess arterial stiffening through quantification of the flow velocity changes adjacent to the blood vessel wall during a cardiac cycle. The rationale of this approach is based on the hypothesis that reduced vascular elasticity leads to a smaller degree of pulsation of the vessel wall, which sequentially results in a smaller (lower magnitude) fluctuation in the blood flow adjacent to the vessel wall. Two patient studies are used to illustrate the feasibility of this approach for assessing arterial stiffness. One patient had a systolic blood pressure (SBP) of 130 mmHg, which was in the pressure level of pre-hypertension. The other patient had a SBP of 175 mmHg, which was in the pressure level of Stage 2 hypertension. The latter patient was more likely to suffer from arterial stiffening compared to the other patient. Both patients had a short contrast-enhanced CT cine scan covering a full cardiac cycle with retrospective ECG gating (as illustrated in Figure 9B). The heart images were retrospectively reconstructed from 5% to 95% RR intervals with a 10% increment in cardiac phase (i.e. 10 cardiac image sets were generated). In each patient, three ROIs were placed adjacent to the wall of the ascending aorta at approximately the same slice location. The ROIs were minimally adjusted if necessary to accommodate the movement of the vessel wall during the cardiac cycle. The CT number (in Hounsfield Unit) in each ROI corresponding to each cardiac phase was recorded. Flow velocity in each ROI at each cardiac phase was derived using the RTT method described in previous sections. As shown in Figure 34C and Figure 34D, the aortic flow velocity in each ROI were less fluctuated over a cardiac cycle in the patient with a very high SBP (175 mmHg, Fig. 34C) than the patient with a lower SBP (130 mmHg, Fig. 34D). The difference in fluctuation was particularly prominent in the systolic phases when the heart contracted (5% to 45% RR intervals). The standard deviation of the flow velocities in the three ROIs over 10 cardiac phases was 13.34 cm/s in the patient with a SBP of 175 mmHg, which was much lower compared to the patient with a SBP of 130 mmHg (standard deviation of 23.23 cm/s), further suggesting the patient who had a much higher systolic blood pressure had a stiffer aorta. The data in this Example 11 shows that dynamic CT coupled with the RTT method may provide a new way to quantitatively evaluate stiffening in different arteries. Furthermore, the preclinical example shown in Figure 17A to 17C illustrates the proposed RTT method can be applied to blood vessels other than the aorta such as the pulmonary vessels.

Experimental Exemplification: Experimental Example 12 (Estimate size of effective orifice area).

Aortic valve area (AVA) is a parameter used for anatomic assessment of aortic stenosis. In aortic stenosis an aperture defined within AVA may be narrowed, leading to reduced blood flow to the ascending aorta from the left ventricle. AVA can be further categorized into the geometric orifice area (GOA) and effective orifice area (EOA). The GOA refers to the anatomic area of the valve aperture, while the EOA is the cross-sectional area of the vena contracta, which is the narrowest central flow region characterized by high-velocity laminar flow. The GOA is not identical to the EOA (Figure 35A; as shown in Saikrishnan et al., Circulation, 2014). The EOA represents the region in the ascending aorta with the largest pressure gradient across the narrowed aortic valve, and as such, is considered as a more reliable metric than the GOA for assessment of aortic stenosis. The EOA is conventionally assessed with Doppler echocardiology in clinical settings. Conventional CT can measure the GOA but not the EOA. However, the RTT method described herein may facilitate the delineation of the EOA with CT. A patient study is shown in Figure 35 for illustration. After an intravenous bolus injection of contrast solution, the patient had a dynamic contrast-enhanced CT scan with prospective ECG gating at every 1 or 2 diastoles (see Figure 9A for illustration of the scan protocol). The total duration of the scan was approximately 30 seconds. Figure 35B shows the contrast-enhanced cardiac image reformatted into the coronal view at one time point corresponding to the wash-in phase of the contrast agent in the ascending aorta. Multiple regions of interest (ROIs) were placed in the ascending aorta in the reformatted heart image. These ROIs were further classified as a first group of ROIs within the two central reference lines and a second group of ROIs outside the two central lines. The graph in Figure 35C shows the changes in mean CT number in these ROIs (EOA plot is an average of CT number values measured for all ROIs 4-15 within central reference lines; similarly the outside EOA plot is an average for all ROIs 26-35 outside the central reference lines) over a short duration corresponding to the contrast wash-in phase (i.e. $\Delta$HU/$\Delta$t in Equation 17). The ROIs within the two central lines exhibited steady increase in CT numbers over time. On the contrary, the ROIs located outside the two central lines showed relatively unsteady change in the mean CT number over the same period of time. As explained in previous sections, the time rate of change of CT number ($\Delta$HU/$\Delta$t) is closely related to flow velocity, and a steady increase in HU over time implies a steady flow velocity, which is the characteristics of laminar flow. As stated at the beginning of this Example, the EOA is the region characterized by high-velocity laminar flow. Hence, either the relative or absolute flow velocity derived with the RTT method may be used to estimate the size of the EOA. To illustrate further, Figure 35D depicts the approximate EOA region in the ascending aorta of the same patient. The diameter of the EOA was estimated to be the average of the upper and lower dimensions of the region (10 mm and 8.8 mm respectively, and the average was 9.4 mm). Assuming the EOA has a circular shape as shown in the schematic in Figure 35A, the EOA area was estimated to be 69 $mm^2$ or 0.69 $cm^2$. In comparison, the EOA assessed by Doppler echocardiology was 0.7 $cm^2$. The finding indicates it is feasible to determine the EOA using the relative or absolute flow velocity assessed with dynamic CT acquisition. Localizing the EOA within the AVA is possible by comparing flow characteristics of ROIs placed on either side (ascending aorta side vs. LVOT side) of a putative expected EOA location as ROIs placed on the ascending aorta side would demonstrate relatively greater turbulent flow patterns, while ROIs placed on the LVOT side would demonstrate relatively greater steady flow patterns.

Experimental Exemplification: Experimental Example 13 (Perfusion - blood flow in tissues).

Since the 4D blood flow imaging method can assess flow velocity (mL/min), perfusion (in mL/min per gram) can be calculated if the mass of the downstream tissue (e.g. myocardium) is known. A method based on calibration and blood volume to estimate the mass of tissue is being tested. Motwani et al. (Motwani et al. Systolic versus Diastolic Acquisition in Myocardial Perfusion MR Imaging. Radiology, Vol 262(3), pg 816-823) show that systolic and diastolic perfusion measurement may have different implications (see Figure 2 of Motwani et al.) and diagnostic accuracy (see the ROC in Figure 5 of Motwani et al.). No available CT or MR perfusion method offers a sufficiently high temporal resolution to assess both systolic and diastolic perfusion in a single study. Therefore, the perfusion assessment technique described herein would be a novel improvement over currently available techniques.

The Experimental Examples make clear that the RTT method can be used to assess flow velocity in large blood vessels (such as the aorta). The RTT method can also be extended to evaluate flow velocity at the capillary level (tissue perfusion). While CT or MRI does not have sufficient spatial resolution to image a single capillary, individual capillaries in a small tissue region can be lumped together as a single blood vessel to assess the mean flow velocity with the RTT method (Figure 36A). An important step for this application is to properly estimate the volume of the tracer solution going into the selected tissue region of interest. An example is provided in Figure 36 for illustration. The patient had a dynamic contrast-enhanced CT scan with prospective ECG triggering at every 1 or 2 diastoles following an intravenous bolus injection of contrast agent (see Figure 9A for illustration of the scan protocol). Figure 36B shows the contrast-enhanced heart image at one time point during the wash-in phase, and the ROIs placed in the image. The corresponding time-enhancement curves are shown in Figure 36C, Figure 36D, and Figure 36E.

Approximately 2% of the total mass of the injected tracers (13475 mg $\times$ 0.02 = 269.5 mg) was distributed to each major coronary artery including the right coronary artery (RCA). The posterior descending artery (PD) is a branch of the distal RCA and supplied blood to the inferior wall of the heart. The total area of the inferior wall of the heart was found to be 2784 $mm^2$. The myocardial ROI placed within the inferior wall was 15 $mm^2$ (Figure 36B), which was approximately 0.5% to the total area of the inferior wall. Assuming 50% of the tracers distributed to the RCA was also distributed to the PD (assumption based on the radii of the branches in the RCA), and assuming the volume of contrast agent passing through the PD was evenly distributed in the inferior wall, the total mass of iodine tracers distributed in the myocardial ROI was estimated to be approximately 0.7 mg. The average capillary density in the myocardium of a human heart is approximately 2200 capillaries per $mm^2$ (Rakusan K et al, Circulation 1992;86:38-46). Since the average radius of a capillary is approximately 5 to 10 $\mu$m, the total cross-sectional area of the capillary in the selected myocardial ROI was estimated to be 0.04 $cm^2$. Together with the time rate of change of CT number during the wash-in phase, the flow velocity in the selected myocardial ROI was estimated to be 0.134 cm/s or 1.34 mm/s. This magnitude of flow velocity is within the ranges of capillary flow velocities (~0.5 mm/s to 1.5 mm/s) reported in previous publications (Ye F et al, Journal of Biomedical Optics 2020,25(1):016003; Ivanov KP et al, Microvascular Research 1981,22(2):143-55; Diem et al. Biophysical Journal 2019,117:2316-2323). If the total myocardial mass (in gram) is known, then the flow rate or flow velocity per unit mass of tissue can also be derived by estimating the mass of tissue corresponding to the selected myocardial ROI.

Experimental Exemplification: Experimental Example 14 (non-isotropic voxel).

As shown in Equation (11B), the flow velocity V is depending on A which is the area of the control surface. The fact that V is inversely proportional to A can be explained by the principle of continuity (e.g. a smaller lumen yields a faster blood flow velocity to maintain the same volumetric flow rate). In the mathematical derivation section equations (see progression of Equation (8B) to Equation (9)) can be further simplified by choosing an isotropic voxel (identical dimensions in x, y and z directions) for image analysis. This Experimental Example 14 shows that the RTT method would work even if the region of interest is non-isotropic. This study patient had a dynamic contrast-enhanced CT scan with prospective ECG gating at every 1 or 2 diastoles after an intravenous bolus injection of contrast agent. Figure 37A and Figure 37B show the ascending aorta in the short-axis orientation at two time points during the wash-in phase of contrast agent. The two selected time points were approximately 4 seconds apart. The approximate location of the selected short-axis slice above the aortic valve is illustrated in the coronal view in **Figure 37C.** The study patient had a severe aortic stenosis with a ~0.5 $cm^2$ (50 $mm^2$) EOA (effective orifice area). The circular ROI placed in the short-axis images represents the approximate size of the EOA in the ascending aorta. The 16 small boxes within the circular ROI were the isotropic voxels covering the EOA. The time rate of change of CT number between the two selected time points in the circular (non-isotropic) ROI and the square isotropic ROIs are shown in **Figure 37D and 37E** respectively. It can be seen from the graphs that the $\Delta HU/\Delta t$ in the circular ROI was very close to the average $\Delta HU/\Delta t$ (black line in Fig. 37E; light grey lines in Fig. 37E are $\Delta HU/\Delta t$ plots for individual isotropic ROIs) in the square/isotropic ROIs (28.56 versus 30.09 HU/s). Since $\Delta HU/\Delta t$ is proportional to V, the flow velocity in each isotropic ROI was approximately 16 times higher than the circular ROI (because the area of each square ROI was roughly 1/16 of that of the circular ROI). The flow velocity in each square ROI can be combined to obtain the total flow velocity in the EOA. In this case, the total A is 16 times larger than the A of each square ROI, so the total flow velocity is 16 times lower than the flow velocity of each square ROI, which should be equal to the flow velocity estimated with the circular ROI. In summary, this Example 14 demonstrates that the RTT method can be applied in both isotropic and non-isotropic region of interest to assess flow velocity, if the area can be properly accounted for. The choice of A may be dependent on factors such as the luminal size of the blood vessel of interest.

Several illustrative variants of a method or system for 4D blood flow imaging have been described above. Further variants and modifications are described below. Moreover, guiding relationships for configuring variants and modifications are also described below. Still further variants and modifications are contemplated and will be recognized by the person of skill in the art. It is to be understood that guiding relationships and illustrative variants or modifications are provided for the purpose of enhancing the understanding of the person of skill in the art and are not intended as limiting statements.

For example, the 4D blood flow imaging method 20 as shown Figure 2 is merely illustrative, and should not be considered as limiting to the 4D blood flow imaging method as one or more steps shown in Figure 2 can be substituted or removed as desired for a specific implementation. For example, in a specific implementation CT scanning of a subject may be geographically or temporally displaced from image reconstruction. An example, of a contemplated variant 4D blood flow imaging method includes both projection data from CT scanning and image reconstruction occurring at a prior stage and reconstructed images are stored for analysis at either a later date or for analysis by a third party. The variant 4D blood flow imaging method can initiate by obtaining the stored image data. Contrast agent signal data can then be extracted from the stored image data, optionally without explicitly identifying a target blood vessel in the image data. A time-enhancement curve is generated based on the contrast agent signal data, the time-enhancement curve having an upslope plotted from data points obtained during an increase phase of the contrast agent signal data, and a downslope plotted from data points obtained during a decline phase of the contrast agent signal data. A flow velocity value is then determined according to the same method steps shown in Figure 6.

As another example, the 4D blood flow imaging method and system are not limited to computed tomography (CT) scanning, and can readily be adapted to other imaging modalities that have sufficient spatial resolution to image blood vessels and exhibit proportional increase in signal intensity in a ROI as a function of the mass of contrast agent present in the ROI (more contrast agent or tracers results in a higher signal in the ROI), including MRI and other X-ray imaging techniques (ie., X-ray imaging techniques other than CT imaging), including for example fluoroscopy. X-ray based scans are a form of medical imaging comprising transmission of a high frequency electromagnetic signal that becomes attenuated as it passes through the body of a subject with the remaining signal captured by a detector for subsequent analysis. Data for the Experimental Examples was acquired with a single-energy CT (SECT) scanner. Most clinical CT scanners use single-energy acquisition. However, dual-energy CT (DECT) scanners are also available. Dual-energy CT refers to two X-ray energy sources used for scanning an object instead of a single X-ray energy source. Existing literature shows that dual-energy CT can perform dynamic CT acquisition just like single-energy CT. From the image processing aspect, nothing changes and methods described herein, such as the RTT method, can be applied in both SECT and DECT.

An alternative to X-ray based scans is Magnetic Resonance Imaging (MRI), which has well-recognized medical imaging applications including for example, imaging to diagnose disease in soft tissues such as the brain, lungs, liver, muscles, and heart. MRI scans involve the application of a magnetic field to a patient and the transmission of radio frequency pulses. Resonance energy is emitted by the patient and picked up by a receiver/detector that captures scan data for subsequent analysis. To improve image clarity, both X-ray scans and MRI scans involve the oral or intravenous administration of a contrast agent to a patient. Contrast agents for X-ray imaging techniques include for example iodine-based contrast agents. Contrast agents for MRI imaging techniques include for example gadolinium-based contrast agents. Scan data acquired from X-ray based scanner devices/systems are often referenced as scan data or projection data interchangeably, while scan data acquired from MRI scanner devices/systems are typically referenced as scan data. Thus, the term scan data is understood to encompass the term projection data.

The RTT method described herein was demonstrated with dynamic contrast-enhanced CT imaging data obtained after an intravenous bolus injection of iodine-based contrast agent, and this method is also applicable for dynamic MRI imaging data obtained after intravenous bolus injection of Gadolinium-based (Gd) contrast agent. We have demonstrated with a preclinical study in co-owned PCT application no. PCT/CA2019/050668 (filed 16 May 2019) that a time-enhancement curve in a region of interest can be obtained from dynamic contrast-enhanced MRI imaging in a similar manner to dynamic contrast-enhanced CT imaging. Furthermore, the temporal change in signal intensity (e.g. $T_1$ relaxation time) over time is induced by the movement of Gd contrast agent in the region of interest, and the magnitude of signal alteration is closely related to the concentration of Gd-based molecules (tracers). When a low concentration of Gd contrast agent is used, the change in MRI signal intensity and contrast concentration in a region of interest exhibits a relatively linear relationship, which facilitates the estimation of the time rate of change of mass of tracer (dm/dt in Equation 11) with the RTT method to derive flow velocity.

Contrast agents (also referred to as tracers) for various imaging modalities are established in the current literature and continue to be an active area of development for new alternatives. The 4D blood flow imaging method and system may accommodate any suitable combination of contrast agent and imaging modality provided that the imaging modality affords sufficient temporal and spatial resolution to image a cardiovasculature of interest, for example a blood vessel of interest or a portion of a blood vessel of interest or a heart chamber of interest or a portion of a lumen of a heart chamber of interest.

The 4D blood flow imaging method and system is considered 4D (four-dimensional) because of reconstructed 3D (three-dimensional) image data combined with an advantageous temporal resolution. Efficient image processing provided by Control Volume Analysis adapted with Reynolds Transport Theorem to improve temporal resolution of scan data (for example, acquired from CT or MRI scans) as described herein need not be limited to 3D image data and may also be applied to other types of image data, such as 2D (two-dimensional) image data. Therefore, while the application of the blood flow imaging technique described herein may find significant use in 4D imaging, it is not limited to 4D imaging and can very readily accommodate other imaging modes such as 2D-imaging to improve temporal resolution.

The 4D blood flow imaging method and system is considered 4D (four-dimensional) because of an advantageous time component combined with 3D image data, and more particularly an advantageous fine temporal resolution of 3D image data. The 4D blood flow imaging method and system is characterized by a fine temporal resolution that is based on determination of a change of enhancement during an increase phase or decline phase of enhancement that can be calculated at very short time intervals including time-intervals that are less than 3 seconds, less than 2 seconds, less than 1 second, less than 0.5 seconds or less than any time-interval therebetween. Efficient processing provided by Control Volume Analysis adapted with Reynolds Transport Theorem in combination with sufficient temporal resolution of scan data provided by CT imaging (or other imaging modalties such as MRI) provides tracking of a blood flow characteristic in selected image voxels at very fine temporal resolution that can approach real-time assessment. Therefore, the 4D blood flow imaging method and system can be considered as providing near real-time assessment in that changes in blood flow can be tracked at short time intervals of less than 1 second. For relative flow assessments, since an area under the curve calculation is not required, processing time may approach real-time in that latency from real-time of a flow event to the processing and providing the assessment of the blood flow characteristic of the flow event may be less than 10 seconds, less than 5 seconds, less than 2 seconds or less than anytime therebetween. However, for absolute flow assessments, processing time does not approach real-time in that latency from real-time of a flow event to the processing and providing the assessment of the blood flow characteristic of the flow event is typically greater than 10 seconds. The processing latency is less than currently available 4D flow techniques, often achieving processing times of less than 600 seconds, less than 300 seconds, less than 180 seconds, less than 120 seconds, less than 60 seconds or less than any time therebetween. In circumstances, where batch timing is provided, latency from real-time event of providing the assessment of the blood flow characteristic will typically be less than 60 minutes, less than 30 minutes, less than 15 minutes, less than 10 minutes or less than any time therebetween.

The 4D blood flow imaging method and system includes selection of a target voxel in the acquired image data (ie., acquired pixel data) and analysis of the pixel data in the selected voxel. While voxels provide precision to volumetric imaging, voxel based assessment can also be disadvantaged by large data sets that are unwieldy to manage given the bandwidth of common computers. The systems and methods described herein provide an efficient manipulation of large data files that permits interactive visualization and fine temporal resolution with near real-time assessment using commonly available computers.

A voxel is the smallest 3D element of volume and is typically represented as a cube or a box, with height, width and depth dimensions (or 3D Cartesian coordinate x, y and z dimensions). Just as 2D images are made of several pixels (represented as squares, with height and width, or x and y dimensions) and the smaller the pixel the better the quality of the picture, the same concept applies to a 3D data volume. In data acquisition, each three-dimensional voxel represents a specific x-ray absorption. A voxel stated as isotropic means that all dimensions of the isotropic voxel are the same and typically the isotropic voxel is a perfect cube, with uniform resolution in all directions. In contrast, a voxel stated as anisotropic or non-isotropic means that the anisotropic voxel is not a perfect cube, such that all dimensions of the voxel are not the same (ie., at least one dimension of the anisotropic voxel is different than other dimensions) or that the anisotropic voxel includes partial voxel units (typically more than one voxel unit). The systems and methods described herein provide an efficient manipulation of image data that permits operability with selection of one or both of isotropic or non-isotropic target voxels.

The terms ROI and target voxel are related, as an ROI in reconstructed 3D image data will encompass either a target voxel or a block of neighboring target voxels. In reconstructed 2D image data, an ROI will encompass either a target pixel or a block of neighboring target pixels, and therefore the terms ROI and target pixel are also related. The terms voxel and pixel are related as voxel is a 3D analog of a pixel. Voxel size is related to both the pixel size and slice thickness. Pixel size is dependent on both the field of view and the image matrix.

A selected isotropic target voxel may be a single isotropic voxel or a continuous block of neighboring or adjacent voxels where the block is isotropic. A selected non-isotropic target voxel will typically encompass more than one voxel unit, but may approach a volume of a single voxel unit or may be a continuous block of neighboring or adjacent voxels where the block is non-isotropic. A non-isotropic block of voxels can include parts of voxels at its boundary as would be expected if the target voxel is a non-square shape such as a circle or triangle. Thus, blocks of target voxels or target pixels need not be limited to full voxel or pixel units as an ROI of various shapes (including circles, triangles or even irregular shapes) may be accommodated, and an ROI may defining a block of neighboring voxels or pixels with partial voxels at the boundary of the ROI.

The elapsed time of an imaging scan procedure, equivalent to the time duration of scan data acquisition, can be varied as desired provided that the imaging scan captures at least a portion of both an increase phase and a decline phase of contrast agent at the sampling site so as to obtain sufficient data to estimate shape of the time-enhancement curve. Generally, to capture both increase and decline phases an imaging scan of greater than 5 seconds is needed. In certain examples, imaging scans can be configured to capture scan data for greater than 6 seconds, greater than 7 seconds, greater than 8 seconds, greater than 9 seconds or greater than 10 seconds. Although not constrained by an upper time limit and not constrained by the transit time of contrast agent, most often imaging scans will not extend significantly beyond the expected transit time of contrast agent at a sampling site.

The number of images (also referred to as frames or individual scans) analyzed to generate the time-enhancement curve can be varied as desired provided that the number of images cumulatively captures at least a portion of both an increase phase and a decline phase of contrast agent at the sampling site so as to obtain sufficient data to estimate shape of the time-enhancement curve. Generally, to capture both increase and decline phases an imaging scans of greater than 5 images is needed. In certain examples, imaging scans can be configured to capture scan data for greater than 6 images, greater than 8 images, greater than 10 images, greater than 12 images, greater than 14 images, greater than 16 images, greater than 18 images, or greater than 20 images. Additionally, imaging scans configured to capture at least 10 images are observed to benefit consistency of peak value determinations and curve shape; signal intensity values need not be extracted from all of the at least 10 images, but the at least 10 images often provides a large enough set of images to select a subset of appropriate time-distributed images (typically 5 or more images) that leads to consistency of estimating curve shape.

Some aspects of the 4D blood flow imaging method and system may be operable without generation of a time-enhancement curve having both increase and decrease phases. For example, relative flow velocity may be determined from a time-enhancement curve having a portion of the increase phase only or a portion of the decrease phase only. The generation of a time-enhancement curves having both increase and decrease phases benefit area-under-curve (AUC) calculations and further calculations that input AUC values, including for example tracer density calculations. Therefore, any determination, such as relative flow velocity, that does not require an AUC input or other AUC derived inputs (such as density) does not require a time-enhancement curve having both increase and decrease phases.

The 4D blood flow imaging method and system is considered dynamic due to analysis of a plurality of images as distinguished from static techniques that evaluate a single image. Most commercially available CT angiography techniques are static. Furthermore, commercially available CTangiography techniques that are minimally dynamic (evaluating 2 to 3 images) do not recognize or consider benefits of acquiring scan data from both the increase phase and decline phase of contrast agent transit or generating a time-enhancement curve having an upslope, peak and downslope. Furthermore, CTangiography studies that obtain 2 or 3 images at slightly different time frames, for motion correction, or for the doctor to select the best image that is least affected by motion, may also be considered a static technique.

A plurality of images, for example at least 5 images, for generating a time-enhancement curve are considered to be a plurality of corresponding images with the correspondence of images referring to a time-ordered sequence of multiple images located in the same sampling site or slice or in a group of adjacent sampling sites or slices. Thus, correspondence of images is spatially limited to a single sampling site or a group of adjacent sampling sites (or to a single ROI or a group of adjacent ROIs), and correspondence of images does not include sampling sites spatially separated to be upstream versus downstream of a source of blood flow aberration. For example, when determining a blood flow characteristic comprises a comparison of corresponding values calculated from first and second time-enhancement curves, the first time-enhancement curve may be generated from a first plurality (or set) of corresponding images from a first sampling site located upstream of a suspected source of a blood flow aberration and the second time-enhancement curve may be generated from a second plurality (or set) of corresponding images from a second sampling site located downstream of the suspected source of the blood flow aberration. In this example, the first set of corresponding images will not be intermingled with the second set of corresponding images as the first and second sampling sites are spatially separated by an intervening suspected source of blood flow aberration.

Each set or plurality of corresponding images is time-ordered or time-resolved to generate a time-enhancement curve. The time-enhancement curve has an upslope, a peak and a downslope. Time-ordering is needed to generate the time-enhancement curve so that the upslope of the time enhancement curve is interpolated from time-specific contrast agent signal data points acquired during an increase phase of contrast agent transit, and the downslope of the time enhancement curve is interpolated from time-specific contrast agent signal data points acquired during a decline phase of contrast agent transit. Accordingly, acquisition of scan data and reconstruction of image data occurs with reference to a time-ordering scheme such that each set of corresponding images obtained from the image data can be arranged in a time-ordered sequence. A time-ordering scheme can be any convenient scheme including a time stamp with a real-time identifier, a relative-time identifier such as elapsed time from bolus injection, or any customized time identifier that can be used for identifying absolute or relative time of each image and time-resolved sequencing of the set of corresponding images. Established protocols for time intervals between contrast agent administration and image acquisition may be adopted in devising a time ordering scheme. Furthermore, established timing techniques, for example bolus tracking, may be adopted to optimize timing of scan acquisition and time-ordering of image data.

The time-enhancement curve is a plot of contrast agent signal intensity versus time derived from scan data of a contrast agent transit at a single sampling site or a group of adjacent sampling sites. The time-enhancement curve may also be referred to as a time-density curve, signal intensity time curve, time-dependent signal intensity, time-intensity curve among other variations. The term enhancement within the term time-enhancement curve refers to an increase in measured contrast signal intensity relative to a baseline or reference value such as signal intensity measured at a minimal level of contrast agent or measured at a residual level of contrast agent or measured in absence of contrast agent. Qualitative terms describing a contrast agent transit, such as prior to entry, entry, wash-in, increase phase, decline phase, wash-out, clearance and subsequent to clearance, are referenced to a bolus injection event or more generally a contrast agent administration event, such that each of these terms, except prior to entry, describing a portion of a contrast agent transit that occurs subsequent to an associated injection or administration event. The term prior to entry may correspond to a time range that may begin earlier than the injection or administration event.

In many examples, the 4D blood flow imaging method and system includes generation of at least one time-enhancement curve. However, in certain examples that do not require assessment of a time-enhancement curve, for example a relative flow velocity assessment, a generation of a time-enhancement curve may not be necessary and therefore in these examples a set of corresponding images may be queried to identify and select an image with peak signal intensity and extract a peak enhancement value without establishing a time-enhancement curve. A risk of extracting a peak enhancement value without a time-enhancement curve is that the selected image of peak signal intensity may be an outlier that may not be apparent in absence of a comparison to a time-enhancement curve; however, this risk may be acceptable for generalized screening assessments, such as assessments of multiple sampling sites of multiple vessels in an organ in data acquired from a single scan session used as a proactive screening tool to identify blood flow aberrations. Regardless of optionality of generation of a time-enhancement curve, the 4D blood flow imaging method and system requires image data comprising a plurality of corresponding images capturing at least a portion of one of an increase phase and a decline phase of contrast agent transit through a blood vessel of interest or other cardiovasculature of interest.

The 4D blood flow imaging method and system described herein allows for determination of a blood flow characteristic. A blood flow characteristic may be any metric that assesses blood flow at a region of interest in a subject. A blood flow characteristic includes, for example, flow rate, flow velocity, flow acceleration, flow pressure and reconstruction of heart-induced pulsation. Heart-induced pulsation refers to temporal variation of flow rate / flow velocity arising from the heart contraction and relaxation (which lead to forward ejection and backward suction of blood respectively). Rate, velocity, and acceleration are metrics of blood flow. The 4D blood flow imaging technique may be complemented with other blood flow assessment techniques as desired, for example blood flow assessment or blood pressure assessment (using Bernoulli's equation) as described in co-owned International PCT Application No. PCT/CA2019/050668 filed 16 May 2019 which also describes fractional flow reserve (FFR) and shear stress as blood flow characteristics that may be quantified; and also describes area under the curve, rate of change of area under the curve, peak (maximum value) of the curve, and blood volume as further examples of a blood flow characteristic.

A blood flow characteristic can be determined from raw signal intensity measurement or enhancement measurements. In CT, measured signal intensity can be stated as CT number, while enhancement infers a normalization against a reference value or a subtraction of signal intensities.

The determination of a blood flow characteristic can minimally require determination of a time rate of change of a signal intensity or an enhancement and typically include determination of a time rate of change of a parameter including for example, time rate of change of signal intensity, time rate of change of enhancement, time rate of change tracer mass, time rate of change of flow velocity, time rate of change of flow pressure, and the like. The various time rate of change parameters are related as described in above mathematical derivations. For example, Equation (16) shows that $\Delta HU/\Delta t$ can be converted into dm/dt, which is proportional to the flow velocity according to Equation (11B). Hence, the plots shown in Figure 23 can be used to assess the time rate of change of flow velocity (flow acceleration).

Assessment of blood flow and determination of a blood flow characteristic can provide a diagnostic result. For example, determining time-enchancement curves at first and second sampling sites (or first and second ROIs in the same sampling slice) yields a first time-enhancement curve and a second time-enhancement curve; and estimating of the blood flow characteristic comprises a determination including corresponding values calculated from the first and second time-enhancement curves. As another example, a relative flow velocity or absolute flow velocity may be determined at one or more ROIs. The blood flow characteristic value may in itself provide a diagnostic result. In further examples, corresponding values calculated from the first and second time-enhancement curves or first and second flow velocities are compared and a difference in the corresponding values beyond a predetermined threshold is indicative of a diagnostic result. Thresholds and corresponding diagnostic results can be adopted from relevant literature and medical guidelines. Furthermore, with repeated use of the 4D blood flow imaging method and system, various correlations of metrics, thresholds and diagnostic results may be developed.

A region of interest (ROI) is an area on a digital image that circumscribes or encompasses a desired anatomical location, for example a blood vessel of interest or a portion of a lumen of a blood vessel of interest or heart chamber of interest or any other cardiovasculature of interest. The terms ROI and target voxel or target pixels are related as the ROI defines an area that encompasses one or more voxels (in 3D imaging) or one or more pixels (in 2D imaging). The terms voxel and pixel are related in that both rely on pixel data, but voxel is a 3D-analog of pixel and is an accumulation of pixel data from multiple slices in a 3D image.

Image processing systems permit extraction of pixel data from ROI on images, including for example an average parametric value computed for all pixels within the ROI. A sampling site is the location of one or more imaging slices selected to assess a desired anatomical location, such as a blood vessel of interest or heart chamber of interest or any other cardiovasculature of interest. In some examples, analysis of a time-enhancement curve from a single ROI may be sufficient to determine a blood flow characteristic or metric. In other examples, a plurality of ROIs in a single sampling site or a plurality of ROIs in a plurality of sampling sites, or a plurality imaging slices may be analyzed to obtain a plurality of corresponding image sets and to generate a plurality of corresponding time-enhancement curves, and any number of the plurality of corresponding time-enhancement curves may be compared to determine a blood flow characteristic or blood flow metric. Conventional scanners can capture 3D image data for all or part of a blood vessel of interest or other cardiovasculature of interest, and possibly even all or parts of a plurality of vascular structures such as a plurality of blood vessels of interest. Furthermore, a scan can be subdivided into a plurality of slices as desired, and therefore interrogation of multiple sites or slices at an ROI, near an ROI, upstream of an ROI, downstream of any ROI, or any combination thereof, is feasible and convenient. In multi-slice or multi-site imaging modalities, simultaneous tomographic slices or sampling sites may be extracted per scan. Thus, the 4D blood flow imaging method need not be limited to analysis of one or two time-enhancement curves for a scan of a contrast agent transit (entry to clearance) at blood vessel interest and a single scanning procedure with a single bolus injection of contrast agent can support a plurality of slices or sampling sites divided from the scan data as desired.

Motion correction or motion compensation processing of reconstructed image data may be used if ROIs benefit from adjustment to accommodate the movement of the vessel wall during the cardiac cycle. Rules-based or machine learned motion correction or compensation models are available, and may be used as desired for specific implementations.

A cardiovasculature of interest (also referred to as vascular structure of interest) may be any blood flow passage or lumen of the cardiovascular system (also referred to as the circulatory system), and may include any blood vessel of interest (including for example systemic arteries, peripheral arteries, coronary arteries, pulmonary arteries, carotid arteries, systemic veins, peripheral veins, coronary veins, pulmonary veins) or any heart chamber of interest or any heart aperture of interest that can be imaged by a contrast-enhanced imaging technique. The cardiovasculature of interest will typically have a diameter of at least about 0.1 mm, for example a diameter greater than 0.2 mm or a diameter greater than 0.3 mm. The cardiovasculature of interest, such as a blood vessel of interest or a designated portion of the blood vessel of interest, may be identified and targeted for contrast enhanced blood flow imaging to determine a diagnosis of a cardiovascular disorder or a blood vessel disorder or to determine a predisposition to such disorder. A blood vessel of interest can be within any anatomical area or any organ (for example, brain, lung, heart, liver, kidney and the like) in an animal body (for example, a human body).

The 4D blood flow imaging method is not limited to scan data acquired while a subject is in a hyperemic state (also referred to as hyperemic stress or vasodilatory stress) and time-enhancement curves generated from scan data acquired while a subject is in a non-hyperemic state (also referred to as a resting state) can produce a useful result. Inducing a hyperemic state is a well-known medical protocol in blood flow assessment and often includes administration of a vasodilator such as adenosine, sodium nitroprusside, dipyridamole, regadenoson, or nitroglycerin. Mode of administration of the vasodilator may vary depending on an imaging protocol and can include intravenous or intracoronary injection.

To determine a presence of a cardiovascular disorder at a cardiovasculature of interest, such as a blood vessel disorder at a blood vessel of interest, a blood flow characteristic will be analyzed based on at least one time-enhancement curve, including for example a single time-enhancement curve generated from pixel data of an ROI in a scan of a single sampling site, or as another example a plurality of time-enhancement curves respectively generated from a corresponding plurality of sampling sites. In a case of stenosis a comparison of two sampling sites is beneficial to compare a blood flow characteristic determined at a sampling site upstream of the stenosis with a blood flow characteristic determined at a sampling site downstream of the stenosis. More generally, when a blood vessel of interest is identified, a plurality of sampling sites may be designated at or near the blood vessel of interest; a time-enhancement curve generated for each of the plurality of sampling sites; a desired blood flow characteristic based on a respective time-enhancement curve determined for each of the plurality of sampling sites; and comparing the determined blood flow characteristic of each of the plurality of sampling sites to determine a blood vessel disorder. Depending on a specific implementation determining of a blood flow characteristic at one or more sampling sites or determining presence of absence of a blood vessel disorder based on a comparison of blood flow characteristic at a plurality of sampling sites can provide a diagnostic result.

A cardiovascular disorder or a blood vessel disorder (may also be referred to as a vascular disorder) assessed by the method or system described herein can be any unhealthy blood flow aberration such as a functionally significant blood flow restriction or blood flow obstruction in a cardiac or non-cardiac blood vessel or any aberrant blood flow in a heart chamber or heart aperture that can compromise health of a subject including for example, unhealthy blood flow aberrations symptomatic of Heart Chamber Abnormalities, Heart Valve Abnormalities (eg., Aortic Valve Disease), Heart Failure, Atherosclerosis (for example, plaque formation), Carotid Artery Disease, Peripheral Artery Disease including Renal Artery Disease, Aneurysm, Raynaud's Phenomenon (Raynaud's Disease or Raynaud's Syndrome), Buerger's Disease, Peripheral Venous Disease and Varicose Veins, Thrombosis and Embolism (for example, blood clots in veins), Blood Clotting Disorders, Ischemia, Angina, Heat Attack, Stroke and Lymphedema.

The 4D blood flow imaging method and system can be used to assess a suspected cardiovascular disorder or blood flow disorder, for example by providing a determination of a blood flow characteristic at a blood vessel of interest identified in a previous medical examination as possible source of an unhealthy blood flow aberration. Additionally, due in part to scan data capturing multiple blood vessels and the reduced time to process scan data, the 4D blood flow imaging method and system may be used in a first instance to proactively assess blood flow in a specific blood vessel or specific group of blood vessels (for example, a pulmonary artery blood flow assessment) and may be implemented as a screening tool to be an initial indicator to identify a source of unhealthy blood flow aberration such as a functionally significant stenosis.

The 4D blood flow imaging method does not require the scanned subject or patient to hold breath during a scan procedure. Breath-hold is an option in some examples. In other examples, motion correction or motion compensation processing of image data may be used for scan data acquired without breath-hold of the subject or patient. If desired, motion correction or motion compensation processing of image data may be used for scan data acquired with breath-hold, if ROIs benefit from adjustment to accommodate the movement of the vessel wall during the cardiac cycle. Rules-based or machine learned motion correction or compensation models may be used as desired for specific implementations.

Embodiments disclosed herein, or portions thereof, can be implemented by programming one or more computer systems or devices with computer-executable instructions embodied in a non-transitory computer-readable medium. When executed by a processor, these instructions operate to cause these computer systems and devices to perform one or more functions particular to embodiments disclosed herein. Programming techniques, computer languages, devices, and computer-readable media necessary to accomplish this are known in the art.

In an example, a non-transitory computer readable medium embodying a computer program for dynamic angiographic imaging may comprise: computer program code for obtaining image data comprising a plurality of corresponding images capturing at least a portion of both an increase phase and a decline phase of a contrast agent in a cardiovasculature of interest; computer program code for generating at least one time-enhancement curve of the contrast agent based on the image data, the time-enhancement curve having an upslope and a downslope; and computer program code for determining a blood flow characteristic in the cardiovasculature of interest based on the time-enhancement curve. In another related example, the image data comprises at least one image capturing the cardiovasculature of interest prior to entry of the contrast agent. In still another related example, the computer readable medium further comprises computer program code for acquiring scan data of the cardiovasculature of interest from a X-ray based scan or a MRI scan, and reconstructing image data based on the scan data.

The computer readable medium is a data storage device that can store data, which can thereafter, be read by a computer system. Examples of a computer readable medium include read-only memory, random-access memory, CD-ROMs, magnetic tape, optical data storage devices and the like. The computer readable medium may be geographically localized or may be distributed over a network coupled computer system so that the computer readable code is stored and executed in a distributed fashion.

Computer-implementation of the system or method typically comprises a memory, an interface and a processor. The types and arrangements of memory, interface and processor may be varied according to implementations. For example, the interface may include a software interface that communicates with an end-user computing device through an Internet connection. The interface may also include a physical electronic device configured to receive requests or queries from a device sending digital and/or analog information. In other examples, the interface can include a physical electronic device configured to receive signals and/or data relating to the 4D blood flow imaging method and system, for example from an imaging scanner or image processing device.

Any suitable processor type may be used depending on a specific implementation, including for example, a microprocessor, a programmable logic controller or a field programmable logic array. Moreover, any conventional computer architecture may be used for computer-implementation of the system or method including for example a memory, a mass storage device, a processor (CPU), a graphical processing unit (GPU), a Read-Only Memory (ROM), and a Random-Access Memory (RAM) generally connected to a system bus of data-processing apparatus. Memory can be implemented as a ROM, RAM, a combination thereof, or simply a general memory unit. Software modules in the form of routines and/or subroutines for carrying out features of the system or method can be stored within memory and then retrieved and processed via processor to perform a particular task or function. Similarly, one or more method steps may be encoded as a program component, stored as executable instructions within memory and then retrieved and processed via a processor. A user input device, such as a keyboard, mouse, or another pointing device, can be connected to PCI (Peripheral Component Interconnect) bus. If desired, the software may provide an environment that represents programs, files, options, and so forth by means of graphically displayed icons, menus, and dialog boxes on a computer monitor screen. For example, any number of blood flow images and blood flow characteristics may be displayed, including for example a time-enhancement curve.

Computer-implementation of the system or method may accommodate any type of end-user computing device including computing devices communicating over a networked connection. The computing device may display graphical interface elements for performing the various functions of the system or method, including for example display of a blood flow characteristic determined for a cardiovasculature of interest. For example, the computing device may be a server, desktop, laptop, notebook, tablet, personal digital assistant (PDA), PDA phone or smartphone, and the like. The computing device may be implemented using any appropriate combination of hardware and/or software configured for wired and/or wireless communication. Communication can occur over a network, for example, where remote control of the system is desired.

If a networked connection is desired the system or method may accommodate any type of network. The network may be a single network or a combination of multiple networks. For example, the network may include the internet and/or one or more intranets, landline networks, wireless networks, and/or other appropriate types of communication networks. In another example, the network may comprise a wireless telecommunications network (e.g., cellular phone network) adapted to communicate with other communication networks, such as the Internet. For example, the network may comprise a computer network that makes use of a TCP/IP protocol (including protocols based on TCP/IP protocol, such as HTTP, HTTPS or FTP).

Embodiments described herein are intended for illustrative purposes without any intended loss of generality. Accordingly, the foregoing detailed description is not intended to limit scope, applicability, or configuration of claimed subject matter.

## Claims

**1.** A computer implemented method for blood flow imaging comprising:

obtaining image data comprising a plurality of corresponding images capturing at least a portion of both an increase phase (56) and a decline phase (58) of a contrast agent in a cardiovasculature of interest;
selecting a target voxel (72) within a lumen of the cardiovasculature of interest in the image data;
generating a time-enhancement curve (76) of the contrast agent in the target voxel based on the image data;
selecting first and second time points from the time-enhancement curve (82);

determining a time rate of change of enhancement between the selected first and second time points based on subtraction of image data at the selected first and second time points (84);
converting the time rate of change of enhancement to a time rate of change of contrast agent mass (85);
determining a blood flow characteristic through the target voxel based on the time rate of change of contrast agent mass.

**2.** The method of claim 1, further comprising determining a baseline value for the time-enhancement curve based on at least one image capturing the cardiovasculature of interest prior to entry of the contrast agent (54).

**3.** The method of claim 2, wherein determining the blood flow characteristic comprises determining an area under the time-enhancement curve and determining a time duration of the contrast agent passing through the target voxel as a time interval that the signal intensity in the time-enhancement curve is greater than the baseline value.

**4.** The method of claim 3, wherein determining the blood flow characteristic comprises determining the density of the contrast agent (88) passing through the target voxel based on the area under the time-enhancement curve and the time duration.

**5.** The method of claim 4, wherein determining the blood flow characteristic comprises determining an absolute flow velocity using Reynolds transport theorem.

**6.** The method of claim 5, wherein determining the blood flow characteristic comprises determining a heart-induced pulsation of the absolute flow velocity.

**7.** The method of claim 4, wherein determining the blood flow characteristic comprises determining a flow acceleration using Reynolds transport theorem, and determining the flow acceleration comprises electrocardiogram-gating during obtaining the image data and wherein the first time point is at a first phase of a cardiac cycle and the second time point is at a second phase of the cardiac cycle.

**8.** The method of claim 5, wherein determining the blood flow characteristic comprises determining a flow pressure by applying Bernoulli's equation.

**9.** A system for blood flow imaging comprising:

a memory for storing image data comprising a plurality of corresponding images capturing at least a portion of both an increase phase (56) and a decline phase (58) of a contrast agent in a cardiovasculature of interest;
a processor configured to generate a time-enhancement curve (76) of the contrast agent in a target voxel based on the image data; determine a time rate of change of enhancement between first and second time points based on subtraction of image data at the first and second time points (84); convert the time rate of change of enhancement to a time rate of change of contrast agent mass (85); and determine a blood flow characteristic through the target voxel based on the time rate of change of contrast agent mass.

**10.** The system of claim 9, wherein the processor is further configured to determine a baseline value for the time-enhancement curve based on at least one image capturing the cardiovasculature of interest prior to entry of the contrast agent (54).

**11.** The system of claim 10, wherein determining the blood flow characteristic comprises determining an area under the time-enhancement curve and determining a time duration of the contrast agent passing through the target voxel as a time interval that the signal intensity in the time-enhancement curve is greater than the baseline value.

**12.** The system of claim 11, wherein determining the blood flow characteristic comprises determining the density of the contrast agent (88) passing through the target voxel based on the area under the time-enhancement curve and the time duration.

**13.** The system of claim 12, wherein determining the blood flow characteristic comprises determining an absolute flow velocity using Reynolds transport theorem.

**14.** The system of claim 13, wherein determining the blood flow characteristic comprises determining a heart-induced pulsation of the absolute flow velocity.

**15.** The system of claim 12, wherein determining the blood flow characteristic comprises determining a flow acceleration using Reynolds transport theorem, and determining the flow acceleration comprises electrocardiogram-gating during obtaining the image data and wherein the first time point is at a first phase of a cardiac cycle and the second time point is at a second phase of the cardiac cycle.

**16.** The system of claim 13, wherein determining the blood flow characteristic comprises determining a flow pressure by applying Bernoulli's equation.

**17.** A non-transitory computer readable medium embodying computer readable code for executing a method for blood flow imaging comprising:

computer readable code for obtaining image data comprising a plurality of corresponding images capturing at least a portion of both an increase phase (56) and a decline phase (58) of a contrast agent in a cardiovasculature of interest;
computer readable code for selecting a target voxel (72) within a lumen of the cardiovasculature of interest in the image data;
computer readable code for generating a time-enhancement curve (76) of the contrast agent in the target voxel based on the image data;
computer readable code for selecting first and second time points from the time-enhancement curve (82);
computer readable code for determining a time rate of change of enhancement between the selected first and second time points based on subtraction of image data at the selected first and second time points (84);
computer readable code for converting the time rate of change of enhancement to a time rate of change of contrast agent mass (85);
computer readable code for determining a blood flow characteristic through the target voxel based on the time rate of change of contrast agent mass.

**18.** The computer readable medium of claim 17, further comprising computer readable code for executing the method of any one of claims 2 to 8.

**Patentansprüche**

**1.** Computerimplementiertes Verfahren zur Blutflussbildgebung, umfassend:

Erhalten von Bilddaten, die eine Vielzahl von entsprechenden Bildern umfassen, die zumindest einen Teil sowohl einer Anstiegsphase (56) als auch einer Abfallphase (58) eines Kontrastmittels in einer Kardiovaskulatur von Interesse erfassen;
Auswählen eines Zielvoxels (72) innerhalb eines Lumens der Kardiovaskulatur von Interesse in den Bilddaten;
Erzeugen einer Zeitverbesserungskurve (76) des Kontrastmittels in dem Zielvoxel basierend auf den Bilddaten;
Auswählen von ersten und zweiten Zeitpunkten aus der Zeitverbesserungskurve (82);
Bestimmen einer Zeitänderungsrate von Verbesserung zwischen den ausgewählten ersten und zweiten Zeitpunkten basierend auf Subtraktion von Bilddaten zu den ausgewählten ersten und zweiten Zeitpunkten (84);
Umwandeln der Zeitänderungsrate von Verbesserung in eine Zeitänderungsrate von Kontrastmittelmasse (85);
Bestimmen einer Blutflusseigenschaft durch das Zielvoxel basierend auf der Zeitänderungsrate von Kontrastmittelmasse.

**2.** Verfahren nach Anspruch 1, ferner umfassend Bestimmen eines Basiswertes für die Zeitverbesserungskurve basierend auf zumindest einem Bild, das die Kardiovaskulatur von Interesse vor Eintritt des Kontrastmittels (54) erfasst.

**3.** Verfahren nach Anspruch 2, wobei das Bestimmen der Blutflusseigenschaft Bestimmen eines Bereichs unter der Zeitverbesserungskurve und Bestimmen einer Zeitdauer des Kontrastmittels, das durch das Zielvoxel verläuft, als ein Zeitintervall umfasst, in dem die Signalintensität in der Zeitverbesserungskurve größer als der Basiswert ist.

**4.** Verfahren nach Anspruch 3, wobei das Bestimmen der Blutflusseigenschaft Bestimmen der Dichte des Kontrastmittels (88), das durch das Zielvoxel verläuft, basierend auf dem Bereich unter der Zeitverbesserungskurve und der Zeitdauer umfasst.

5. Verfahren nach Anspruch 4, wobei das Bestimmen der Blutflusseigenschaft Bestimmen einer absoluten Flussgeschwindigkeit unter Verwendung von Reynolds-Transporttheorem umfasst.

6. Verfahren nach Anspruch 5, wobei das Bestimmen der Blutflusseigenschaft Bestimmen eines herzinduzierten Pulsierens der absoluten Flussgeschwindigkeit umfasst.

7. Verfahren nach Anspruch 4, wobei das Bestimmen der Blutflusseigenschaft Bestimmen einer Flussbeschleunigung unter Verwendung von Reynolds-Transporttheorem umfasst, und das Bestimmen der Flussbeschleunigung Elektrokardiogramm-Gating während des Erhaltens der Bilddaten umfasst und wobei der erste Zeitpunkt in einer ersten Phase eines Herzzyklus ist und der zweite Zeitpunkt in einer zweiten Phase des Herzzyklus ist.

8. Verfahren nach Anspruch 5, wobei das Bestimmen der Blutflusseigenschaft Bestimmen eines Flussdrucks durch Anwenden von Bernoulli-Gleichung umfasst.

9. System zur Blutflussbildgebung, umfassend:

einen Speicher zum Speichern von Bilddaten, die eine Vielzahl von entsprechenden Bildern umfassen, die zumindest einen Teil sowohl einer Anstiegsphase (56) als auch einer Abfallphase (58) eines Kontrastmittels in einer Kardiovaskulatur von Interesse erfassen;
einen Prozessor, der dazu konfiguriert ist, eine Zeitverbesserungskurve (76) des Kontrastmittels in einem Zielvoxel basierend auf den Bilddaten zu erzeugen; eine Zeitänderungsrate von Verbesserung zwischen ersten und zweiten Zeitpunkten basierend auf Subtraktion von Bilddaten zu den ersten und zweiten Zeitpunkten (84) zu bestimmen; die Zeitänderungsrate von Verbesserung in eine Zeitänderungsrate von Kontrastmittelmasse umzuwandeln (85); und eine Blutflusseigenschaft durch das Zielvoxel basierend auf der Zeitänderungsrate von Kontrastmittelmasse zu bestimmen.

10. System nach Anspruch 9, wobei der Prozessor ferner dazu konfiguriert ist, einen Basiswert für die Zeitverbesserungskurve basierend auf zumindest einem Bild zu bestimmen, das die Kardiovaskulatur von Interesse vor Eintritt des Kontrastmittels (54) erfasst.

11. System nach Anspruch 10, wobei das Bestimmen der Blutflusseigenschaft Bestimmen eines Bereichs unter der Zeitverbesserungskurve und Bestimmen einer Zeitdauer des Kontrastmittels, das durch das Zielvoxel verläuft, als ein Zeitintervall umfasst, in dem die Signalintensität in der Zeitverbesserungskurve größer als der Basiswert ist.

12. System nach Anspruch 11, wobei das Bestimmen der Blutflusseigenschaft Bestimmen der Dichte des Kontrastmittels (88), das durch das Zielvoxel verläuft, basierend auf dem Bereich unter der Zeitverbesserungskurve und der Zeitdauer umfasst.

13. System nach Anspruch 12, wobei das Bestimmen der Blutflusseigenschaft Bestimmen einer absoluten Flussgeschwindigkeit unter Verwendung von Reynolds-Transporttheorem umfasst.

14. System nach Anspruch 13, wobei das Bestimmen der Blutflusseigenschaft Bestimmen eines herzinduzierten Pulsierens der absoluten Flussgeschwindigkeit umfasst.

15. System nach Anspruch 12, wobei das Bestimmen der Blutflusseigenschaft Bestimmen einer Flussbeschleunigung unter Verwendung von Reynolds-Transporttheorem umfasst, und das Bestimmen der Flussbeschleunigung Elektrokardiogramm-Gating während des Erhaltens der Bilddaten umfasst, und wobei der erste Zeitpunkt in einer ersten Phase eines Herzzyklus ist und der zweite Zeitpunkt in einer zweiten Phase des Herzzyklus ist.

16. System nach Anspruch 13, wobei das Bestimmen der Blutflusseigenschaft Bestimmen eines Flussdrucks durch Anwenden von Bernoulli-Gleichung umfasst.

17. Nichtflüchtiges computerlesbares Medium, das computerlesbaren Code zum Ausführen eines Verfahrens zur Blutflussbildgebung verkörpert, umfassend:

computerlesbaren Code zum Erhalten von Bilddaten, die eine Vielzahl von entsprechenden Bildern umfassen, die zumindest einen Teil sowohl einer Anstiegsphase (56) als auch einer Abfallphase (58) eines Kontrastmittels in einer Kardiovaskulatur von Interesse erfassen;

computerlesbaren Code zum Auswählen eines Zielvoxels (72) innerhalb eines Lumens der Kardiovaskulatur von Interesse in den Bilddaten;
computerlesbaren Code zum Erzeugen einer Zeitverbesserungskurve (76) des Kontrastmittels in dem Zielvoxel basierend auf den Bilddaten;
computerlesbaren Code zum Auswählen von ersten und zweiten Zeitpunkten aus der Zeitverbesserungskurve (82);
computerlesbaren Code zum Bestimmen einer Zeitänderungsrate von Verbesserung zwischen den ausgewählten ersten und zweiten Zeitpunkten basierend auf Subtraktion von Bilddaten zu den ausgewählten ersten und zweiten Zeitpunkten (84);
computerlesbaren Code zum Umwandeln der Zeitänderungsrate von Verbesserung in eine Zeitänderungsrate von Kontrastmittelmasse (85);
computerlesbaren Code zum Bestimmen einer Blutflusseigenschaft durch das Zielvoxel basierend auf der Zeitänderungsrate von Kontrastmittelmasse.

**18.** Computerlesbares Medium nach Anspruch 17, ferner umfassend computerlesbaren Code zum Ausführen des Verfahrens nach einem der Ansprüche 2 bis 8.

## Revendications

**1.** Procédé mis en œuvre par ordinateur d'imagerie du débit sanguin comprenant :

l'obtention de données d'image comprenant une pluralité d'images correspondantes capturant au moins une partie à la fois d'une phase d'augmentation (56) et d'une phase de déclin (58) d'un agent de contraste dans un système cardiovasculaire d'intérêt ;
la sélection d'un voxel cible (72) dans une lumière du système cardiovasculaire d'intérêt dans les données d'image ;
la génération d'une courbe temps-rehaussement (76) de l'agent de contraste dans le voxel cible sur la base des données d'image ;
la sélection de premier et second points temporels à partir de la courbe temps-rehaussement (82) ;
la détermination d'un taux temporel de variation du rehaussement entre les premier et second points temporels sélectionnés sur la base de la soustraction de données d'image aux premier et second points temporels sélectionnés (84) ;
la conversion du taux temporel de variation du rehaussement en un taux temporel de variation de la masse de l'agent de contraste (85) ;
la détermination d'une caractéristique de débit sanguin à travers le voxel cible sur la base du taux temporel de variation de la masse de l'agent de contraste.

**2.** Procédé selon la revendication 1, comprenant en outre la détermination d'une valeur de ligne de base pour la courbe temps-rehaussement sur la base d'au moins une image capturant le système cardiovasculaire d'intérêt avant l'entrée de l'agent de contraste (54).

**3.** Procédé selon la revendication 2, dans lequel la détermination de la caractéristique de débit sanguin comprend la détermination d'une aire sous la courbe temps-rehaussement et la détermination d'une durée temporelle de passage de l'agent de contraste à travers le voxel cible en tant qu'intervalle de temps pendant lequel l'intensité du signal dans la courbe temps-rehaussement est supérieure à la valeur de base.

**4.** Procédé selon la revendication 3, dans lequel la détermination de la caractéristique de débit sanguin comprend la détermination de la densité de l'agent de contraste (88) passant à traves le voxel cible sur la base de l'aire sous la courbe temps-rehaussement et de la durée temporelle.

**5.** Procédé selon la revendication 4, dans lequel la détermination de la caractéristique de débit sanguin comprend la détermination d'une vitesse d'écoulement absolue en utilisant le théorème de transport de Reynolds.

**6.** Procédé selon la revendication 5, dans lequel la détermination de la caractéristique de débit sanguin comprend la détermination d'une pulsation induite par le cœur de la vitesse d'écoulement absolue.

**7.** Procédé selon la revendication 4, dans lequel la détermination de la caractéristique de débit sanguin comprend la détermination d'une accélération d'écoulement en utilisant le théorème de transport de Reynolds, et la détermination de l'accélération d'écoulement comprend la synchronisation par électrocardiogramme pendant l'obtention des données d'image et dans lequel le premier point temporel se situe à une première phase d'un cycle cardiaque et le second point temporel se situe à une seconde phase du cycle cardiaque.

**8.** Procédé selon la revendication 5, dans lequel la détermination de la caractéristique de débit sanguin comprend la détermination d'une pression de débit en appliquant l'équation de Bernoulli.

**9.** Système d'imagerie du débit sanguin comprenant :

une mémoire pour stocker des données d'image comprenant une pluralité d'images correspondantes capturant au moins une partie à la fois d'une phase d'augmentation (56) et d'une phase de déclin (58) d'un agent de contraste dans un système cardiovasculaire d'intérêt ;
un processeur configuré pour générer une courbe temps-rehaussement (76) de l'agent de contraste dans un voxel cible sur la base des données d'image ; déterminer un taux temporel de variation du rehaussement entre les premier et second points temporels sur la base de la soustraction de données d'image aux premier et second points temporels (84) ; convertir le taux temporel de variation du rehaussement en un taux temporel de variation de la masse de l'agent de contraste (85) ; et déterminer une caractéristique de débit sanguin à travers le voxel cible sur la base du taux temporel de variation de la masse de l'agent de contraste.

**10.** Système selon la revendication 9, dans lequel le processeur est en outre configuré pour déterminer une valeur de ligne de base pour la courbe temps-rehaussement sur la base d'au moins une image capturant le système cardiovasculaire d'intérêt avant l'entrée de l'agent de contraste (54).

**11.** Système selon la revendication 10, dans lequel la détermination de la caractéristique de débit sanguin comprend la détermination d'une aire sous la courbe temps-rehaussement et la détermination d'une durée temporelle du passage de l'agent de contraste à travers le voxel cible en tant qu'intervalle de temps pendant lequel l'intensité du signal dans la courbe temps-rehaussement est supérieure à la valeur de base.

**12.** Système selon la revendication 11, dans lequel la détermination de la caractéristique de débit sanguin comprend la détermination de la densité de l'agent de contraste (88) passant à travers le voxel cible sur la base de l'aire sous la courbe temps-rehaussement et de la durée temporelle.

**13.** Système selon la revendication 12, dans lequel la détermination de la caractéristique de débit sanguin comprend la détermination d'une vitesse d'écoulement absolue en utilisant le théorème de transport de Reynolds.

**14.** Système selon la revendication 13, dans lequel la détermination de la caractéristique de débit sanguin comprend la détermination d'une pulsation induite par le cœur de la vitesse d'écoulement absolue.

**15.** Système selon la revendication 12, dans lequel la détermination de la caractéristique de débit sanguin comprend la détermination d'une accélération d'écoulement en utilisant le théorème de transport de Reynolds, et la détermination de l'accélération d'écoulement comprend la synchronisation par électrocardiogramme pendant l'obtention des données d'image et dans lequel le premier point temporel se situe à une première phase d'un cycle cardiaque et le second point temporel se situe à une seconde phase du cycle cardiaque.

**16.** Système selon la revendication 13, dans lequel la détermination de la caractéristique de débit sanguin comprend la détermination d'une pression de débit en appliquant l'équation de Bernoulli.

**17.** Support non transitoire lisible par ordinateur incorporant un code lisible par ordinateur pour exécuter un procédé d'imagerie du débit sanguin comprenant :

un code lisible par ordinateur pour obtenir des données d'image comprenant une pluralité d'images correspondantes capturant au moins une partie à la fois d'une phase d'augmentation (56) et d'une phase de déclin (58) d'un agent de contraste dans un système cardiovasculaire d'intérêt ;
un code lisible par ordinateur pour sélectionner un voxel cible (72) dans une lumière du système cardiovasculaire d'intérêt dans les données d'image ;
un code lisible par ordinateur pour générer une courbe temps-rehaussement (76) de l'agent de contraste dans le voxel cible sur la base des données d'image ;

un code lisible par ordinateur pour sélectionner des premier et second points temporels à partir de la courbe temps-rehaussement (82) ;
un code lisible par ordinateur pour déterminer un taux temporel de variation du rehaussement entre les premier et second points temporels sélectionnés sur la base de la soustraction de données d'image aux premier et second points temporels sélectionnés (84) ;
un code lisible par ordinateur pour convertir le taux temporel de variation du rehaussement en un taux temporel de variation de la masse de l'agent de contraste (85) ;
un code lisible par ordinateur pour déterminer une caractéristique de débit sanguin à travers le voxel cible sur la base du taux temporel de variation de la masse de l'agent de contraste.

**18.** Support lisible par ordinateur selon la revendication 17, comprenant en outre un code lisible par ordinateur pour exécuter le procédé selon l'une quelconque des revendications 2 à 8.

FIGURE 1

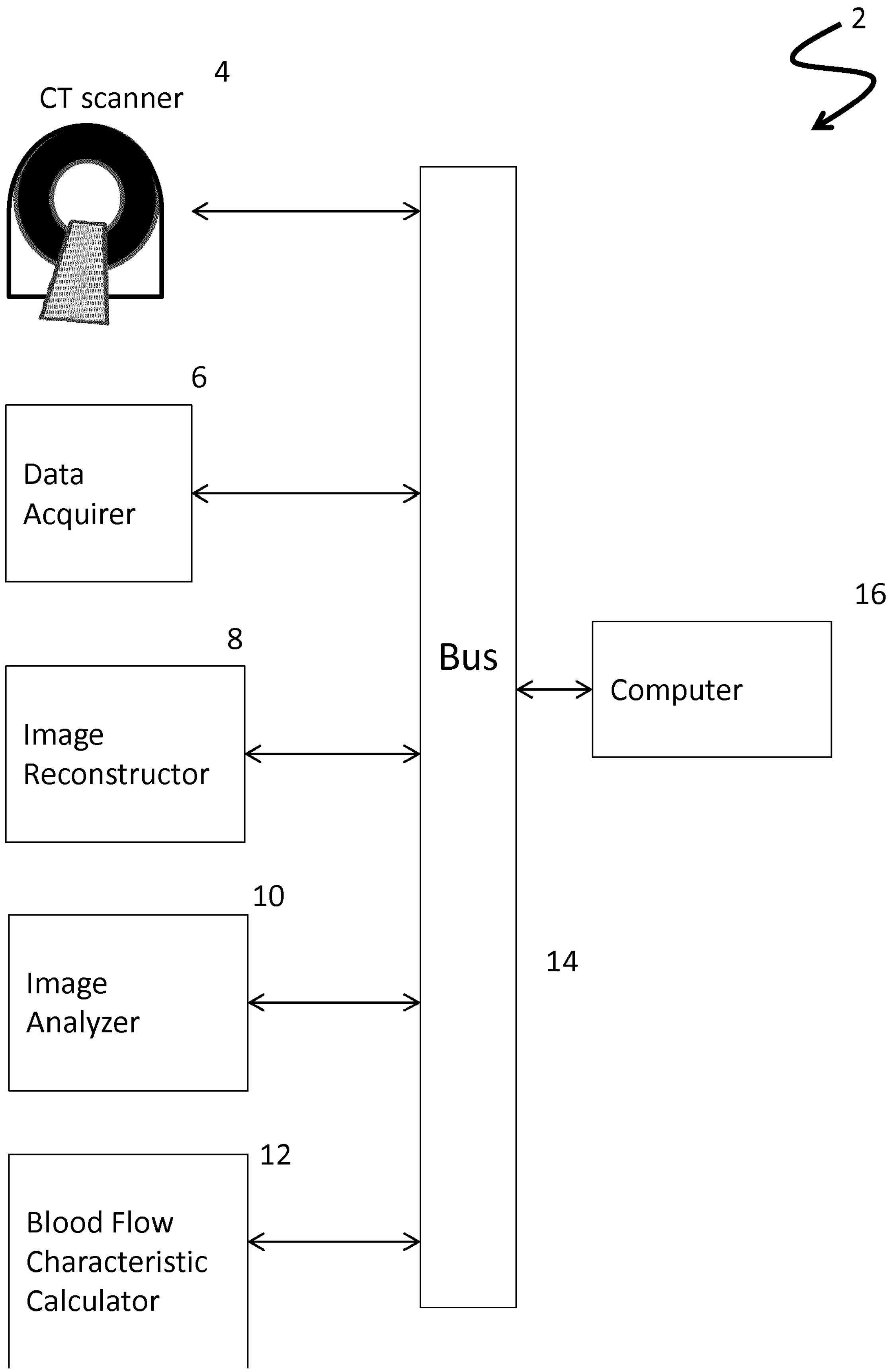
2
4
CT scanner
6
Data Acquirer
8
Image Reconstructor
10
Image Analyzer
12
Blood Flow Characteristic Calculator
Bus
14
16
Computer

FIGURE 2

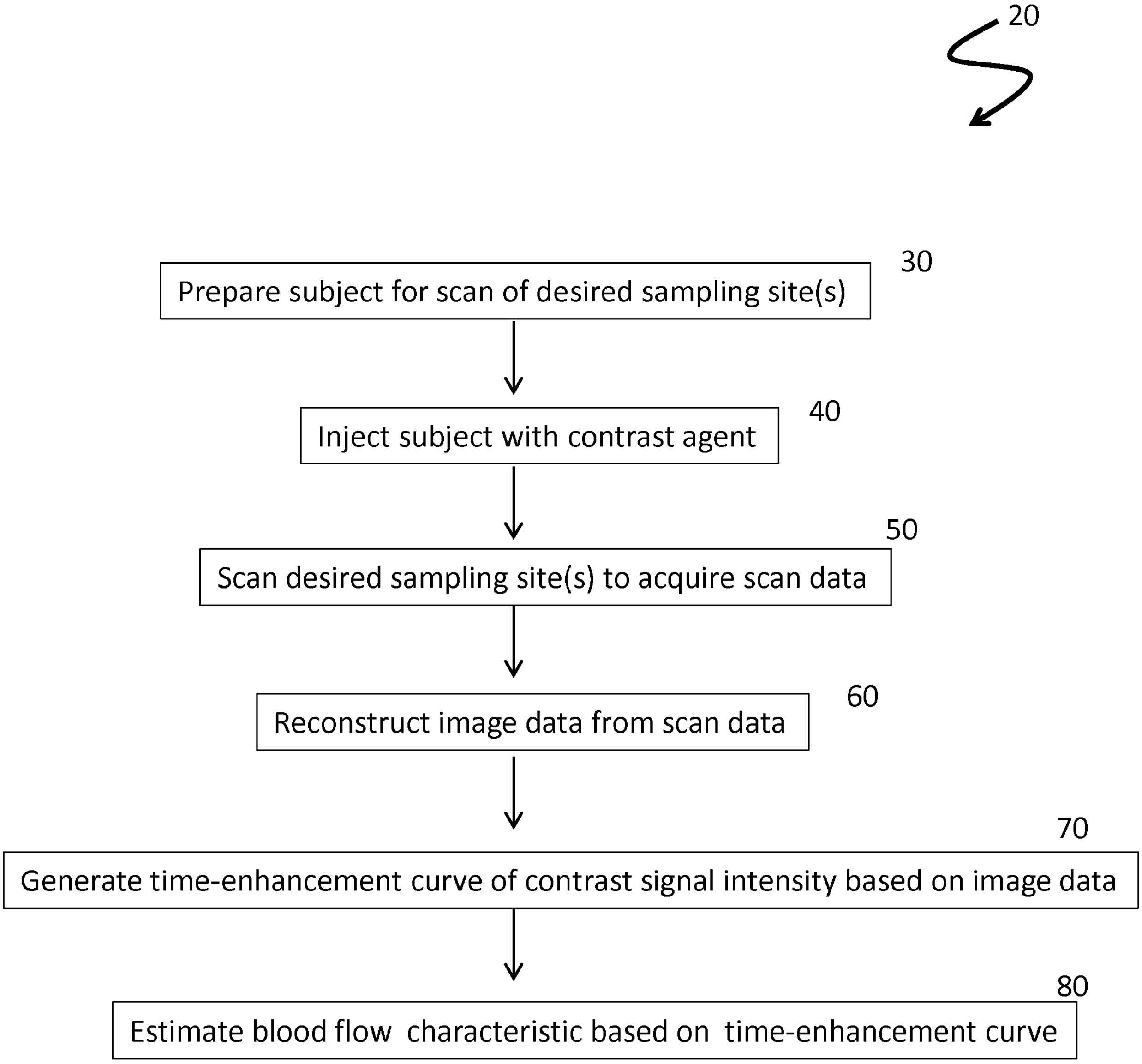
20
30
Prepare subject for scan of desired sampling site(s)
40
Inject subject with contrast agent
50
Scan desired sampling site(s) to acquire scan data
60
Reconstruct image data from scan data
70
Generate time-enhancement curve of contrast signal intensity based on image data
80
Estimate blood flow characteristic based on time-enhancement curve

FIGURE 3

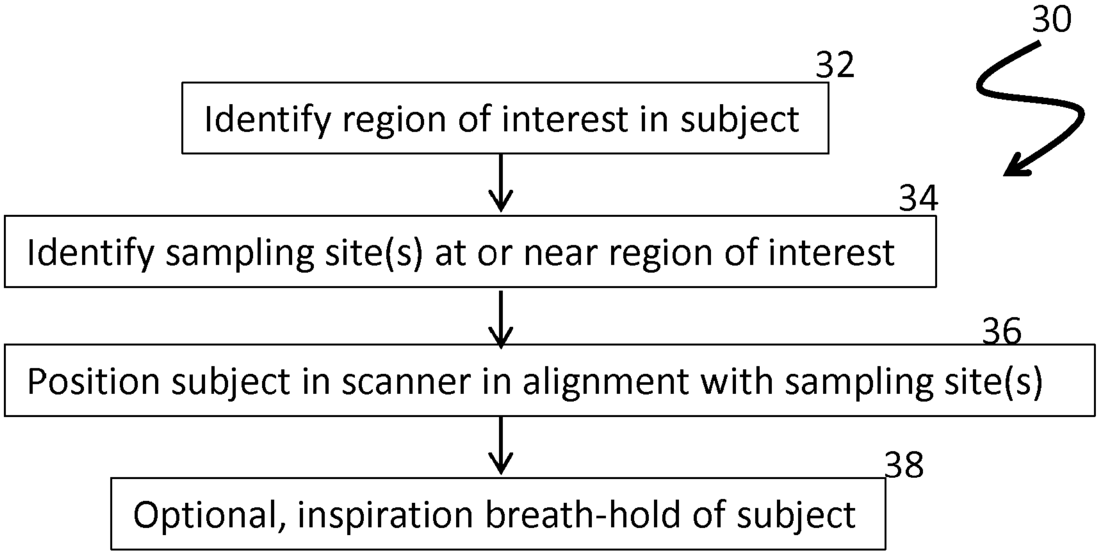


FIGURE 4

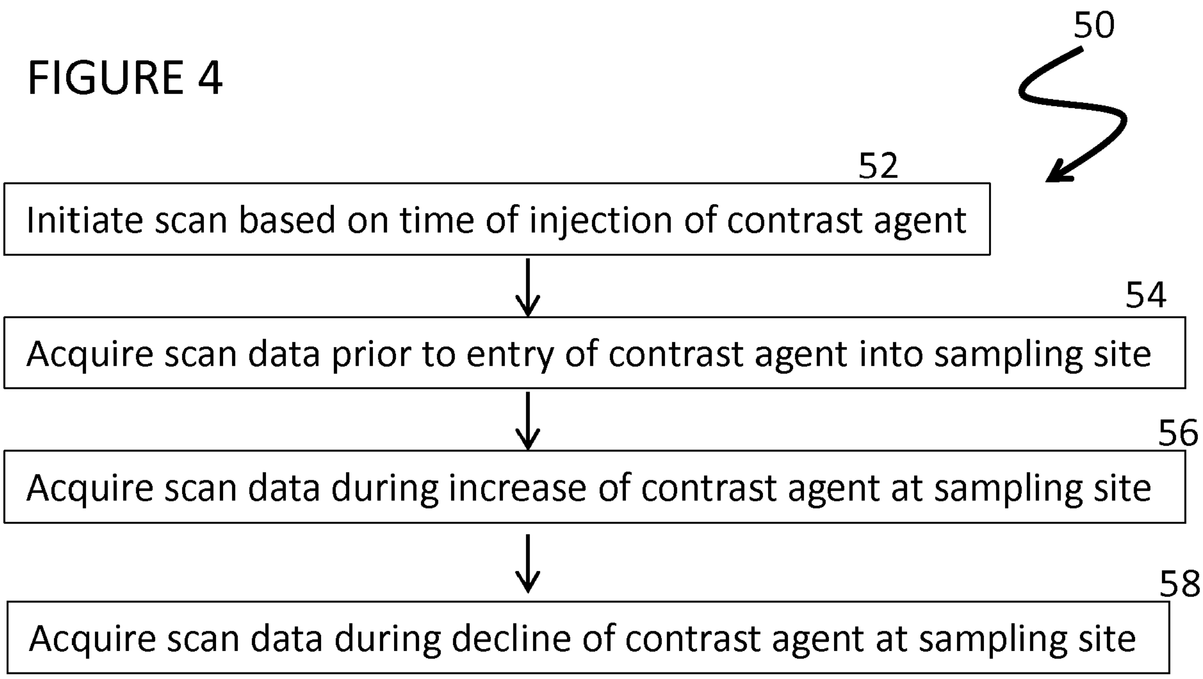

FIGURE 5

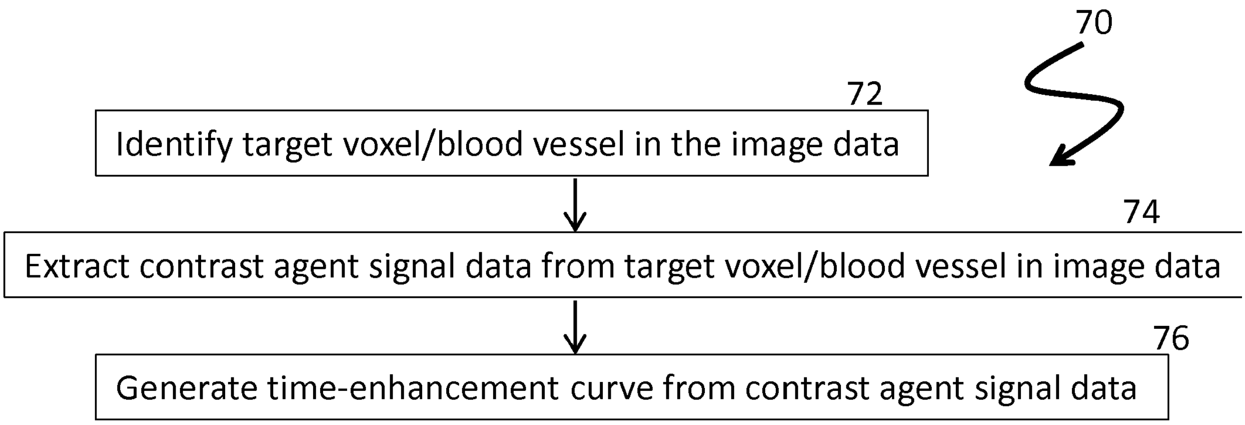


FIGURE 6

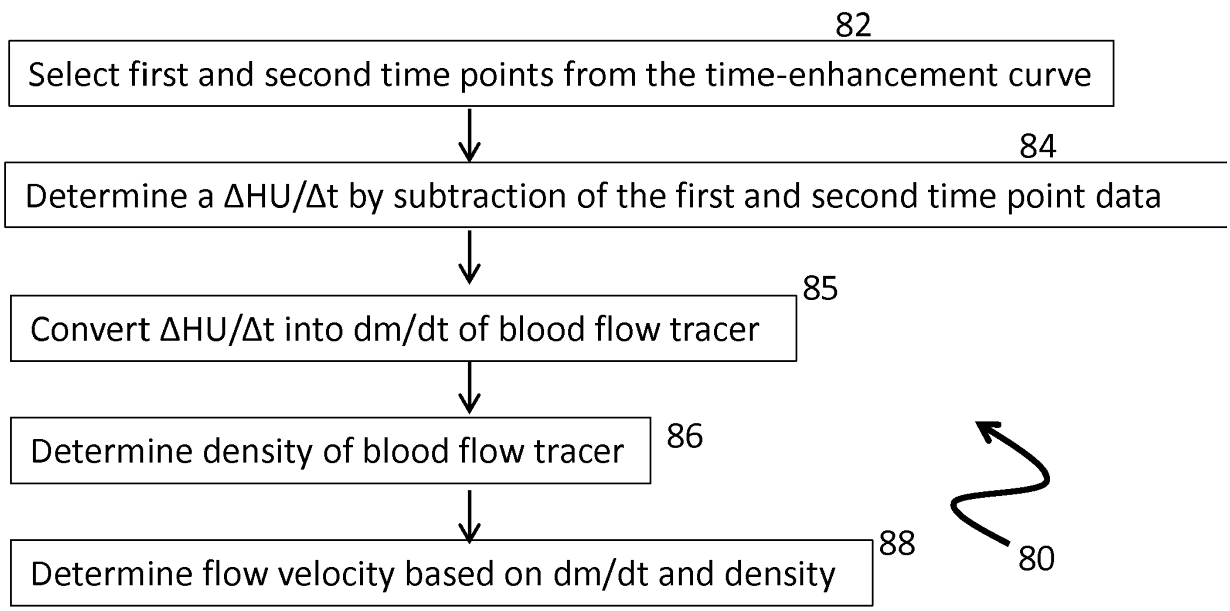

FIGURE 7A

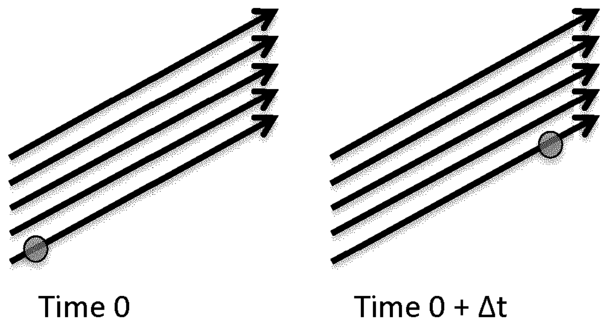


FIGURE 7B

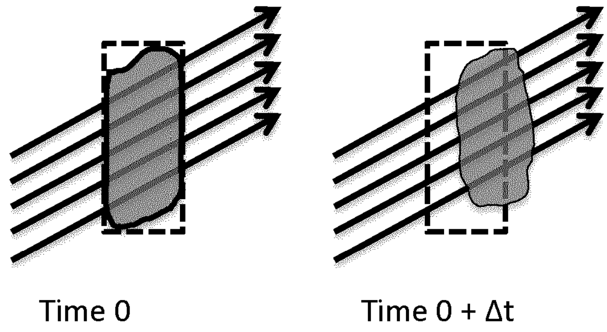


FIGURE 8A

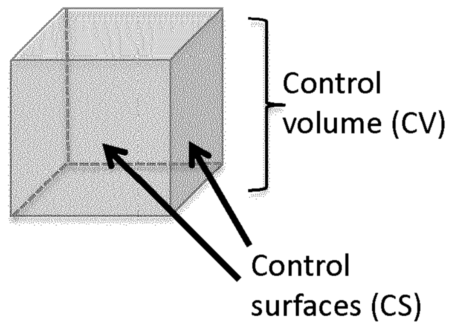


FIGURE 8B

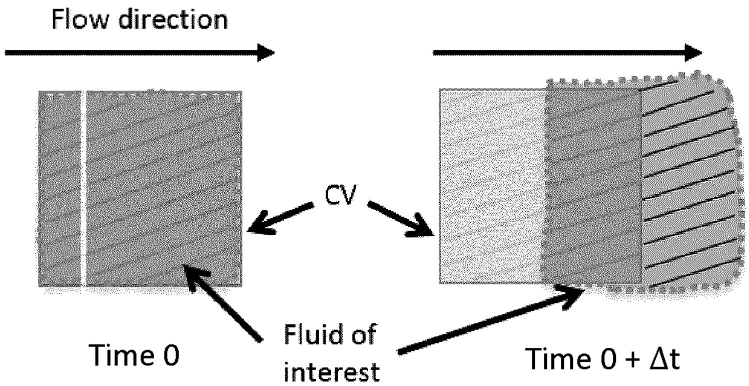


FIGURE 9A

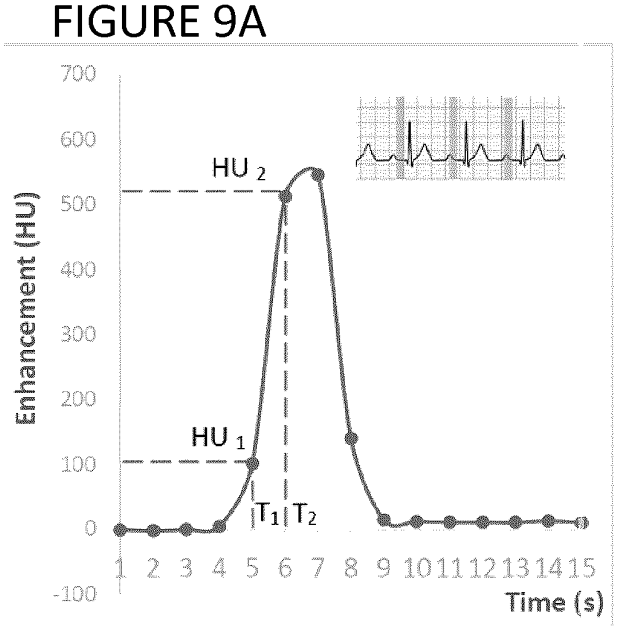


FIGURE 9B

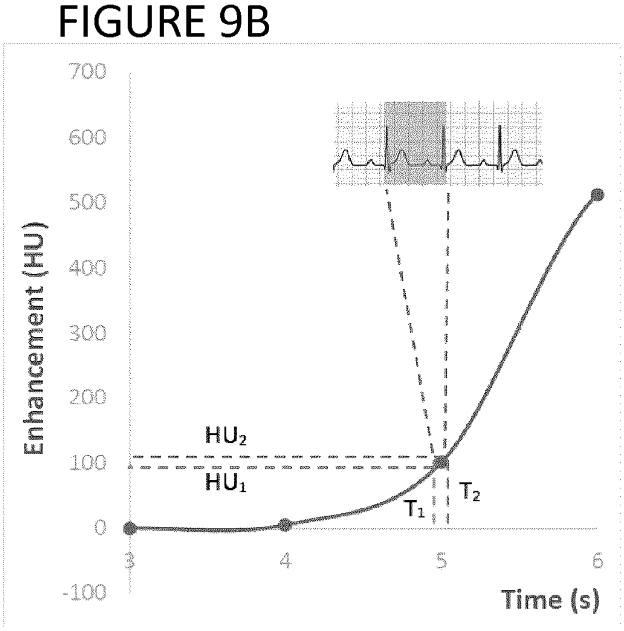

FIGURE 10A

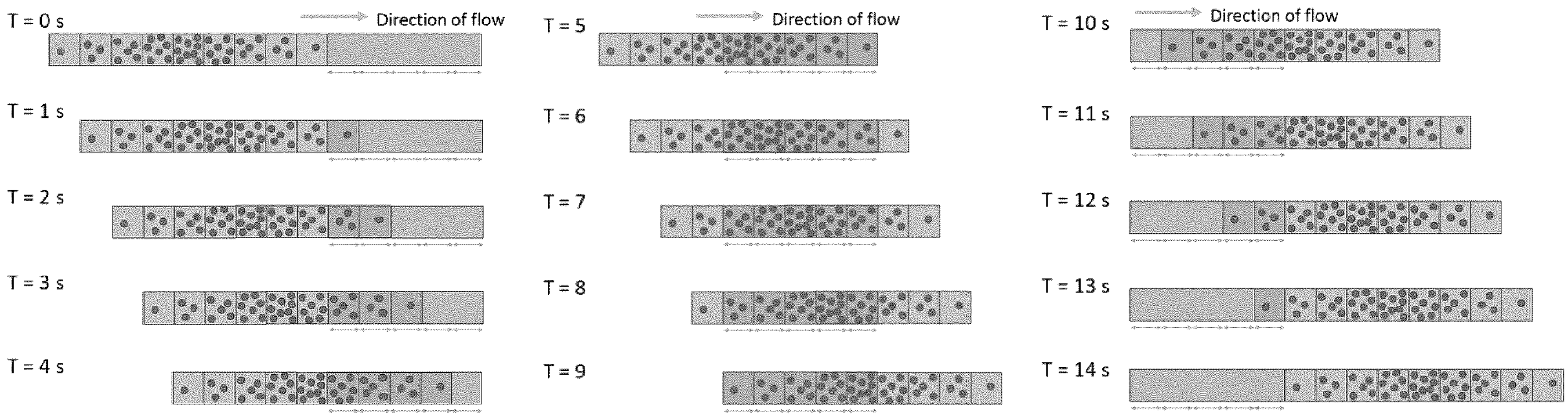


FIGURE 10B

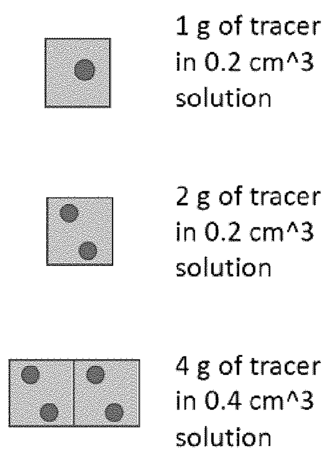


FIGURE 10C

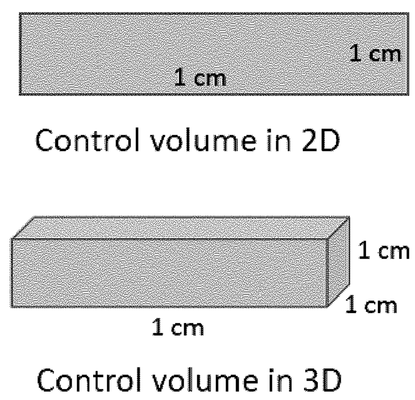


FIGURE 10D

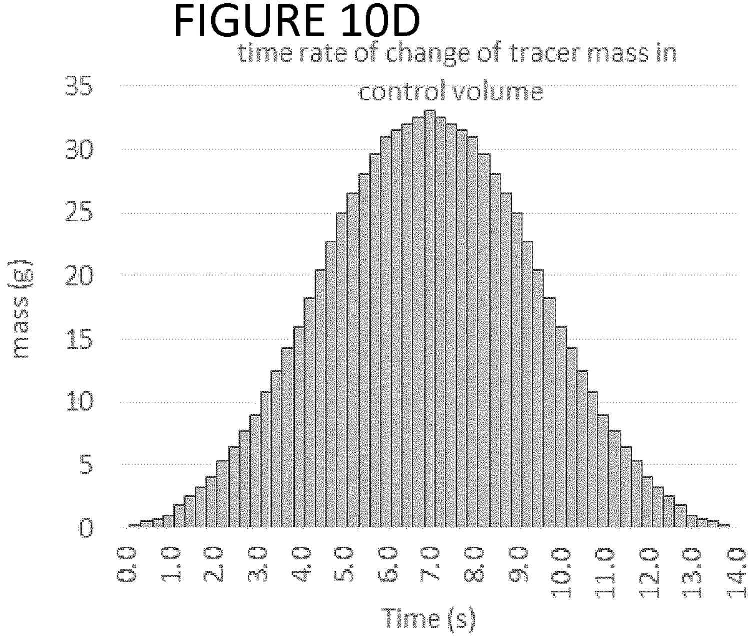

FIGURE 11A

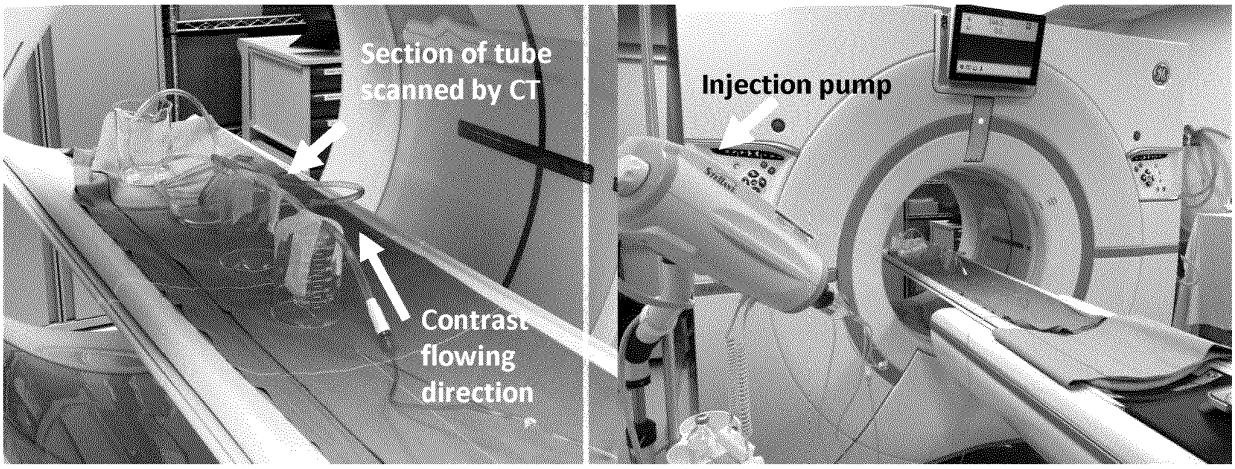


FIGURE 11B

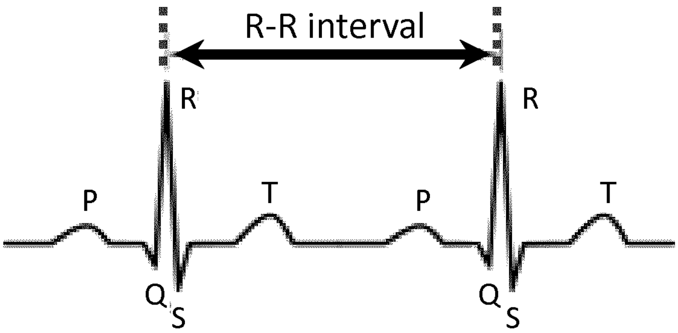


FIGURE 12

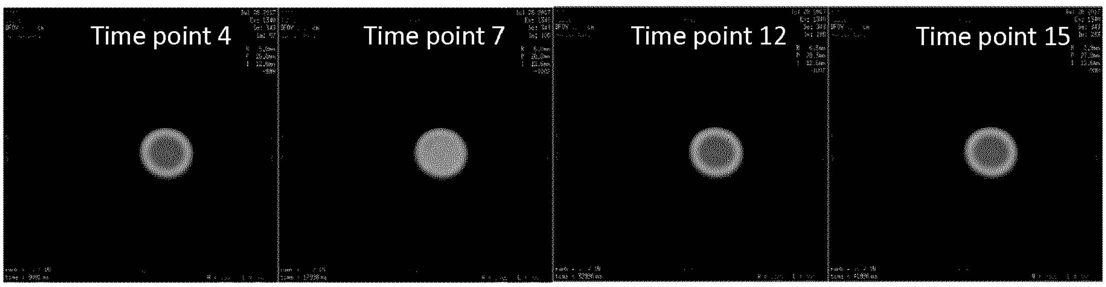

FIGURE 13A

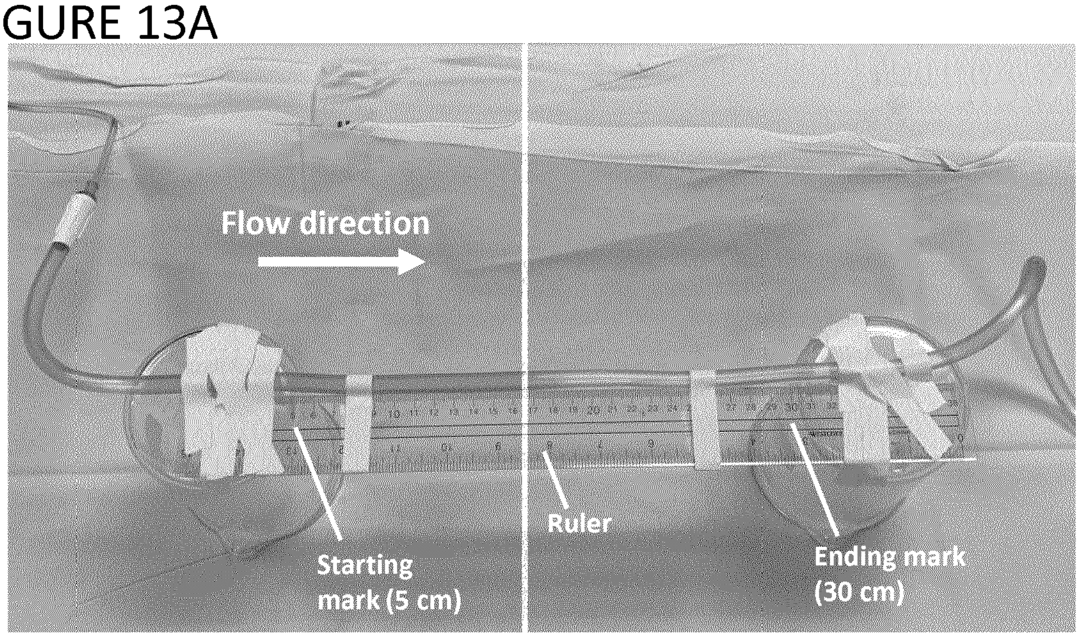


FIGURE 13B

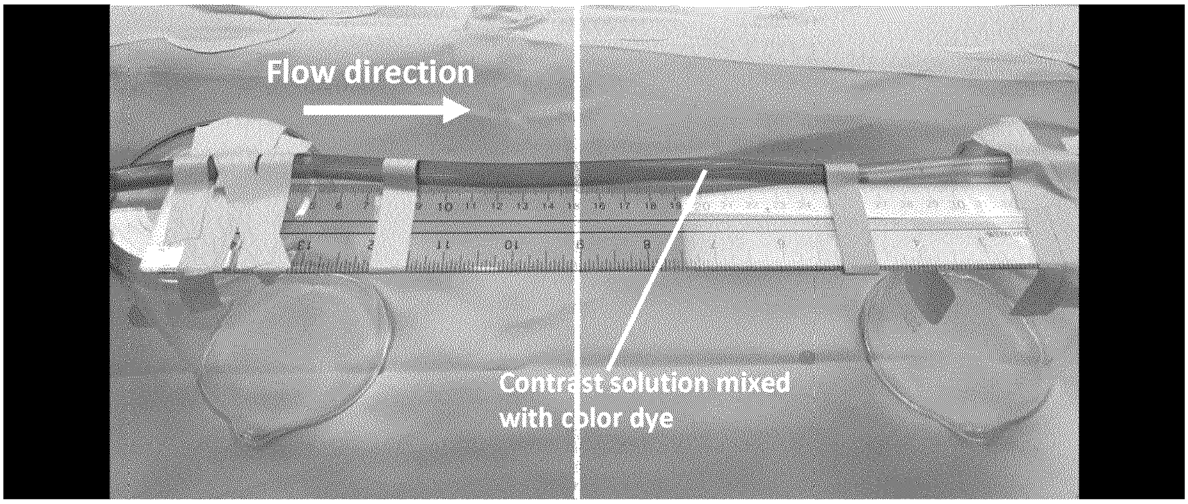

FIGURE 14A

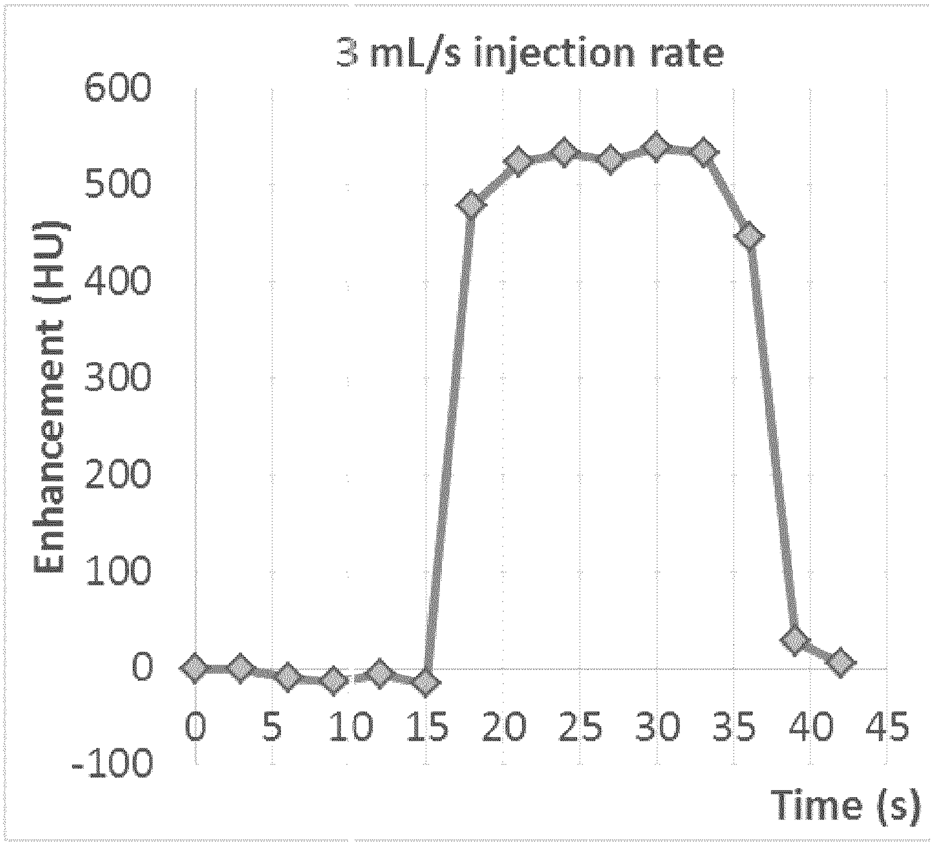


FIGURE 14B

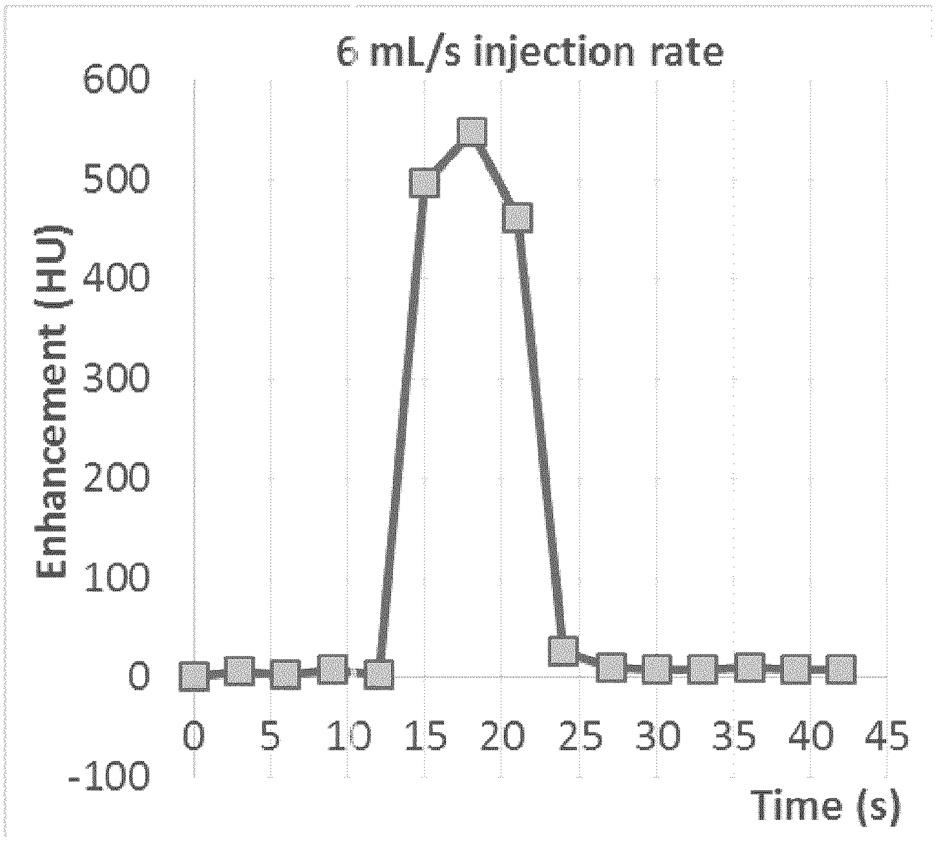

FIGURE 15

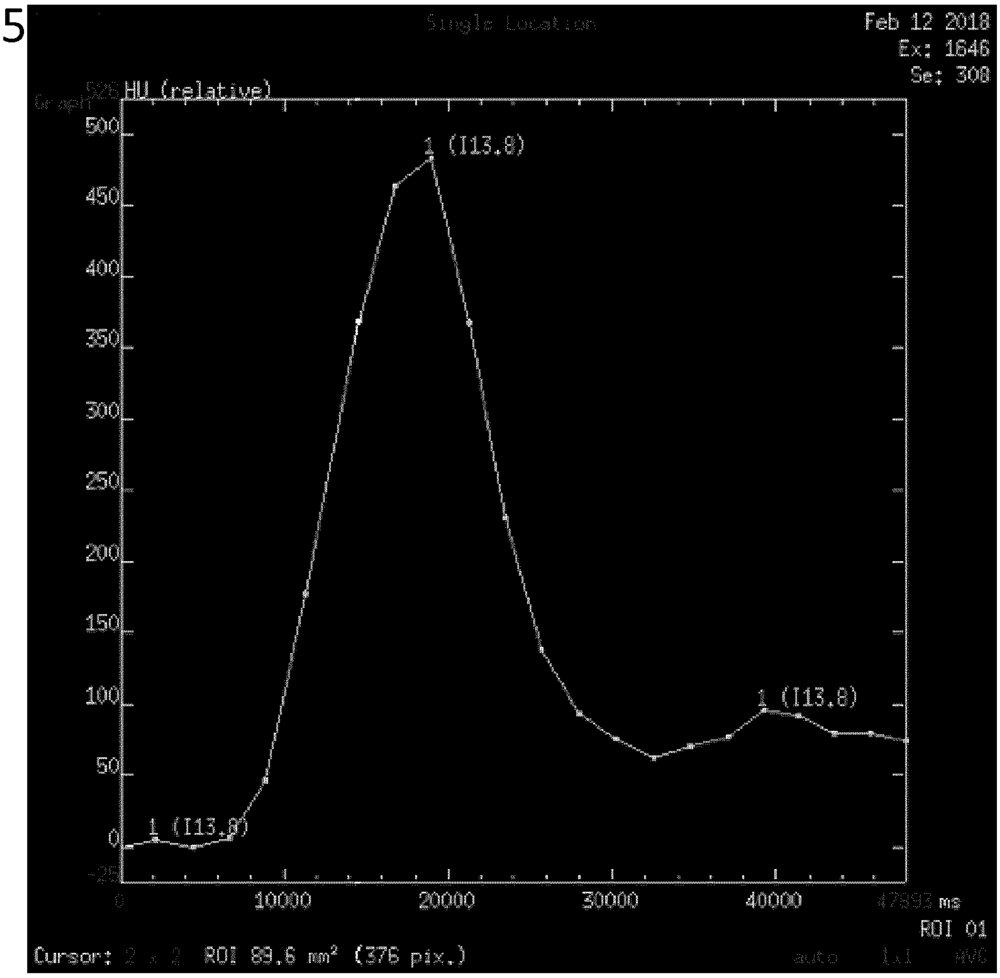


FIGURE 16

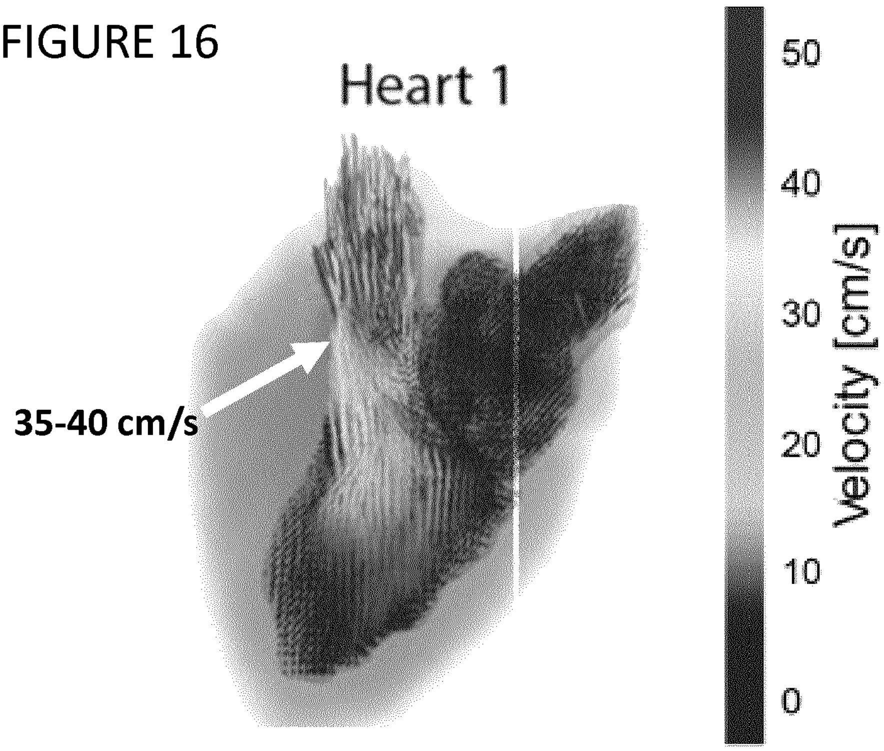

FIGURE 17A

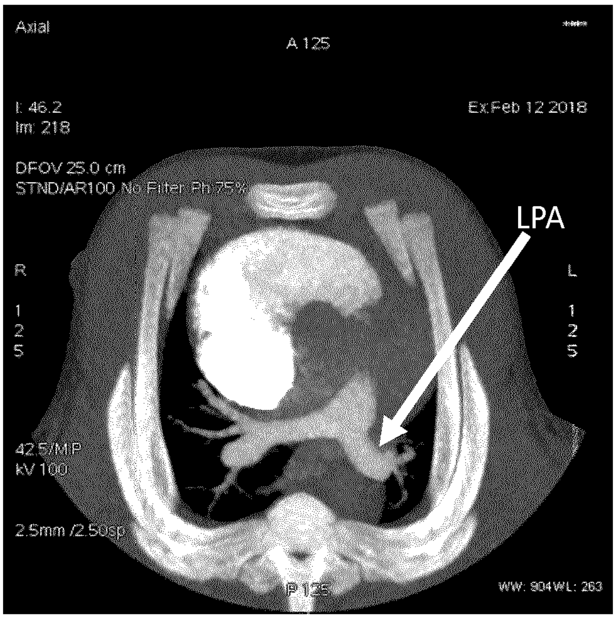


FIGURE 17B

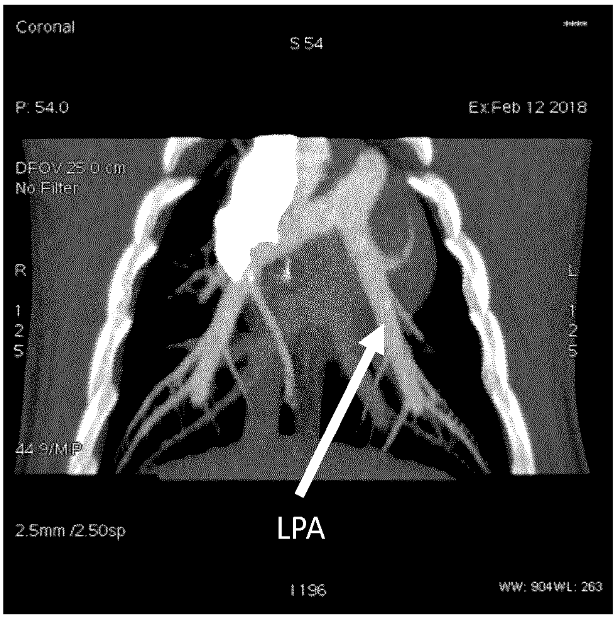


FIGURE 17C

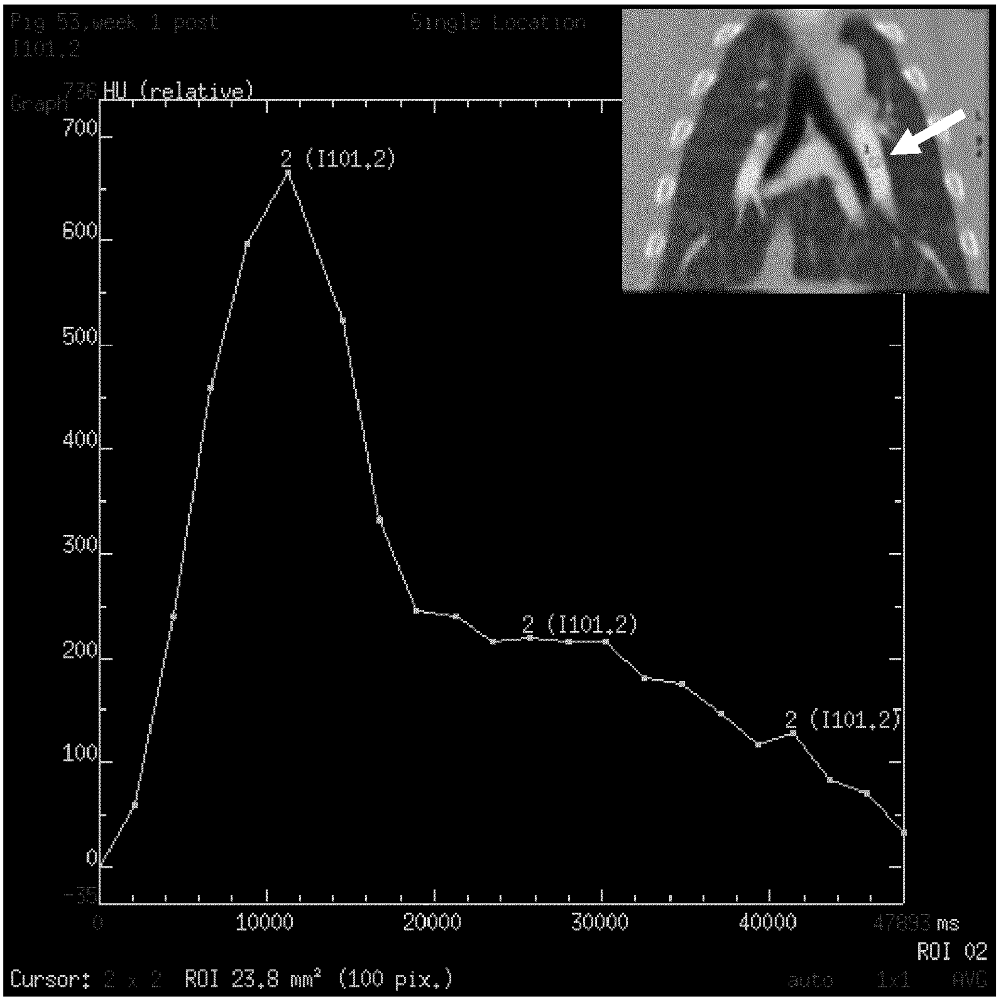

FIGURE 18A

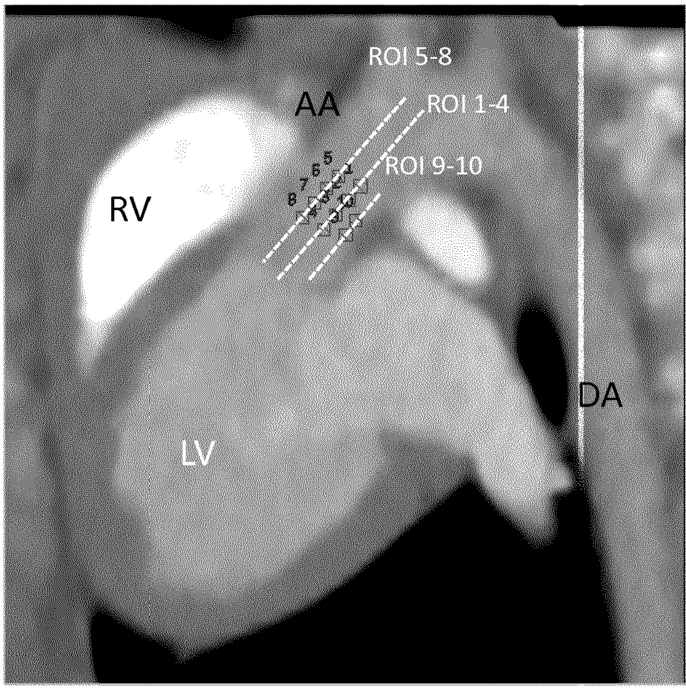


FIGURE 19

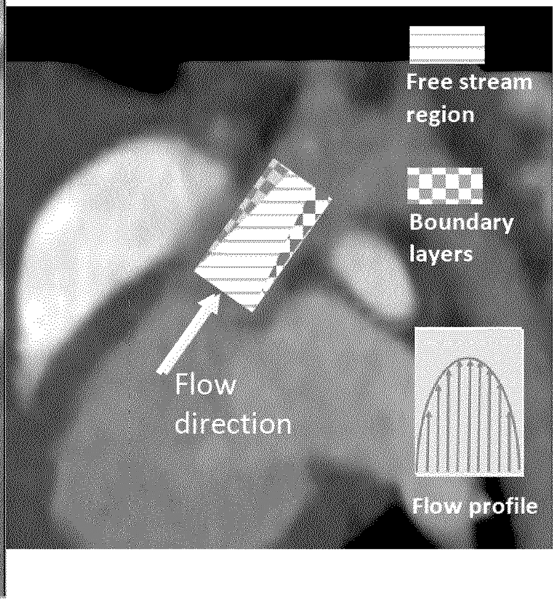


FIGURE 18B

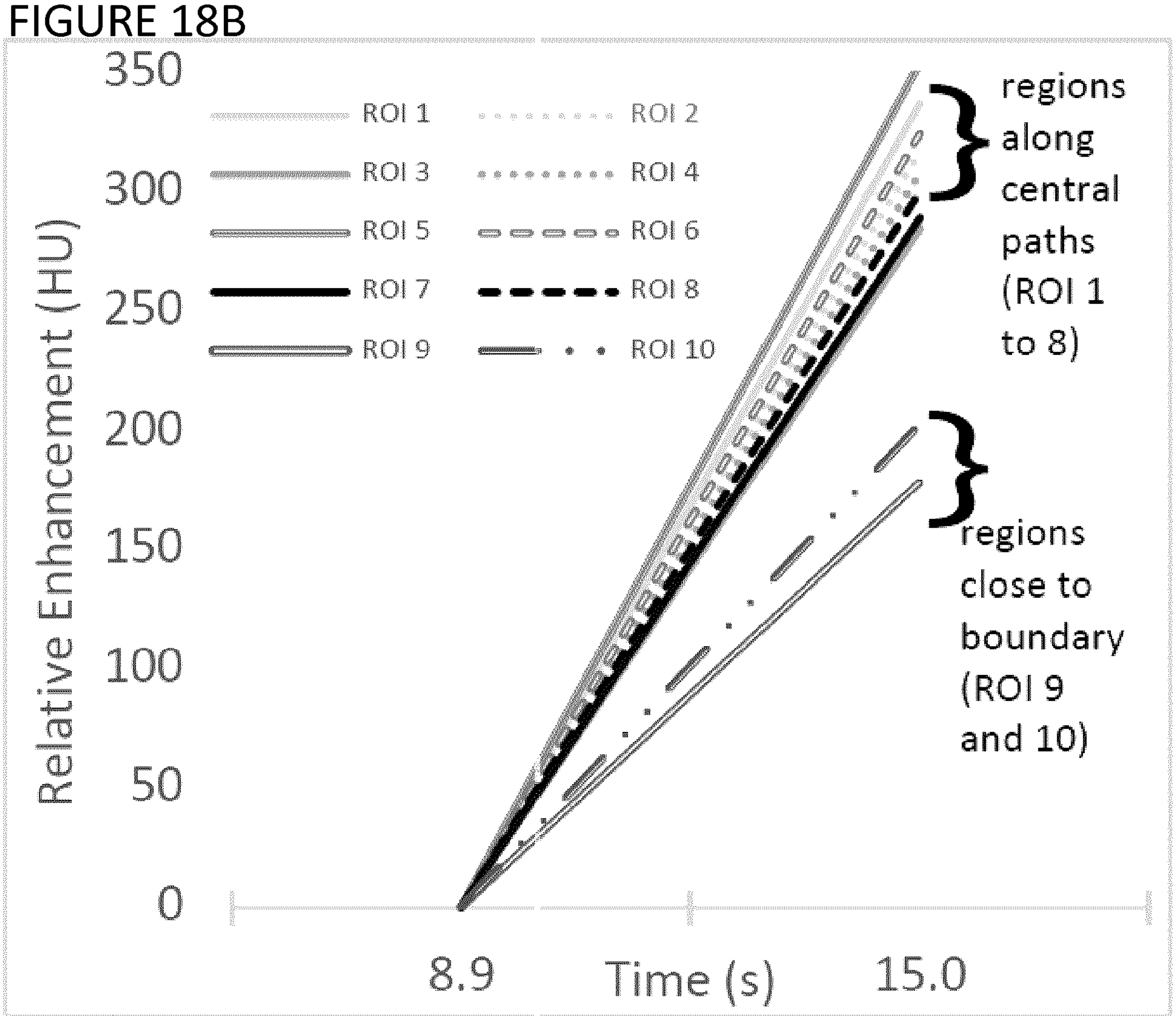

FIGURE 20

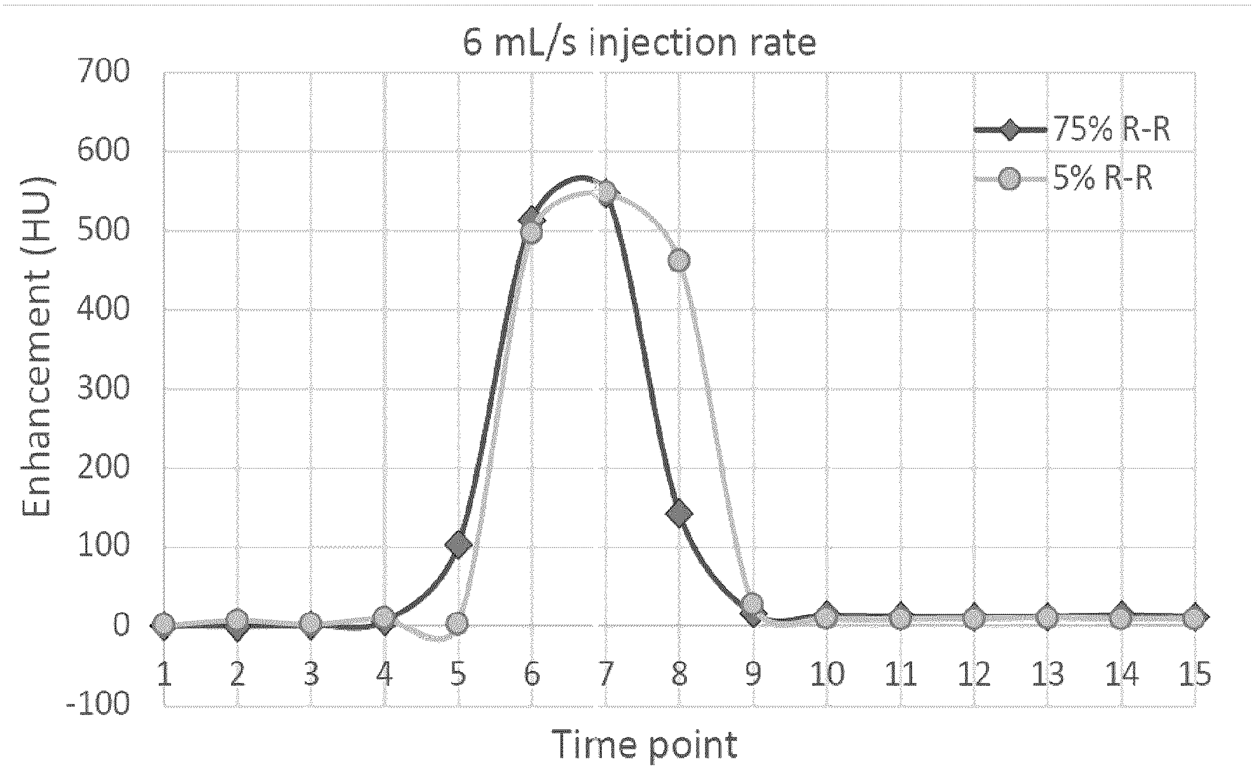


FIGURE 21

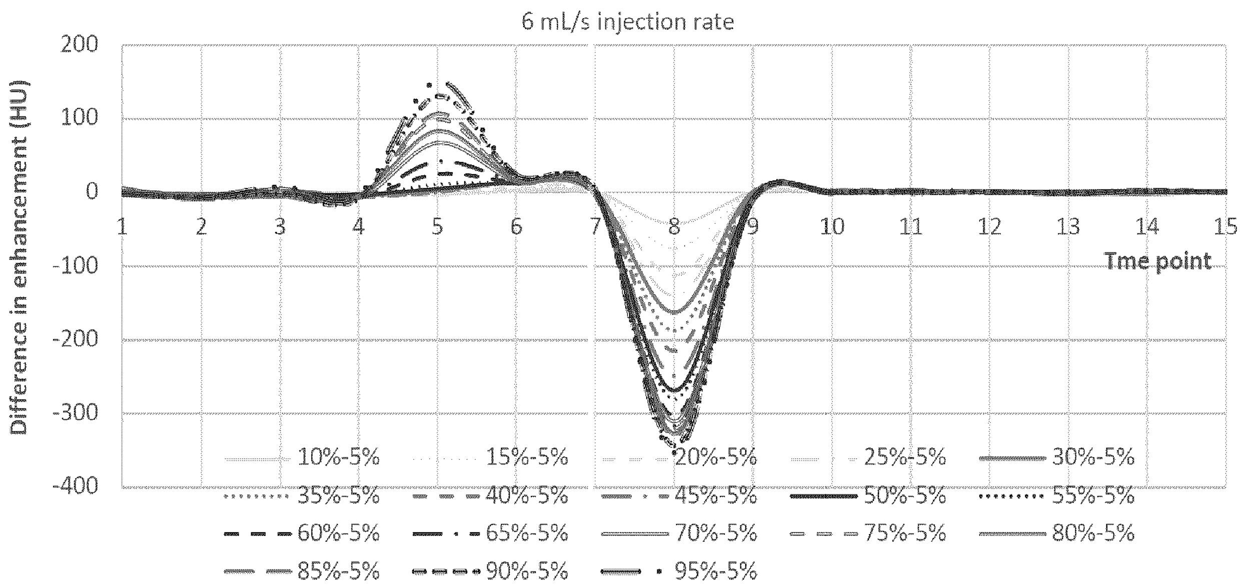

FIGURE 22

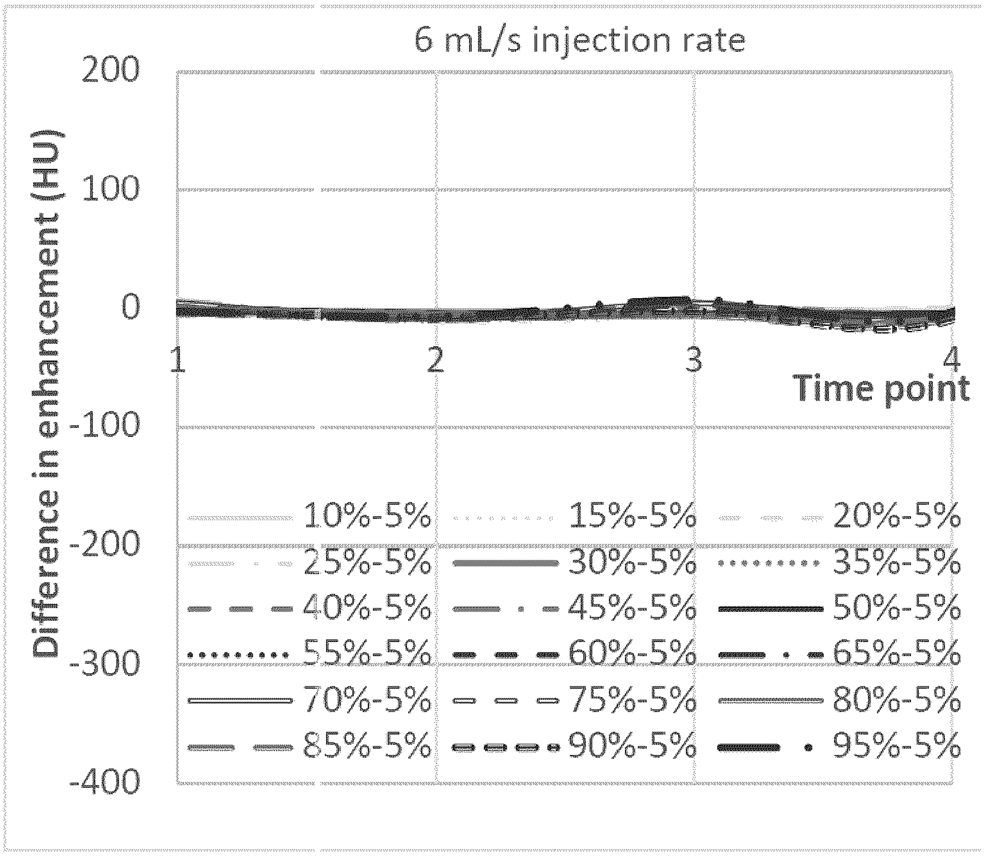


FIGURE 23

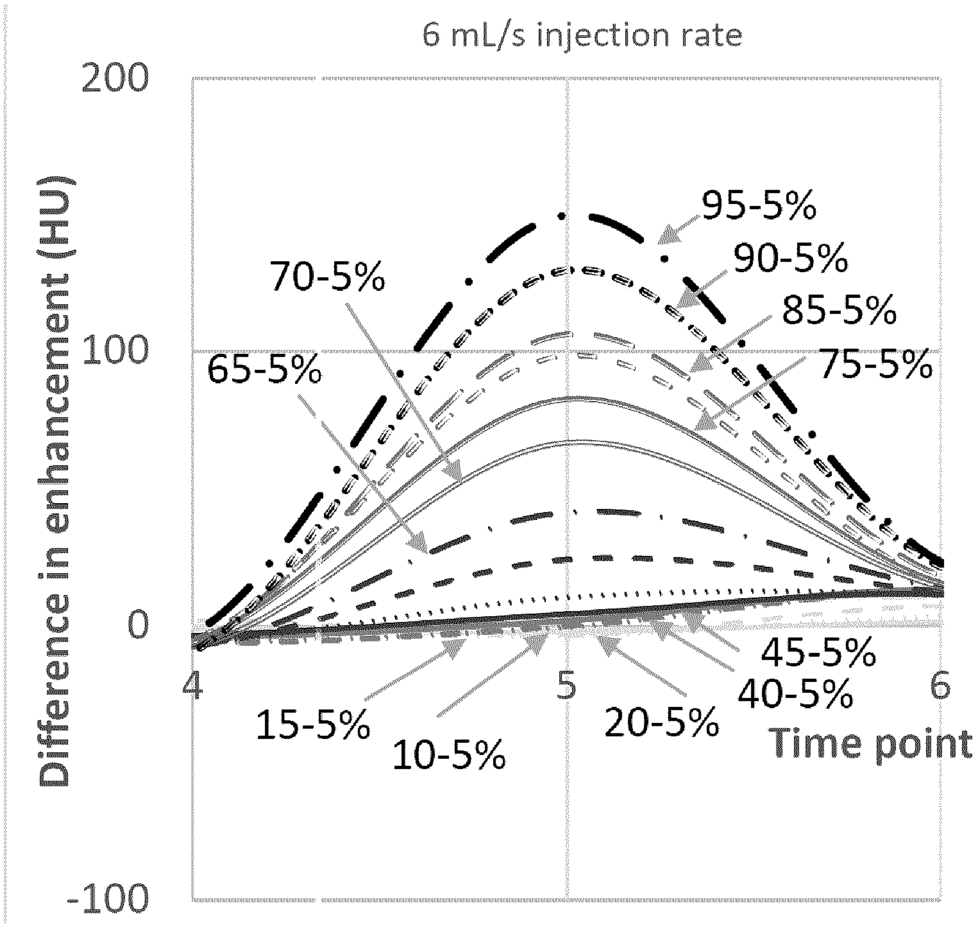

FIGURE 24A

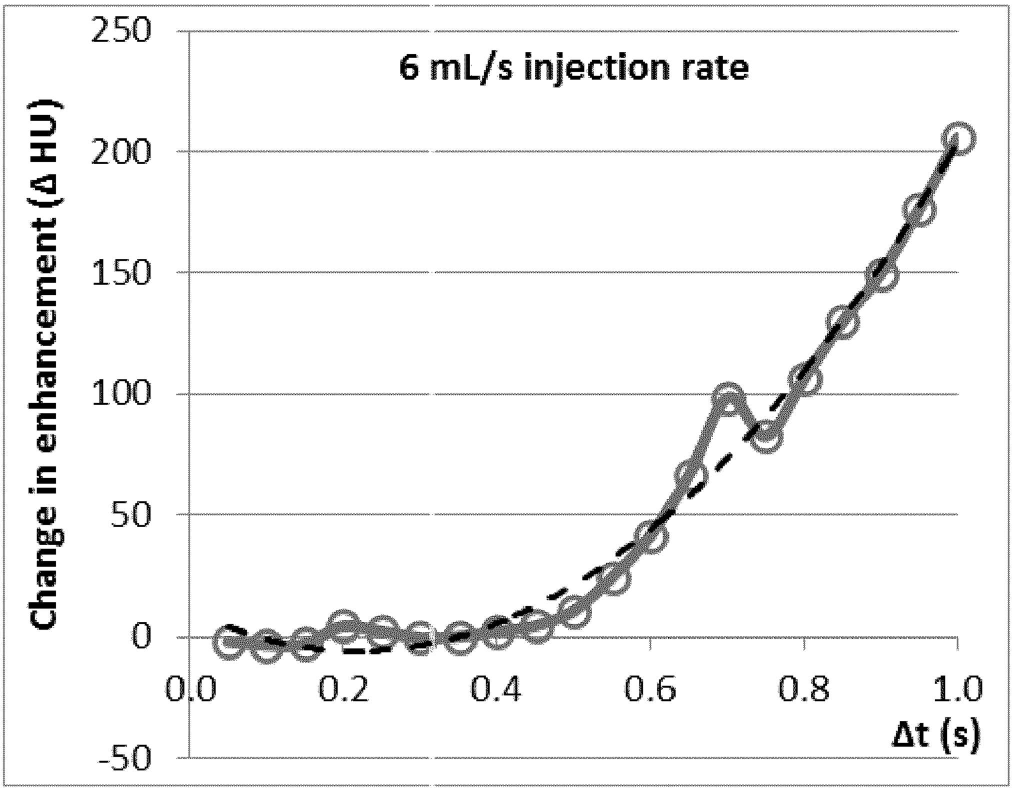


FIGURE 24B

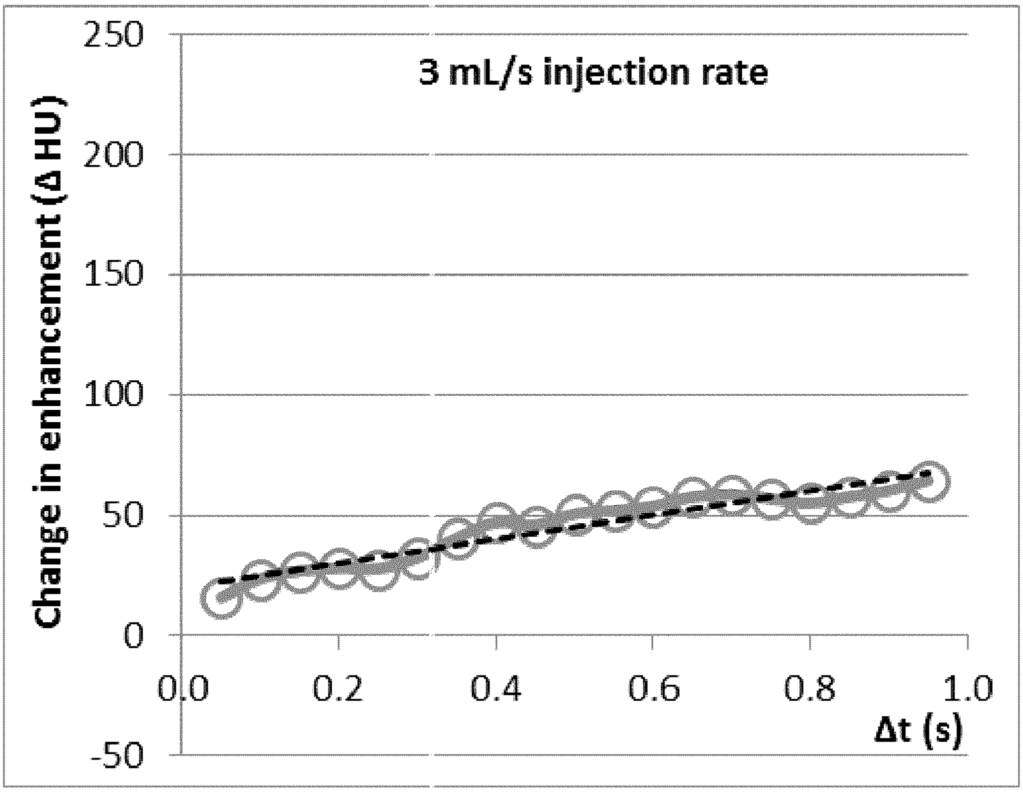

FIGURE 25A

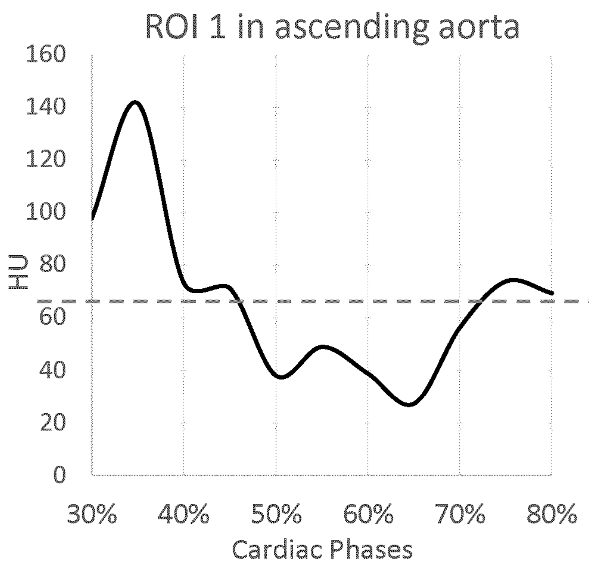


FIGURE 25B

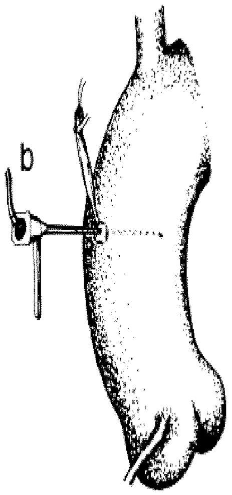


FIGURE 25C

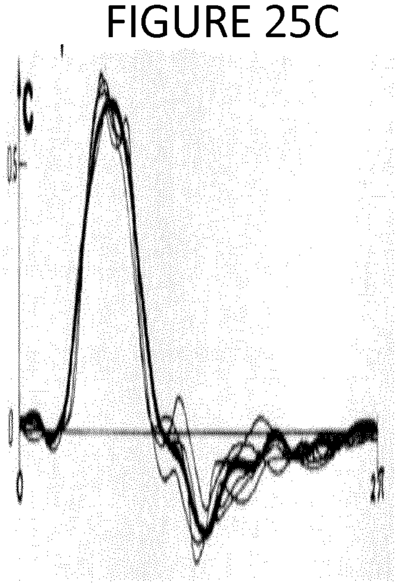


FIGURE 26A

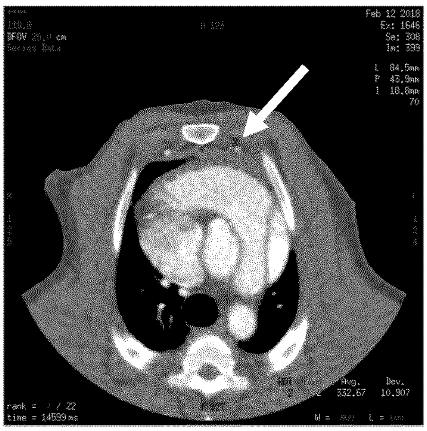

FIGURE 26B

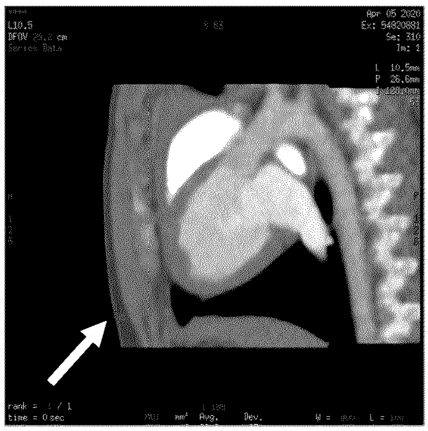

FIGURE 26C

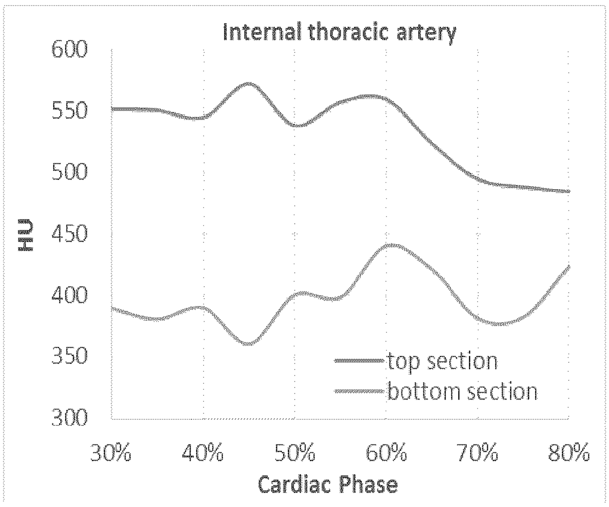


FIGURE 26D

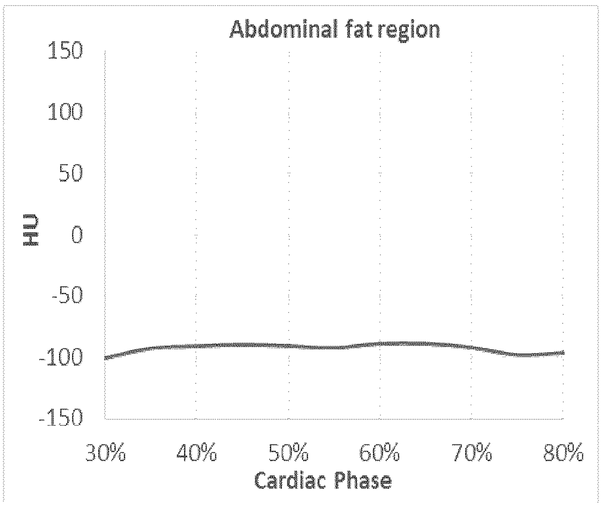

FIGURE 27A

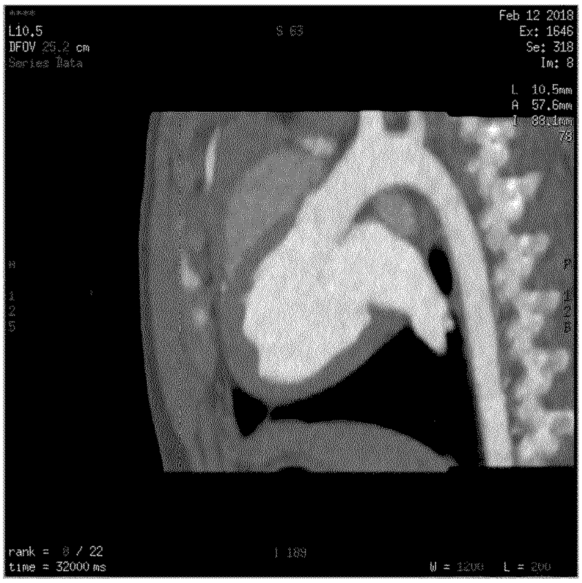

FIGURE 27B

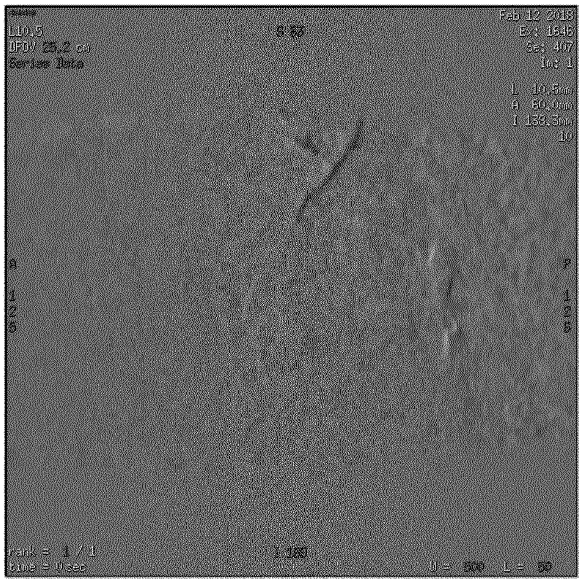

FIGURE 28A

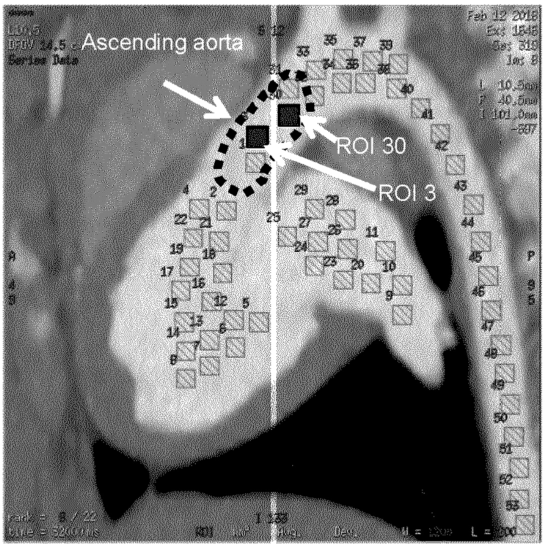


FIGURE 28B

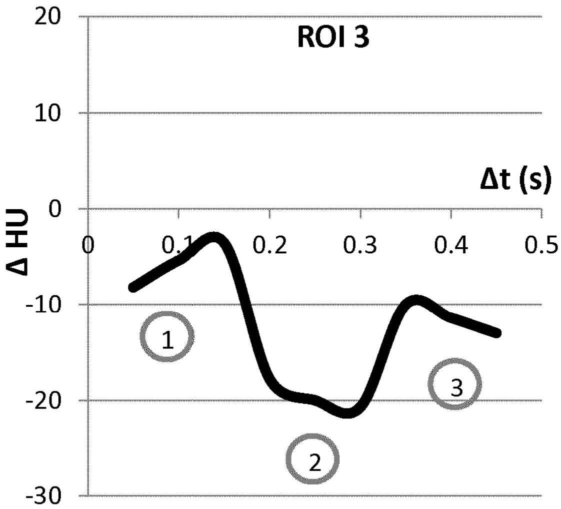


FIGURE 28C

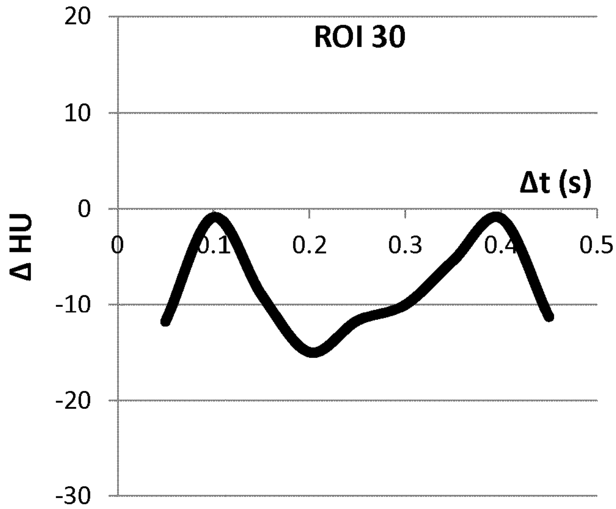

FIGURE 29A

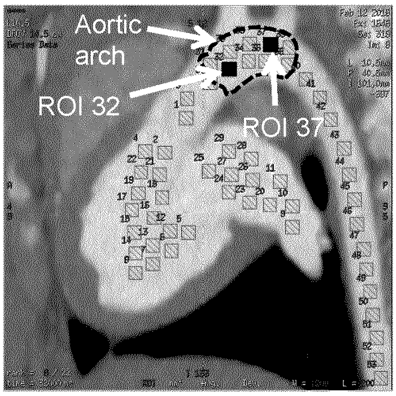


FIGURE 29B

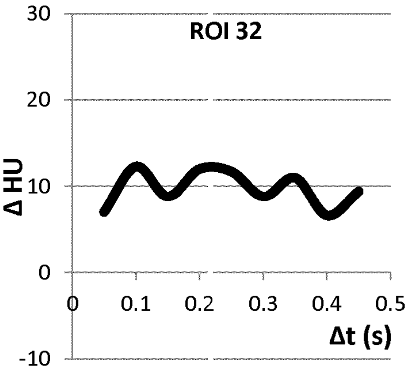


FIGURE 29C

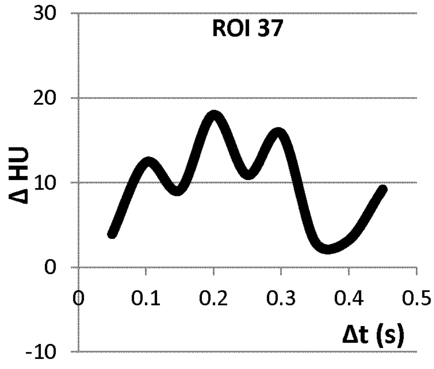


FIGURE 30A

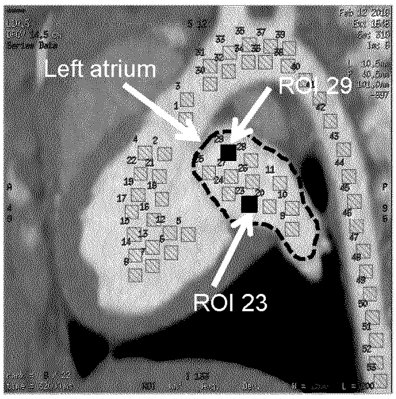


FIGURE 30B

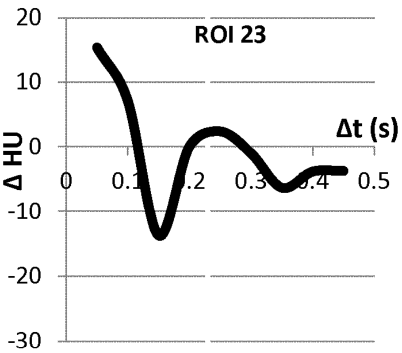


FIGURE 30C

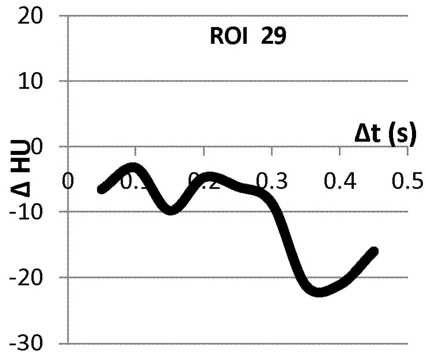


FIGURE 31A

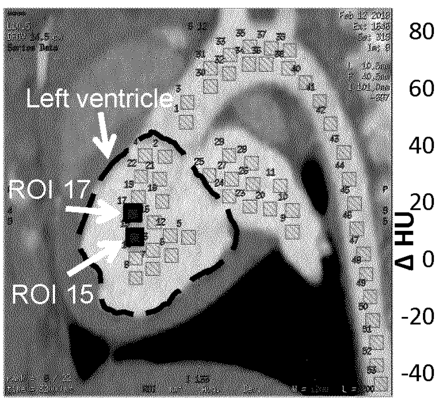


FIGURE 31B

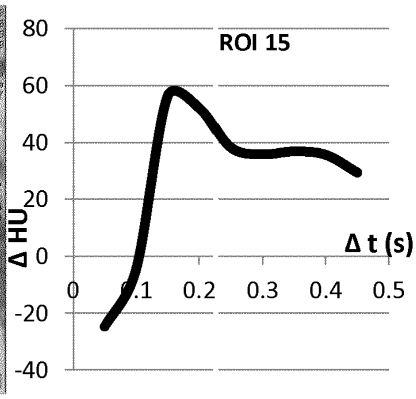


FIGURE 31C

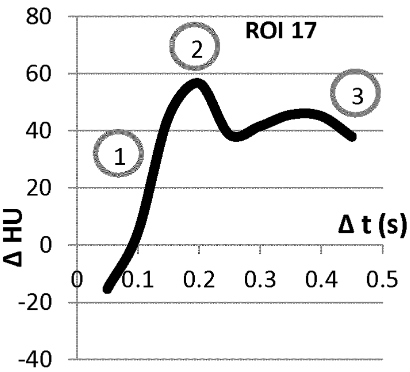

FIGURE 32A

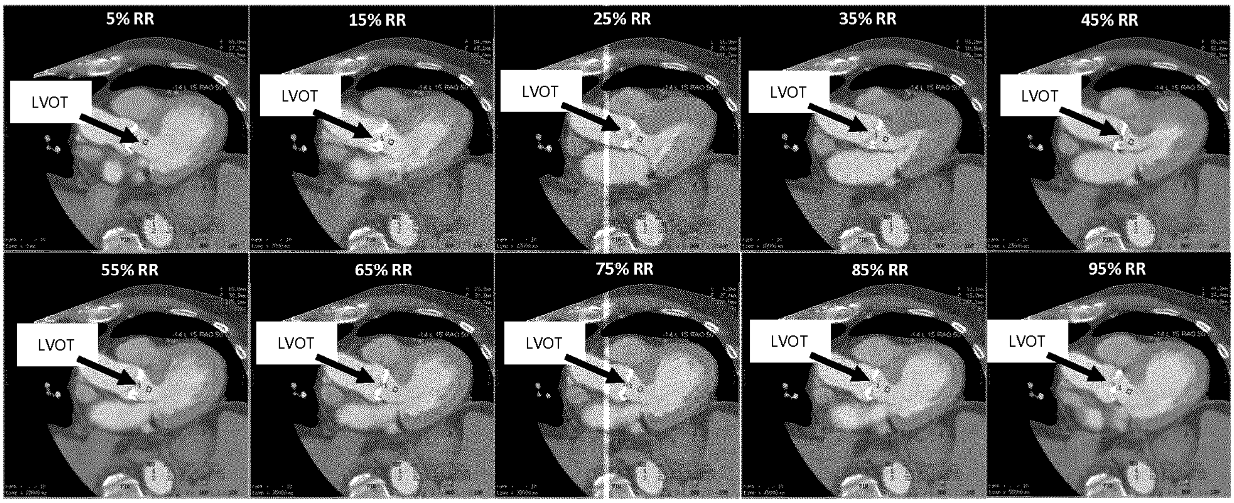


FIGURE 32B

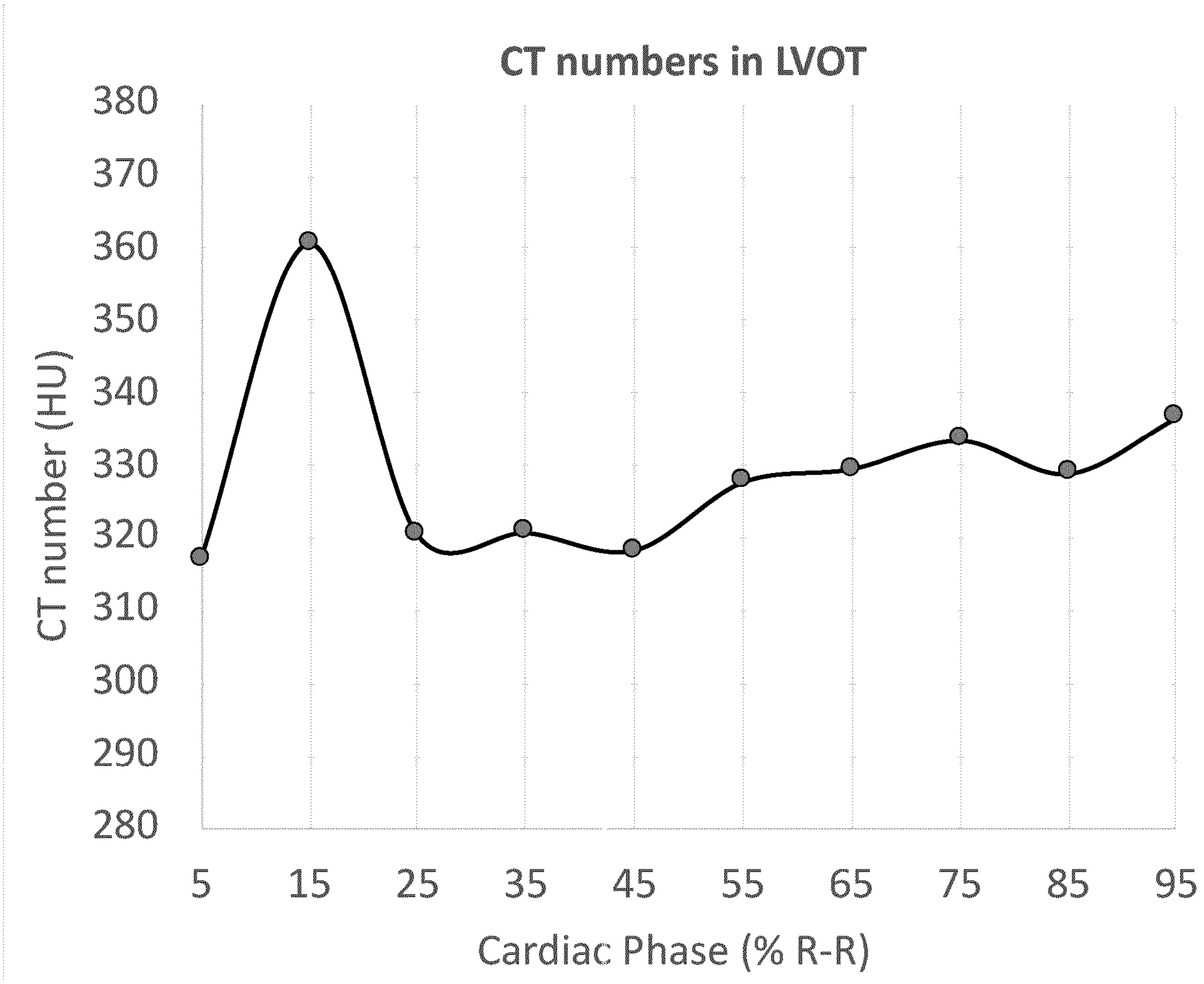

FIGURE 32C

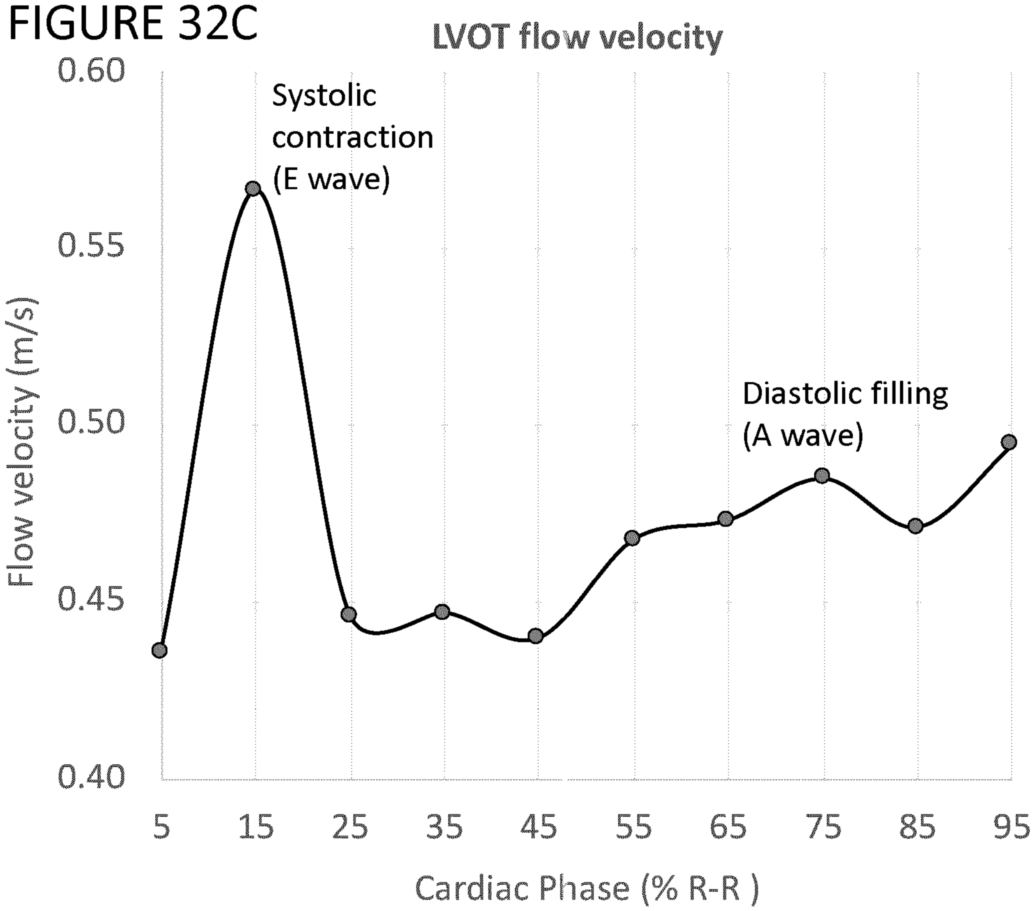


FIGURE 32D

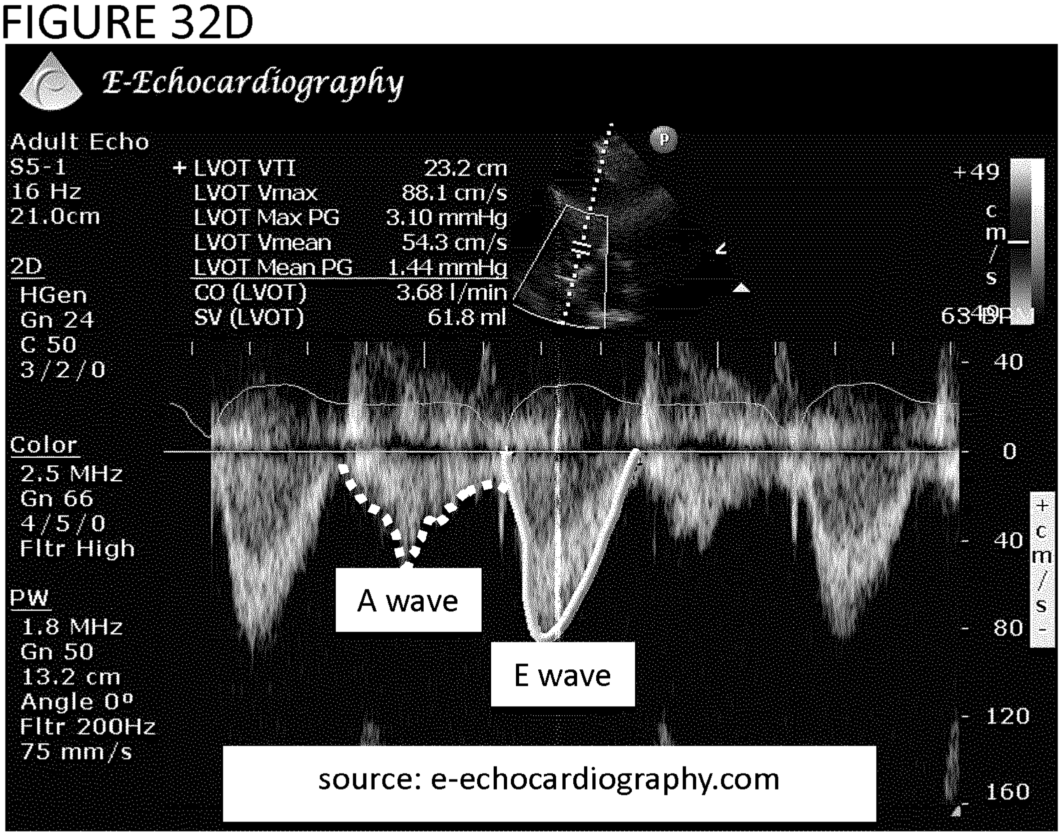

FIGURE 33A

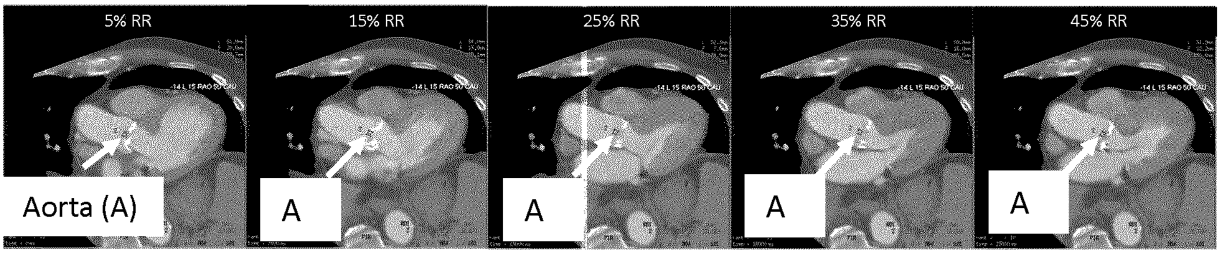


FIGURE 33B

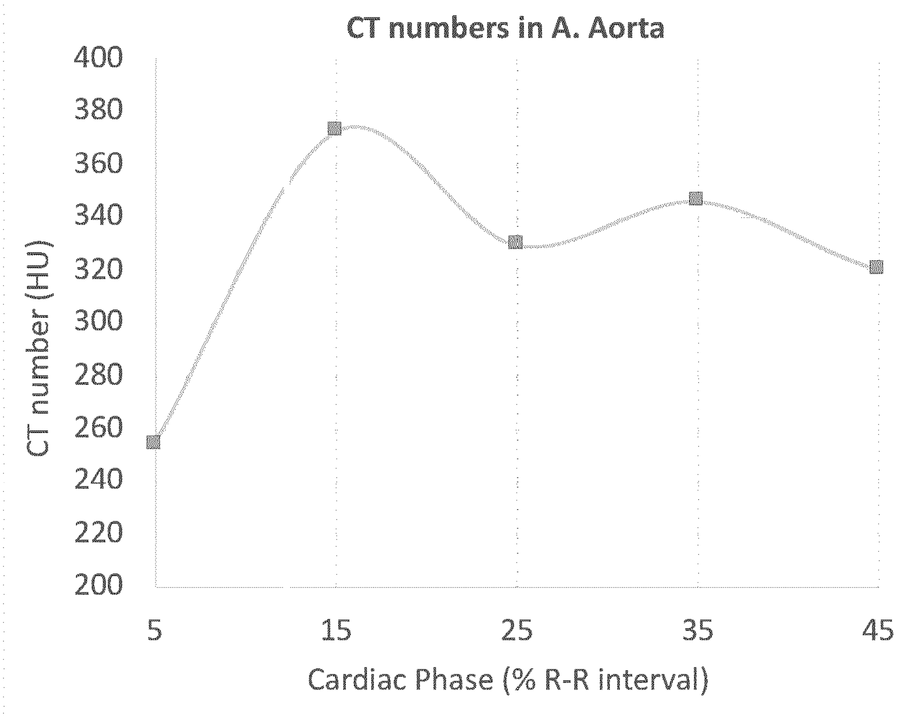


FIGURE 33C

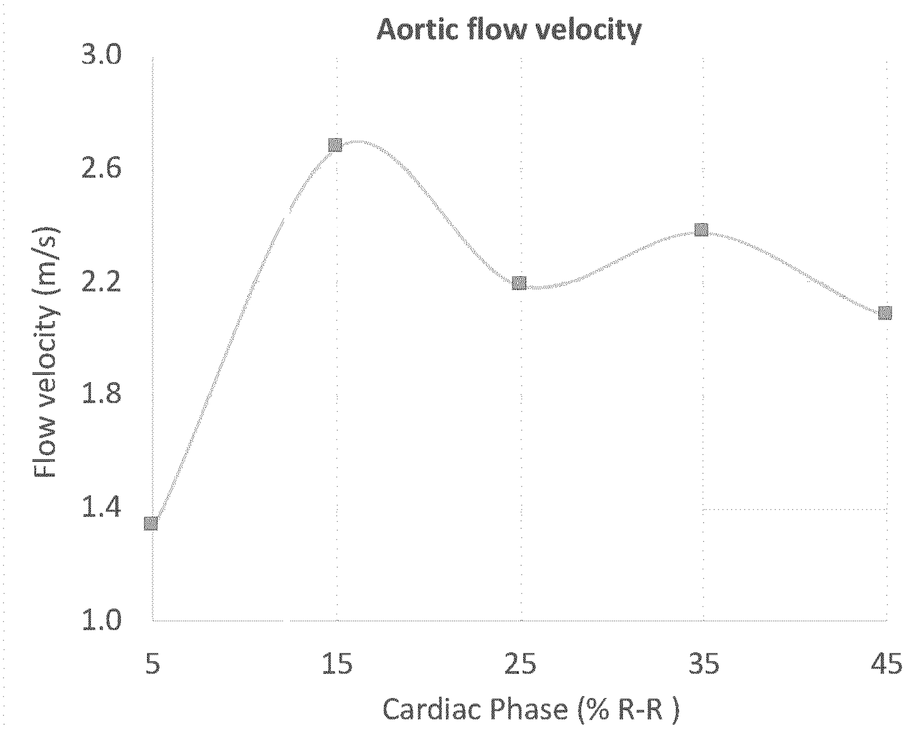

FIGURE 33D

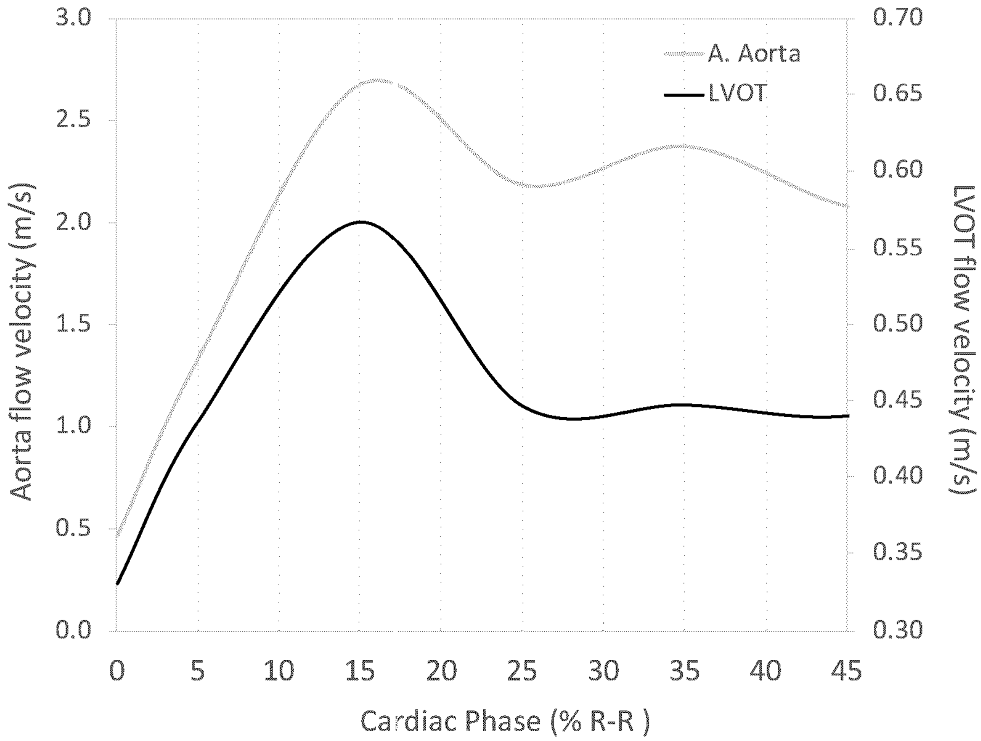


FIGURE 33E

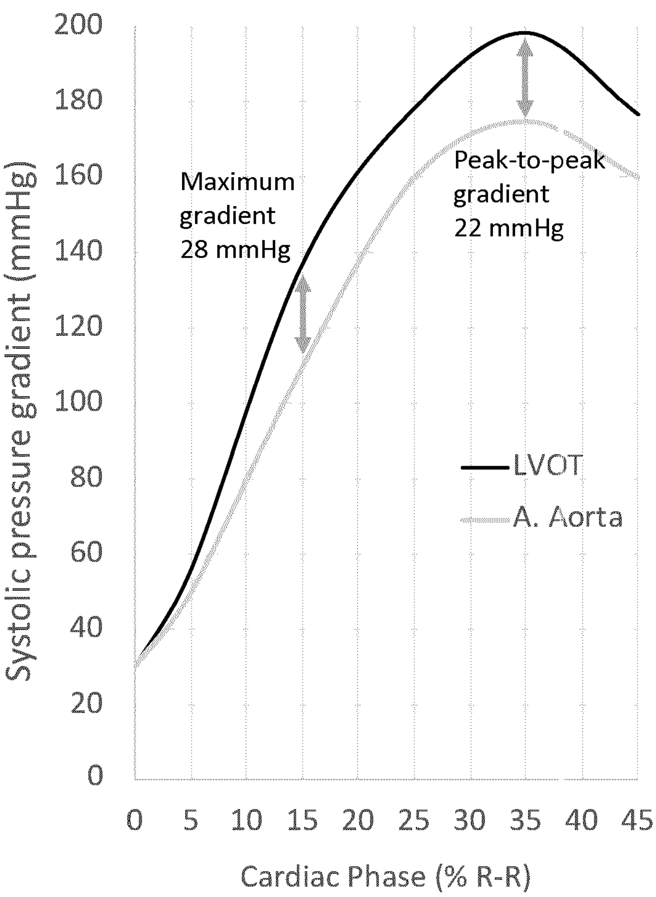


FIGURE 33F

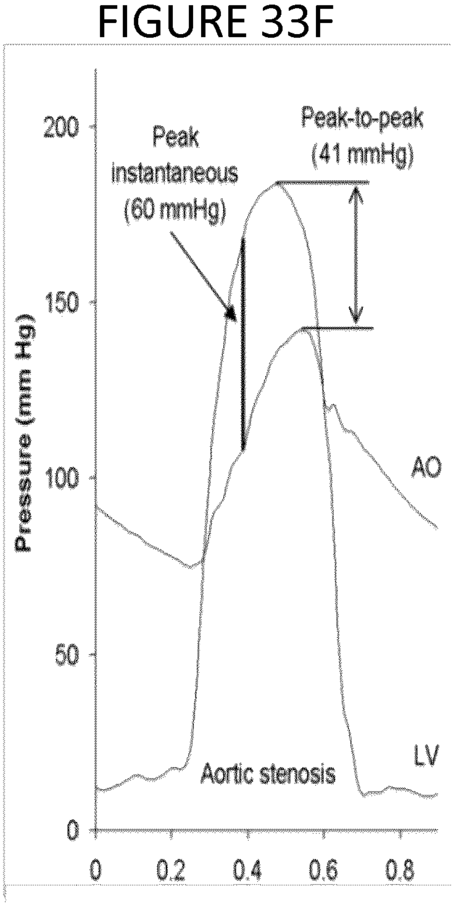


Geske et al. JACC Cardiovascular Interventions 2012.

FIGURE 34A

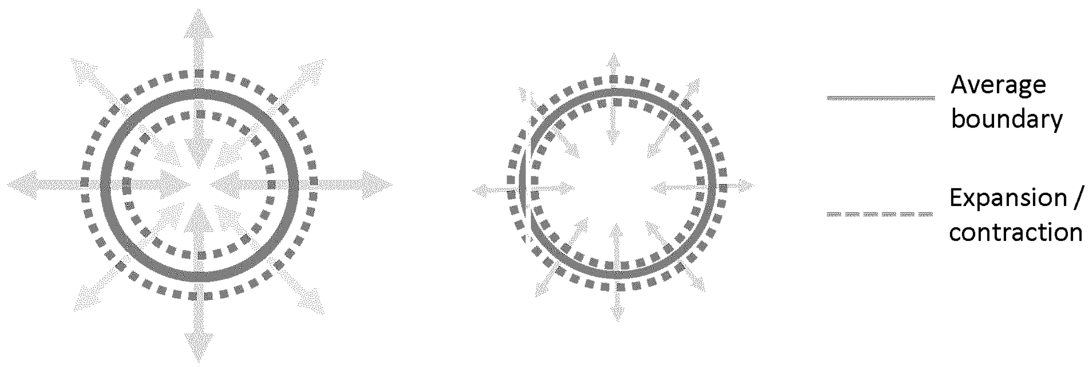


Cross-sectional view of a vessel with normal elasticity

Cross-sectional view of a vessel with abnormal elasticity

FIGURE 34B

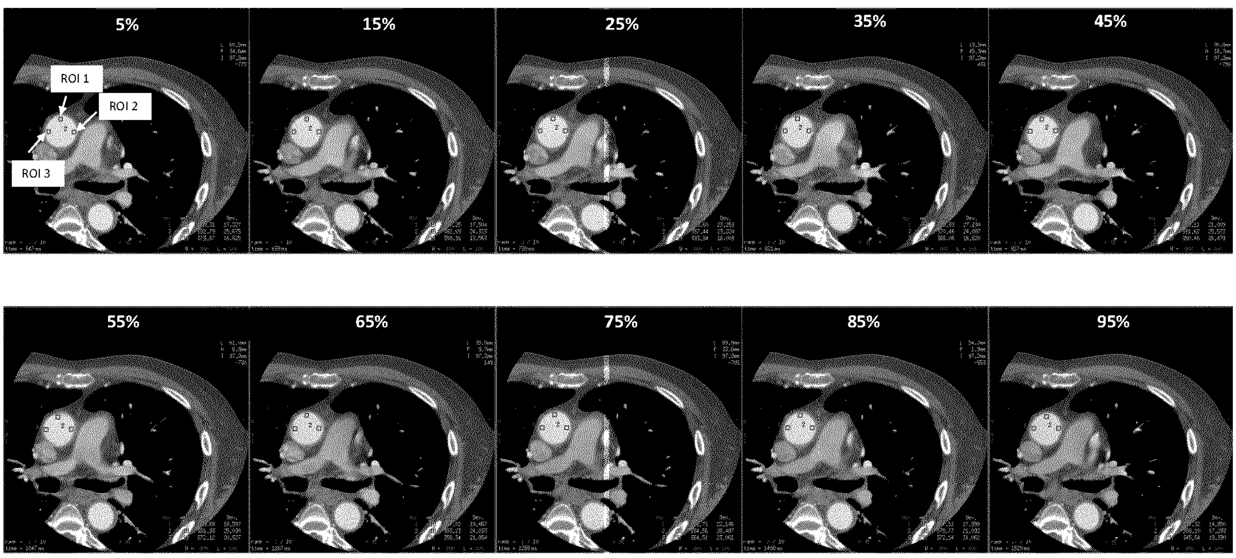

FIGURE 34C

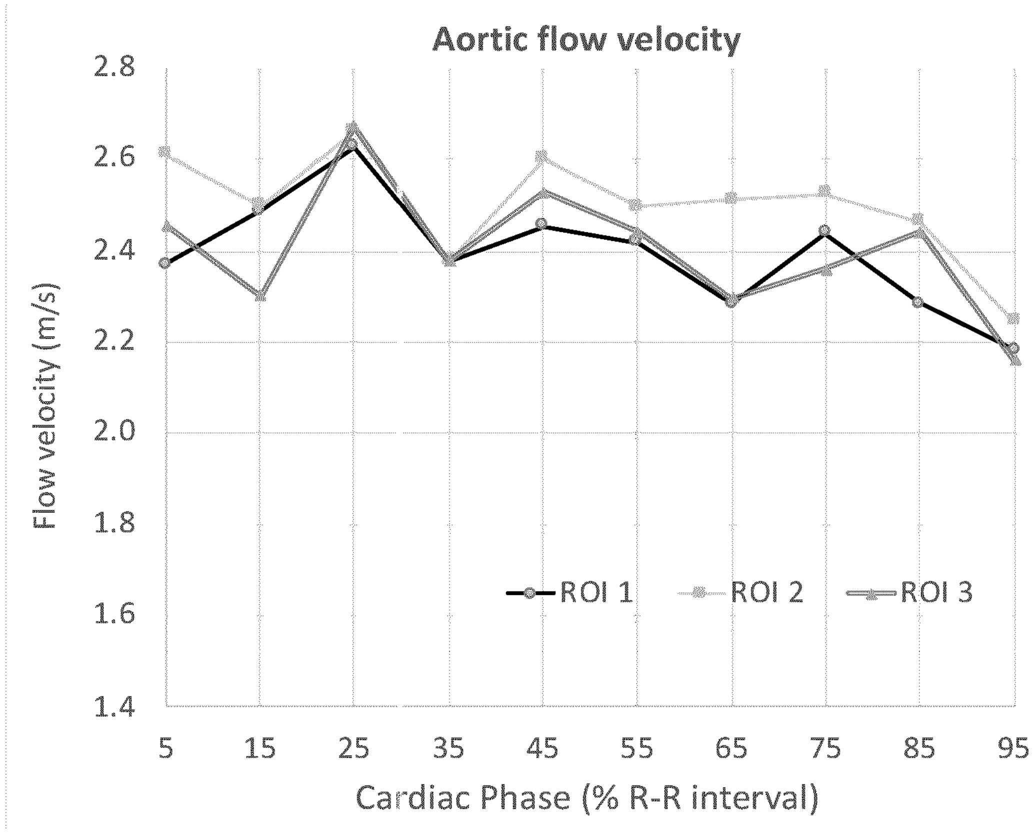


FIGURE 34D

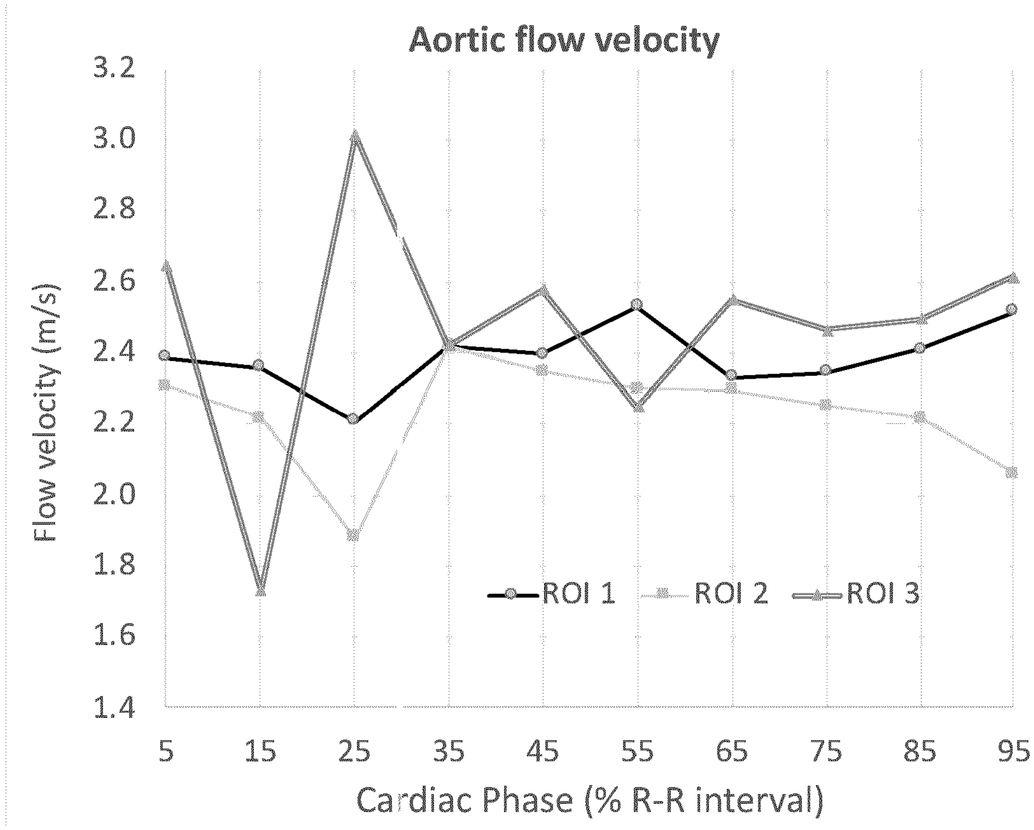

FIGURE 35A

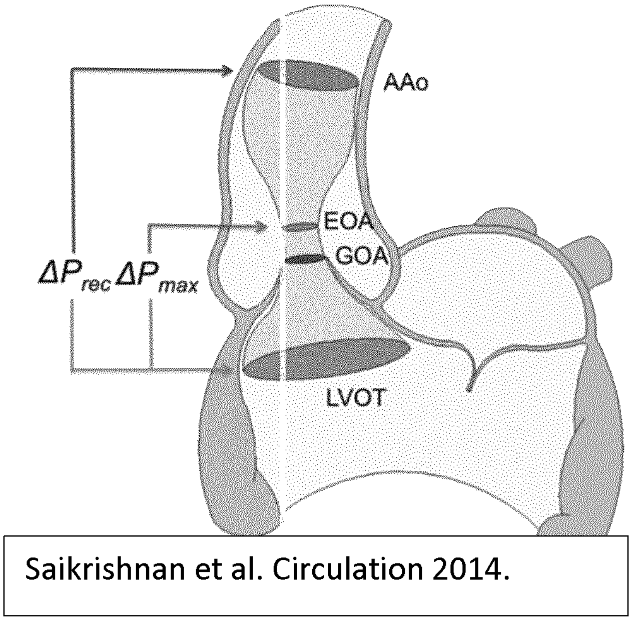


Saikrishnan et al. Circulation 2014.

FIGURE 35B

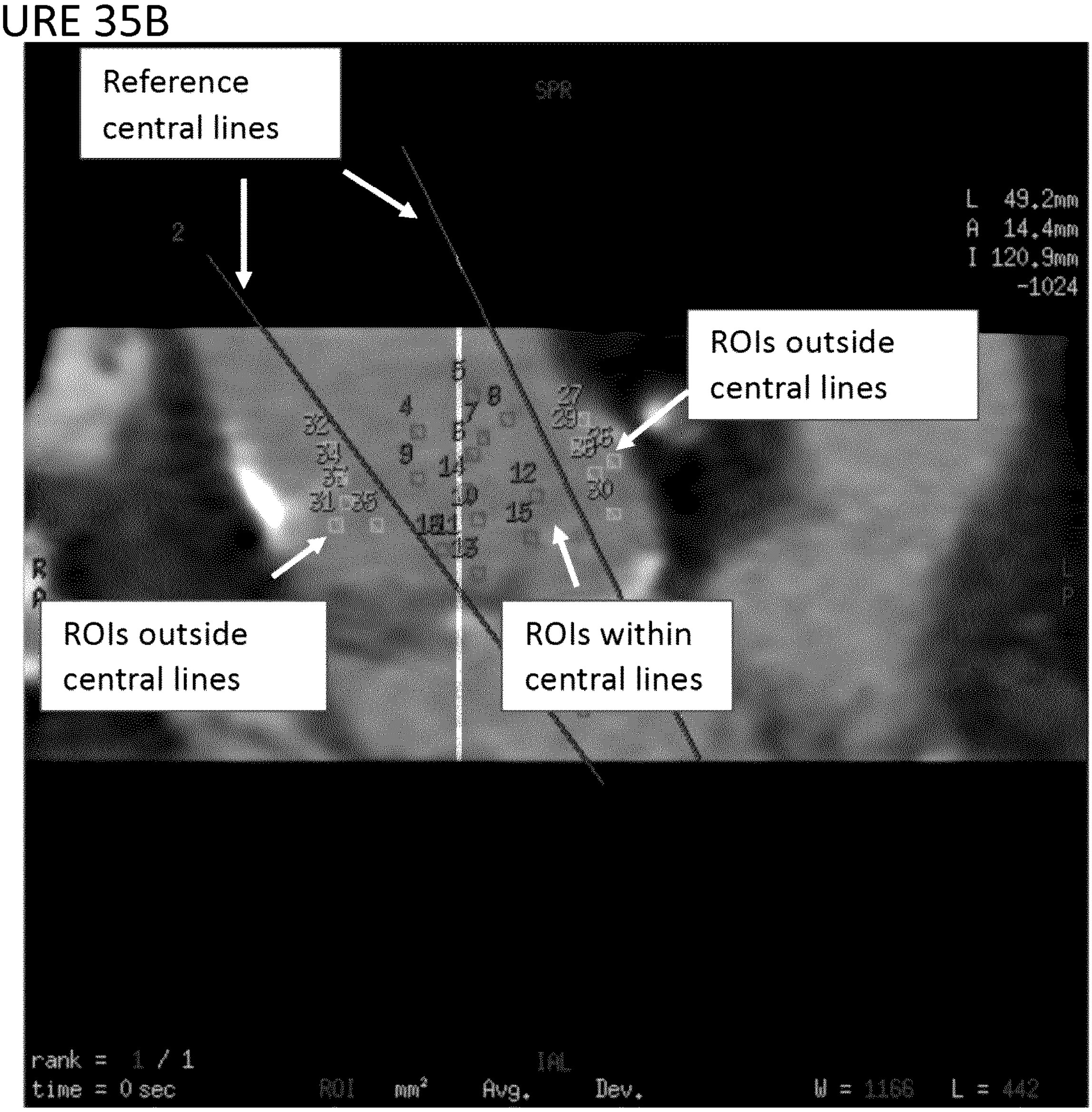

FIGURE 35C

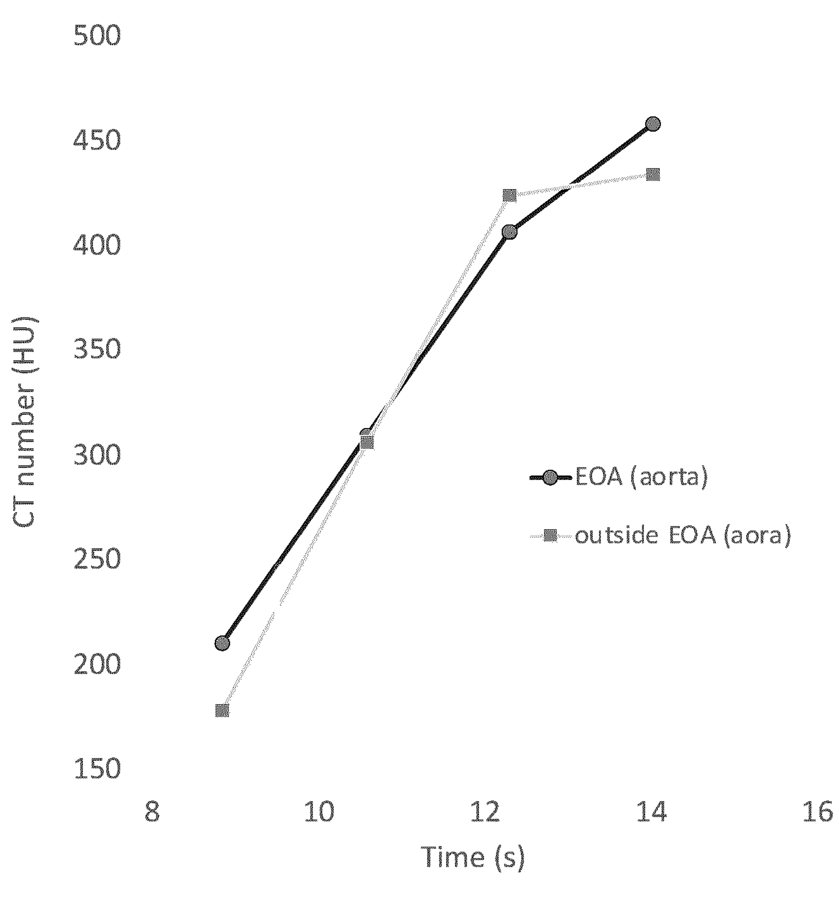


FIGURE 35D

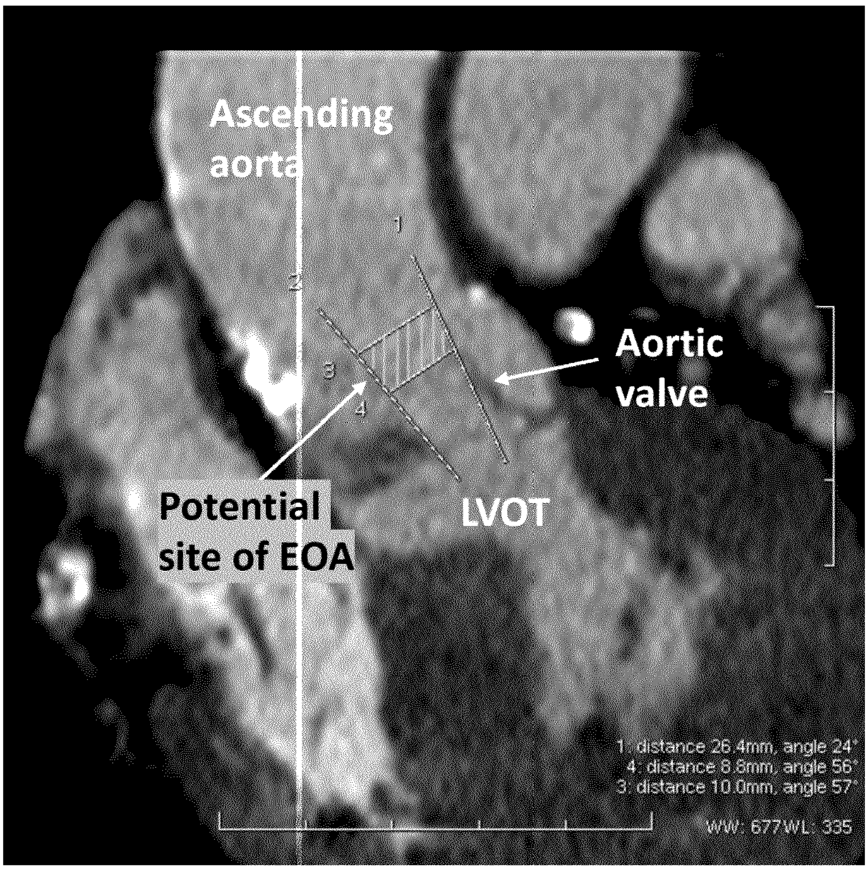

FIGURE 36A

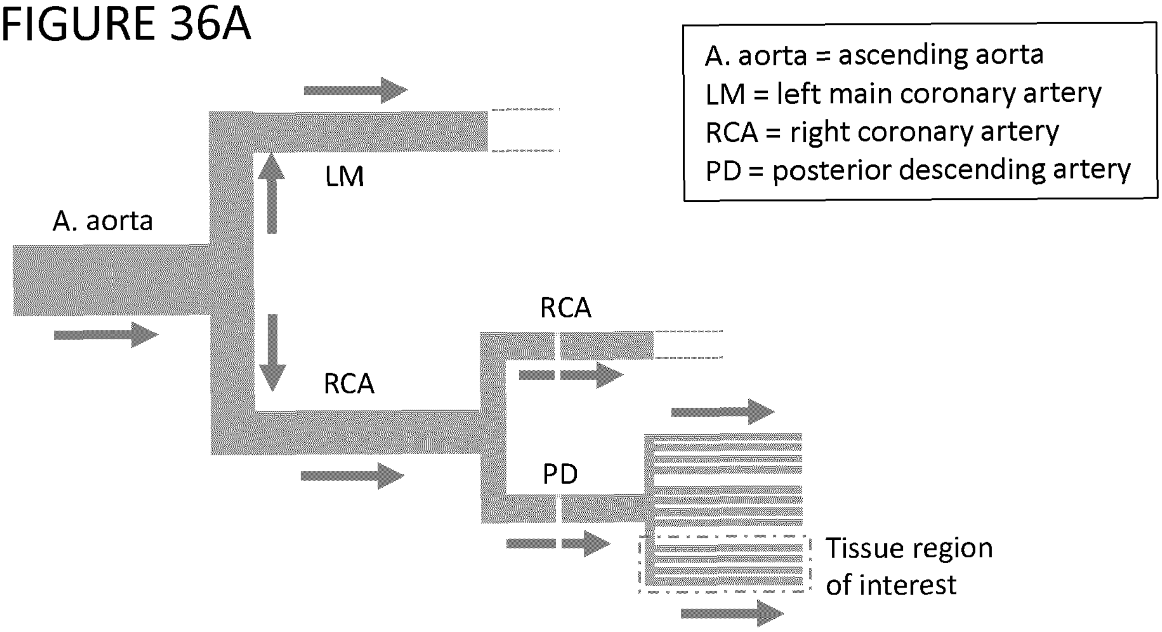


FIGURE 36B

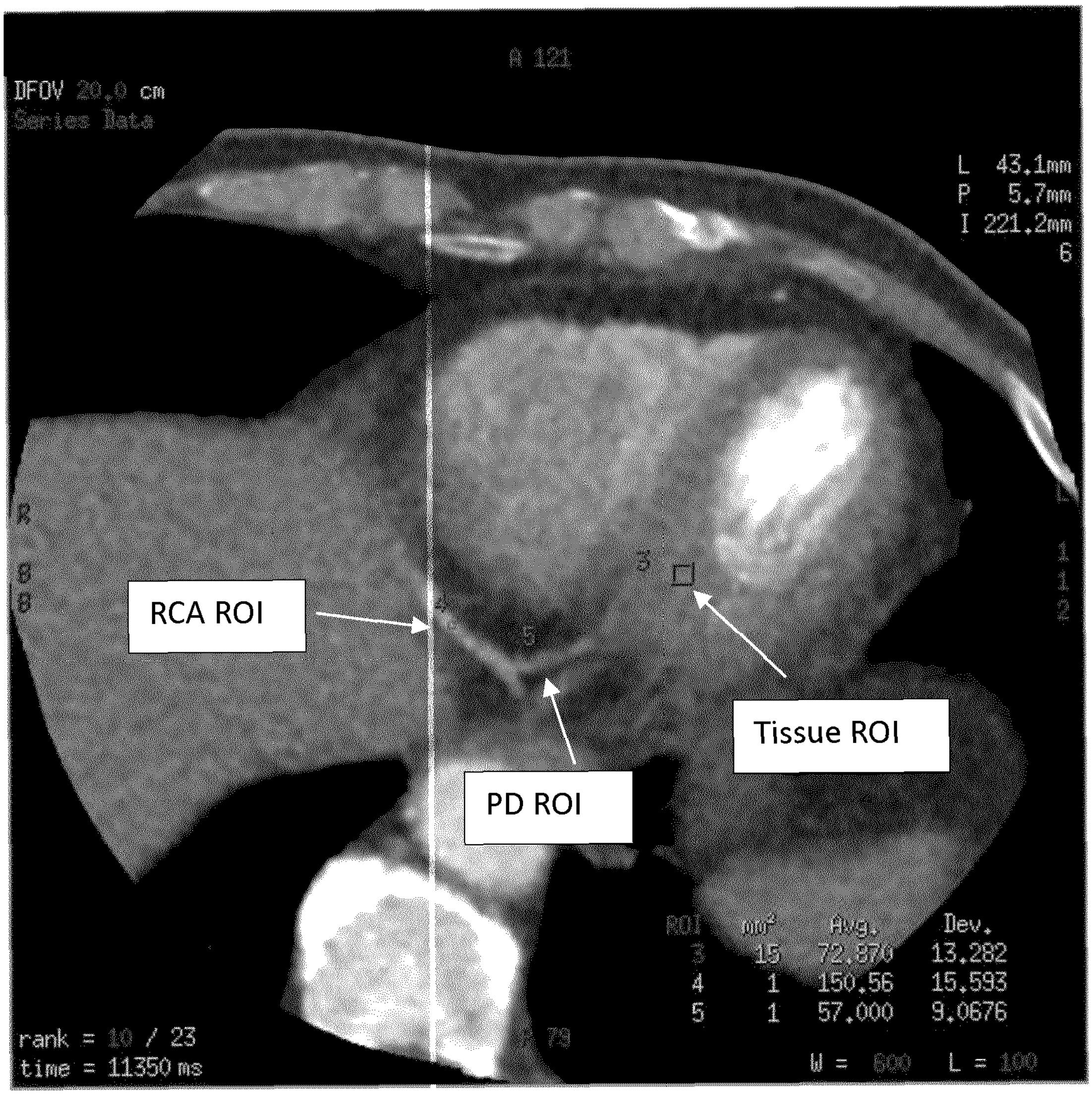

FIGURE 36C

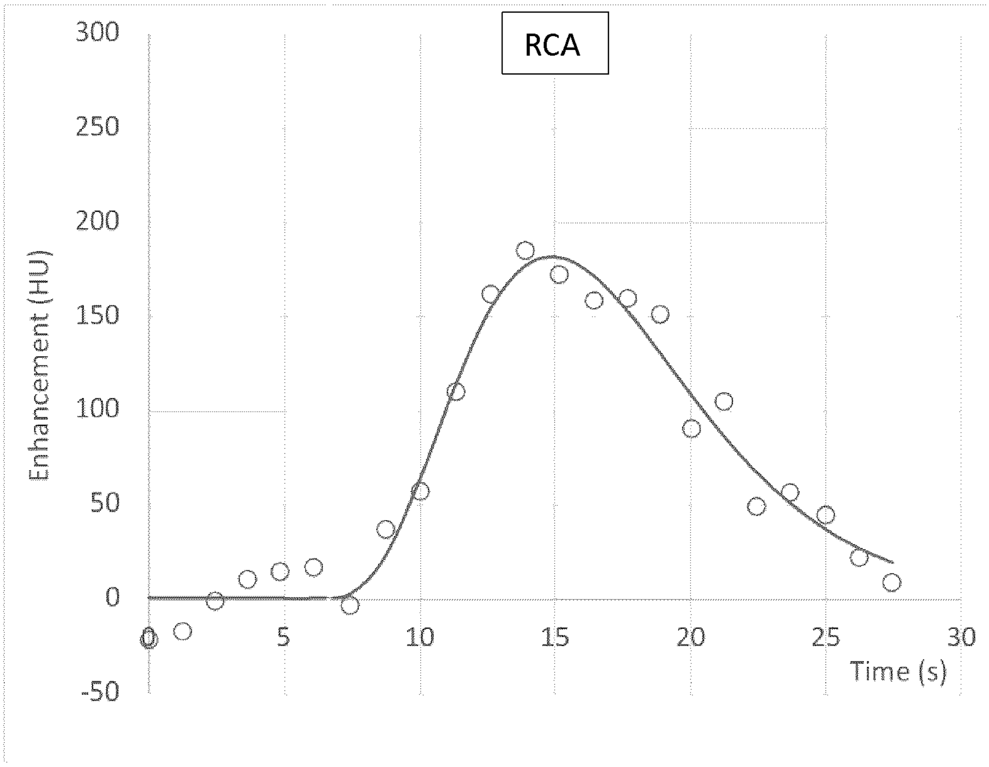


FIGURE 36D

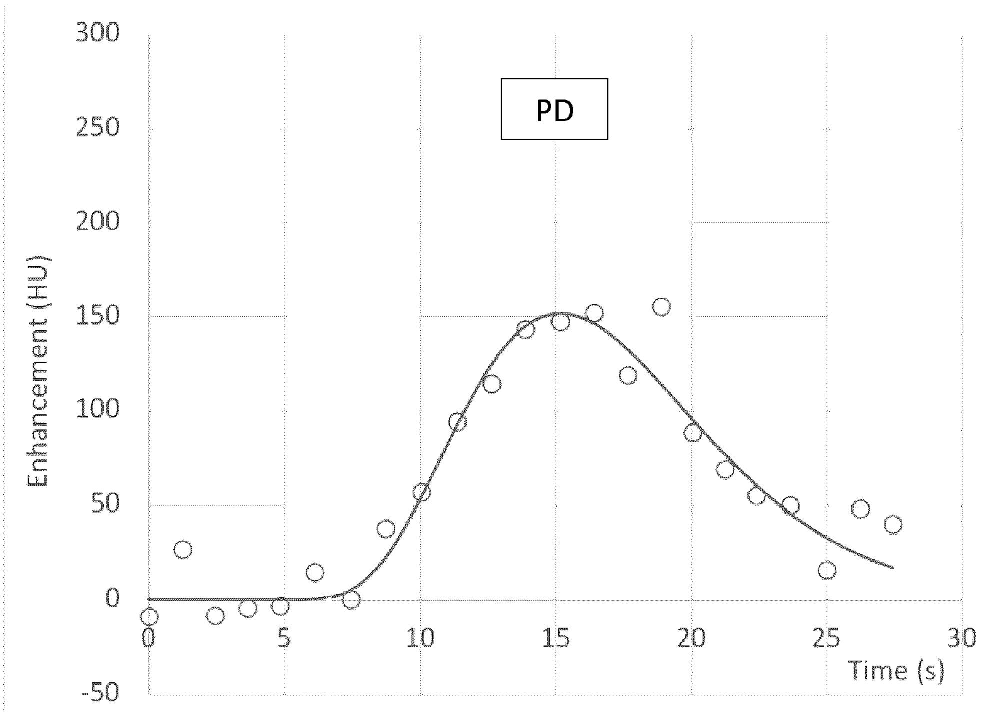

FIGURE 36E

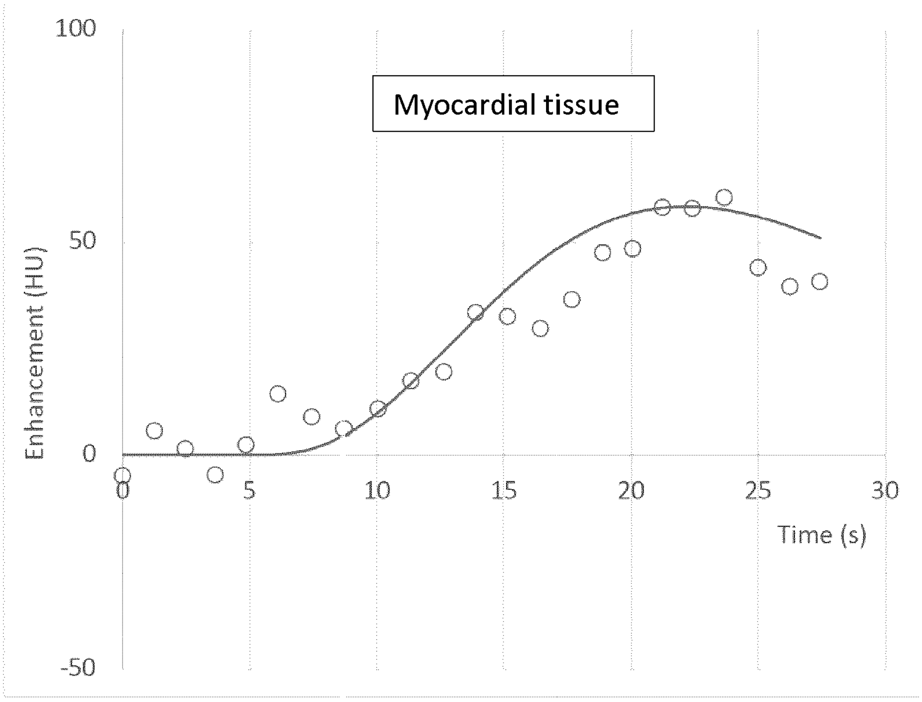


FIGURE 37A

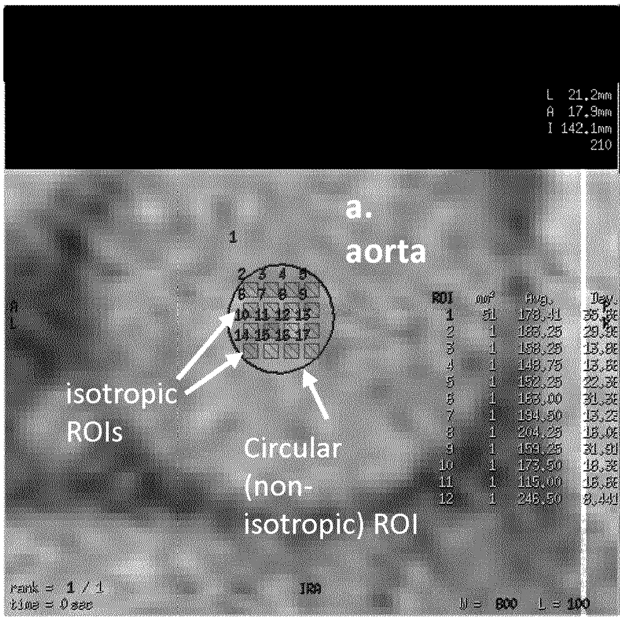


FIGURE 37B

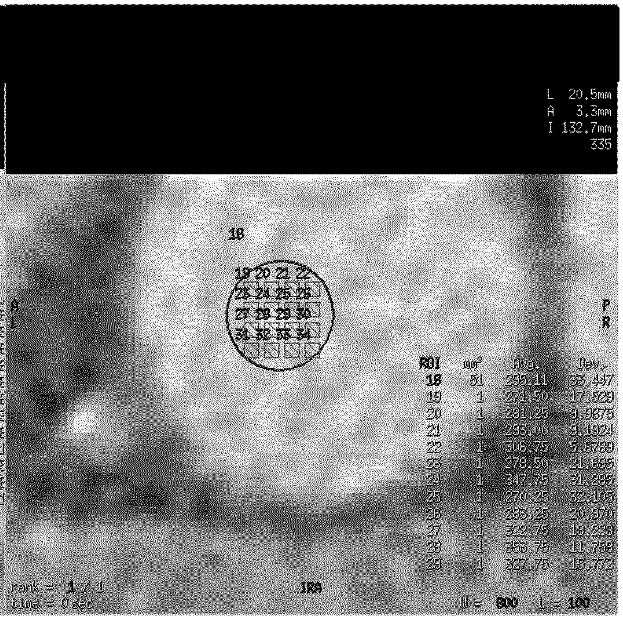

FIGURE 37C

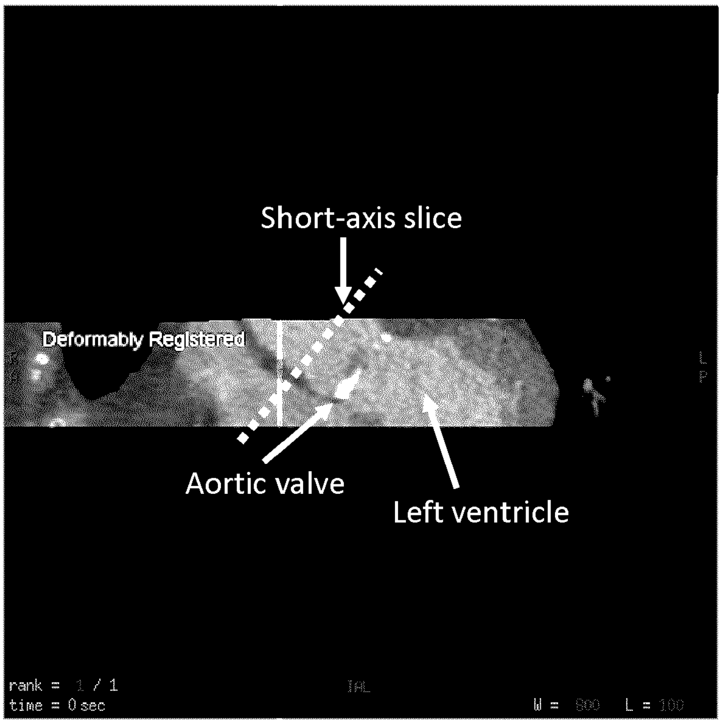


FIGURE 37D

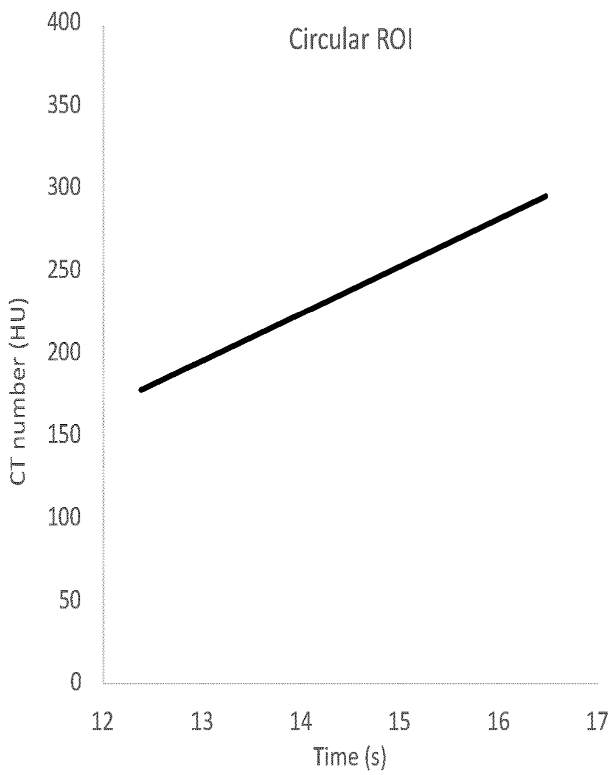


FIGURE 37E

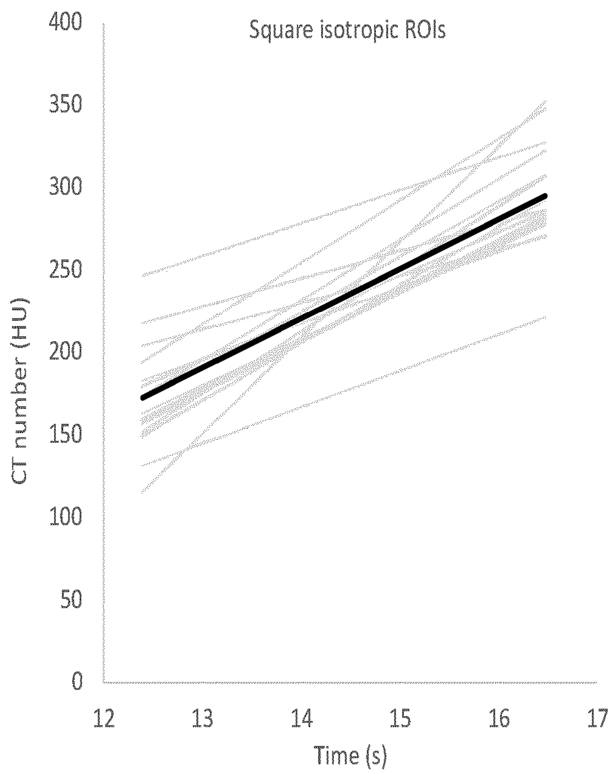

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CA 2019050668 W
- WO 2019218076 A


### Non-patent literature cited in the description


- **LANTZ et al.** Intracardiac Flow at 4D CT: Comparison with 4D Flow MRI. *Radiology*, 2018, vol. 289, 51-58
- **PEPER et al.** *Eur Radiol Exper*, 2019
- **SO A et al.** *Medical Physics*, 2016, vol. 43 (8), 4821
- **SO et al.** *Medical Physics*, 2016
- **ODAGIRL K et al.** *SpringerPlus*, 2016, vol. 5, 1071
- **VAN VARIK et al.** *Mechanisms of arterial remodeling: lessons from genetic diseases. Frontiers in Genetics*, 2012, vol. 3, 1-10
- **MOTWANI et al.** Systolic versus Diastolic Acquisition in Myocardial Perfusion MR Imaging. *Radiology*, vol. 262 (3), 816-823
- **RAKUSAN K et al.** *Circulation*, 1992, vol. 86, 38-46
- **YE F et al.** *Journal of Biomedical Optics*, 2020, vol. 25 (1), 016003
- **IVANOV KP et al.** *Microvascular Research*, 1981, vol. 22 (2), 143-55
- **DIEM et al.** *Biophysical Journal*, 2019, vol. 117, 2316-2323